(19) Europäisches Patentamt
European Patent Office
Office européen des brevets

(11) Veröffentlichungsnummer: **0 349 748 B1**

(12) **EUROPÄISCHE PATENTSCHRIFT**

(45) Veröffentlichungstag der Patentschrift: **19.11.92**

(51) Int. Cl.⁵: **A01N 43/38**, A01N 43/90, //(C07D471/04,235:00,221:00)

(21) Anmeldenummer: **89109458.3**

(22) Anmeldetag: **25.05.89**

(54) **Substituierte N-Aryl-Stickstoffheterocyclen.**

(30) Priorität: **08.06.88 DE 3819439**

(43) Veröffentlichungstag der Anmeldung:
**10.01.90 Patentblatt 90/02**

(45) Bekanntmachung des Hinweises auf die Patenterteilung:
**19.11.92 Patentblatt 92/47**

(84) Benannte Vertragsstaaten:
**BE CH DE FR GB IT LI NL**

(56) Entgegenhaltungen:
**EP-A- 0 068 822**
**EP-A- 0 190 755**
**EP-A- 0 215 424**
**EP-A- 0 234 323**
**EP-A- 0 259 264**
**EP-A- 0 259 265**
**EP-A- 0 260 228**
**EP-A- 0 288 960**
**DE-A- 3 618 501**
**US-A- 3 654 302**
**US-A- 4 124 375**

**Patent Abstract of Japan Band 6, Nr. 139 (C-116)(1017), 28. Juli 1982**

(73) Patentinhaber: **BAYER AG**

**W-5090 Leverkusen 1 Bayerwerk(DE)**

(72) Erfinder: **Schallner, Otto, Dr.**
**Noldeweg 22**
**W-4019 Monheim(DE)**
Erfinder: **Fischer, Reiner, Dr.**
**Nelly-Sachs-Strasse 23**
**W-4019 Monheim(DE)**
Erfinder: **Marhold, Albrecht, Dr.**
**Carl-Duisberg-Strasse 329**
**W-5090 Leverkusen 1(DE)**
Erfinder: **Reubke, Karl-Julius, Dr.**
**Rybnikerstrasse 10**
**W-5000 Köln 80(DE)**
Erfinder: **Santel, Hans-Joachim, Dr.**
**Grünstrasse 9a**
**W-5090 Leverkusen 1(DE)**
Erfinder: **Schmidt, Rudolf, R., Dr.**
**Im Waldwinkel 110**
**W-5060 Bergisch Gladbach 2(DE)**
Erfinder: **Lürssen, Klaus, Dr.**
**August-Kierspel-Strasse 145**
**W-5060 Bergisch Gladbach 2(DE)**
Erfinder: **Strang, Harry, Dr.**
**Unterdorfstrasse 6A**
**W-4000 Düsseldorf 31(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft neue substituierte N-Aryl-Stickstoffheterocyclen, Verfahren zu ihrer Herstellung und ihre Verwendung als Herbizide und als Pflanzenwuchsregulatoren.

Es ist bekannt, daß bestimmte Stickstoffheterocyclen, wie z. B. 5-tert-Butyl-3-(2,4-dichlor-5-isopropoxy-phenyl)-1,3,4-oxadiazol-2-on (Oxadiazone/®Ronstar), herbizide Eigenschaften aufweisen (vgl. US-P 3 835 862). Weitere N-Aryl-Stickstoffheterocyclen mit herbiziden Eigenschaften werden in EP-A-0 260 228, EP-A-0 234 323, EP-A-0 068 822, EP-A-0 313 96 3 , EP-A- 0 337 151, EP-A-0 288 960 und in JA 57-62 256 offenbart.

Die Wirkung der darin beschriebenen Verbindungen ist jedoch bei niedrigen Aufwandmengen bzw. Wirkstoffkonzentrationen unbefriedigend.

Es wurden nun die neuen substituierten N-Aryl-Stickstoffheterocyclen der allgemeinen Formel (I)

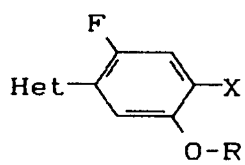

(I)

in welcher

Het für eine der nachstehenden heterocyclischen Gruppierungen

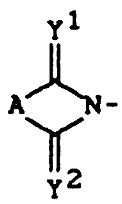

oder

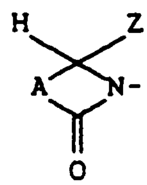

steht,

wobei

A für eine der nachstehenden Gruppierungen steht,

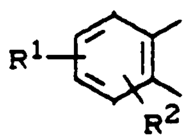 , 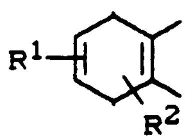 , 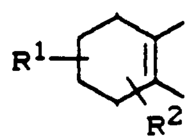 , 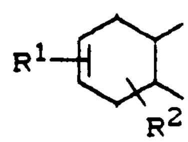 ,

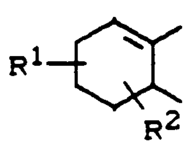 , 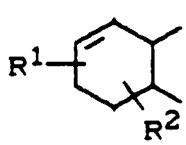 , 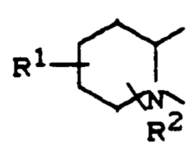 , 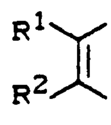 ,

wobei

| | |
|---|---|
| $R^1$ und $R^2$ | jeweils unabhängig voneinander für Wasserstoff, Halogen, Halogenalkyl oder Alkyl stehen, |
| $Y^1$ und $Y^2$ | jeweils für Sauerstoff oder Schwefel stehen und |
| Z | für Wasserstoff, Hydroxy oder Chlor steht, |
| R | für jeweils gegebenenfalls verzweigtes und jeweils durch wenigstens 1 Sauerstoffatom unterbrochenes Alkyl, Alkenyl, Cycloalkyl, Cycloalkenyl, Cycloalkylalkyl oder Cycloalkenylalkyl steht und |
| X | für Wasserstoff oder Halogen steht, |

gefunden,

ausgenommen die Verbindungen in denen

Het für

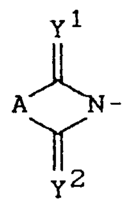 oder 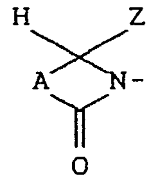 steht,

wobei

A für die Gruppierung

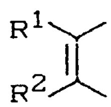 steht,

Z für Hydroxy steht,
X für Wasserstoff oder Halogen steht,
$R^1$ und $R^2$ jeweils unabhängig voneinander für Wasserstoff oder Alkyl stehen,
$Y^1$ für Sauerstoff oder Schwefel steht,
$Y^2$ für Sauerstoff steht und
R für Alkoxyalkyl, Oxiranyl alkyl oder für gegebenenfalls durch 1 oder 2 $C_1$-$C_4$-alkylsubstituierte Dioxolan-2-yl- und 1,3-Dioxan-2-yl-reste steht,

sowie die Verbindungen, in denen

Het für

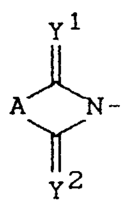 oder 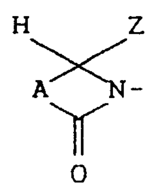 steht,

A für die Gruppierung

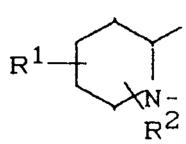 steht,

$R^1$ und $R^2$ jeweils unabhängig voneinander für Wasserstoff oder Alkyl stehen,
$Y^1$ für Sauerstoff oder Schwefel steht,
$Y^2$ für Sauerstoff oder Schwefel steht,
X für Sauerstoff oder Halogen steht,
Z für Hydroxy steht, und
R für Alkoxyalkyl Furanylalkyl, Pyranylalkyl und Oxiranyl alkyl steht,

sowie die Verbindungen, in denen

Het für

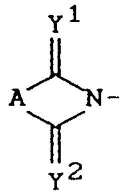 oder 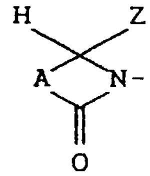 steht,

A für die Gruppierung

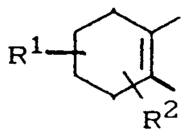 steht,

R[1] und R[2] jeweils unabhängig voneinander für Wasserstoff oder Alkyl stehen,
Y[1] für Sauerstoff oder Schwefel steht,
Y[2] für Sauerstoff oder Schwefel steht,
X für Wasserstoff oder Halogen steht,
Z für Hydroxy steht und
R für Alkoxyalkoxy,

$$-(\underset{\displaystyle Q}{CH})_{1-3}-CH(O\ C_1\text{-}C_4\text{-Alkyl})_2,$$

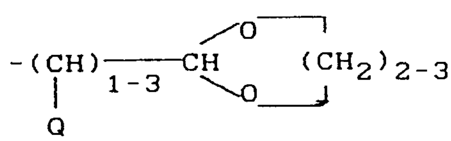

oder für durch wenigstens ein Sauerstoffatom unterbrochenes Cycloalkylalkyl oder Cycloalkenylalkyl steht und
Q für Wasserstoff oder $C_1$-$C_4$-Alkyl steht.

Weiter wurde gefunden, daß man die neuen substituierten N-Aryl-Stickstoffheterocyclen der allgemeinen Formel (I) erhält, wenn man

(a) für den Fall, daß Het für die Gruppierung

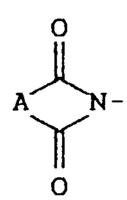 steht und

A, R und X die oben angegebenen Bedeutungen haben,
cyclische Anhydride der allgemeinen Formel (II)

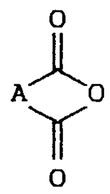 (II)

in welcher

A die oben angegebene Bedeutung hat,

mit Arylaminen der allgemeinen Formel (III)

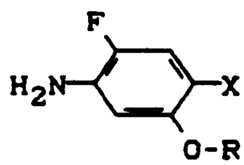 (III)

in welcher

R und X die oben angegebenen Bedeutungen haben,

gegebenenfalls in Gegenwart eines Verdünnungsmittels und gegebenenfalls in Gegenwart eines Reaktionshilfsmittels umsetzt, oder wenn man

(b) für den Fall, daß Het für die Gruppierung

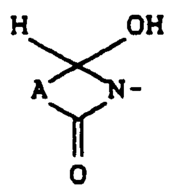 steht und

A, R und X die oben angegebenen Bedeutungen haben,

substituierte Arylimide der allgemeinen Formel (Ia)

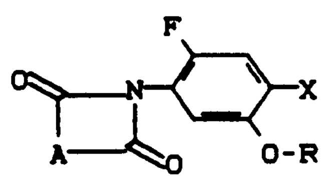 (Ia)

in welcher

A, R und X die oben angegebenen Bedeutungen haben,

mit einem Reduktionsmittel, gegebenenfalls in Gegenwart eines Verdünnungsmittels umsetzt, oder wenn man

(c) für den Fall, daß Het für die Gruppierung

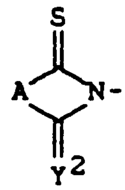 steht und

A, R, X und $Y^2$ die oben angegebenen Bedeutungen haben, substituierte Arylimide der allgemeinen Formel (Ia)

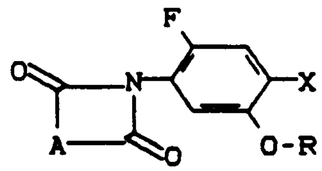 (Ia)

in welcher

A, R und X die oben angegebenen Bedeutungen haben,

mit einem Schwefelungsmittel, gegebenenfalls in Gegenwart eines Verdünnungsmittel umsetzt, oder wenn man

(d) für den Fall, daß Het für die Gruppierung

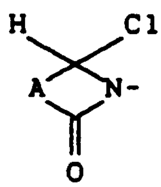 steht und

A, R und X die oben angegebenen Bedeutungen haben, N-Aryl-Stickstoffheterocyclen der allgemeinen Formel (Ib)

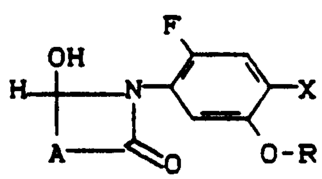 (Ib)

in welcher

A, R und X die oben angegebenen Bedeutungen haben,

mit Thionylchlorid, gegebenenfalls in Gegenwart eines Verdünnungsmittels und gegebenenfalls in Gegenwart eines Reaktionshilfsmittels umsetzt, oder wenn man

(e) für den Fall, daß Het für die Gruppierung

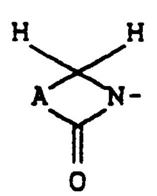 steht und

A, R und X die oben angegebenen Bedeutungen haben,
N-Aryl-Stickstoffheterocyclen der allgemeinen Formel (Ic)

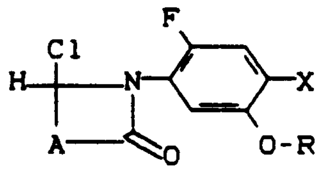

(Ic)

in welcher

A, R und X die oben angegebenen Bedeutungen haben,

mit Wasserstoff, in Gegenwart eines Katalysators sowie gegebenenfalls in Gegenwart eines Verdünnungsmittels und gegebenenfalls in Gegenwart eines Säurebindemittels umsetzt, oder wenn man
(f) für den Fall, daß Het für die Gruppierung

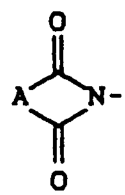

steht und

A, R und X die oben angegebenen Bedeutungen haben,
Hydroxyarylimide der allgemeinen Formel (IV)

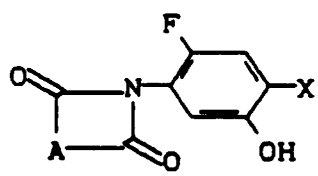

(IV)

in welcher

A und X die oben angegebenen Bedeutungen haben,

mit Alkylierungsmitteln der allgemeinen Formel (V)

$X^1$ - R (V)

in welcher

R die oben angegebene Bedeutung hat und
$X^1$ für eine nucleophile Abgangsgruppe steht,

gegebenenfalls in Gegenwart eines Säureakzeptors und gegebenenfalls in Gegenwart eines Verdünnungsmittels umsetzt, oder wenn man
(g) für den Fall, daß Het für die Gruppierung

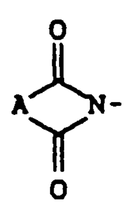

steht und

A und R die oben angegebenen Bedeutungen haben sowie

X für Halogen steht,

substituierte Arylimide der allgemeinen Formel (Id)

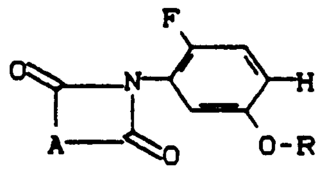

(Id)

in welcher

A und R die oben angegebenen Bedeutungen haben,

mit einem Halogenierungsmittel, gegebenenfalls in Gegenwart eines Katalysators und gegebenenfalls in Gegenwart eines Verdünnungsmittels umsetzt, oder wenn man

(h) für den Fall, daß Het für die Gruppierung

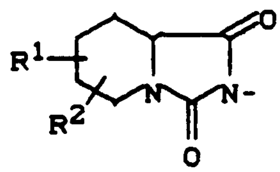

steht und

R, $R^1$, $R^2$ und X die oben angegebenen Bedeutungen haben

Arylamine der allgemeinen Formel (III)

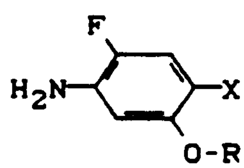

(III)

in welcher

R und X die oben angegebenen Bedeutungen haben,

mit Chlorameisensäureestern der allgemeinen Formel (VI)

$R^3O$ - CO - Cl (VI)

in welcher

$R^3$ für Niederalkyl, Benzyl oder Phenyl steht,

gegebenenfalls in Gegenwart eines Säureakzeptors und gegebenenfalls in Gegenwart eines Verdünnungsmittels umsetzt und die hierbei gebildeten

Arylurethane der allgemeinen Formel (VII)

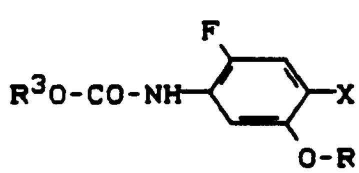

(VII)

in welcher

R, $R^3$ und X die oben angegebenen Bedeutungen haben

mit Piperidin-2-carbonsäureestern der allgemeinen Formel (VIII)

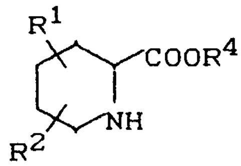 (VIII)

in welcher

| | |
|---|---|
| $R^1$ und $R^2$ | die oben angegebenen Bedeutungen haben und |
| $R^4$ | für Niederalkyl steht, |

gegebenenfalls in Gegenwart eines Verdünnungsmittels umsetzt, ausgenommen die Verbindungen in denen

Het für

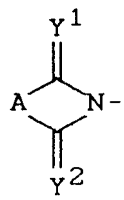 oder 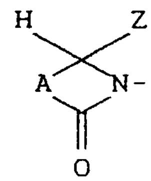 steht,

wobei

A für die Gruppierung

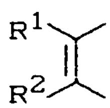 steht,

| | |
|---|---|
| Z | für Hydroxy steht, |
| X | für Wasserstoff oder Halogen steht, |
| $R^1$ und $R^2$ | jeweils unabhängig voneinander für Wasserstoff oder Alkyl stehen, |
| $Y^1$ | für Sauerstoff oder Schwefel steht, |
| $Y^2$ | für Sauerstoff steht und |
| R | für Alkoxyalkyl, Oxiranyl alkyl oder für gegebenenfalls durch 1 oder 2 $C_1$-$C_4$-alkylsubstituierte Dioxolan-2-yl- und 1,3-Dioxan-2-yl-reste steht, |
| | sowie die Verbindungen, in denen |
| Het | für |

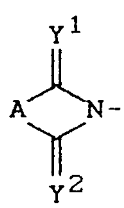 oder 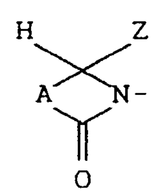 steht,

A für die Gruppierung

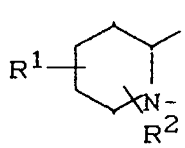 steht,

| | |
|---|---|
| $R^1$ und $R^2$ | jeweils unabhängig voneinander für Wasserstoff oder Alkyl stehen, |
| $Y^1$ | für Sauerstoff oder Schwefel steht, |
| $Y^2$ | für Sauerstoff oder Schwefel steht, |
| X | für Sauerstoff oder Halogen steht, |
| Z | für Hydroxy steht, und |
| R | für Alkoxyalkyl Furanylalkyl, Pyranylalkyl und Oxiranyl alkyl steht, |
| | sowie die Verbindungen, in denen |
| Het | für |

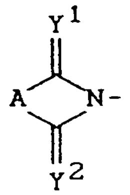 oder 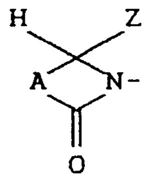 steht,

A für die Gruppierung

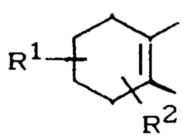 steht,

| | |
|---|---|
| $R^1$ und $R^2$ | jeweils unabhängig voneinander für Wasserstoff oder Alkyl stehen, |
| $Y^1$ | für Sauerstoff oder Schwefel steht, |
| $Y^2$ | für Sauerstoff oder Schwefel steht, |
| X | für Wasserstoff oder Halogen steht, |
| Z | für Hydroxy steht und |
| R | für Alkoxyalkoxy, |

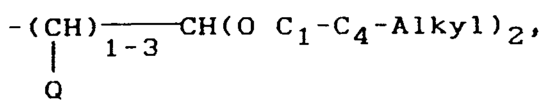

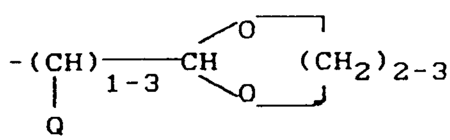

oder für durch wenigstens ein Sauerstoffatom unterbrochenes Cycloalkylalkyl oder Cycloalkenylalkyl steht und

Q für Wasserstoff oder $C_1$-$C_4$-Alkyl steht.

Schließlich wurde gefunden, daß die neuen substituierten N-Aryl-Stickstoffheterocyclen der allgemeinen Formel (I) herbizide und pflanzenwuchsregulierende Eigenschaften aufweisen.

Überraschenderweise sind die erfindungsgemäßen substituierten N-Aryl-Stickstoffheterocyclen der Formel (I) gegen Unkräuter wesentlich stärker wirksam als 5-tert-Butyl-3-(2,4-dichlor-5-isopropoxy-phenyl)-1,3,4-oxadiazol-2-on, welches ein strukturell ähnlicher vorbekannter Wirkstoff gleicher Wirkungsrichtung ist.

Die Erfindung betrifft vorzugsweise Verbindungen der Formel (I), in welcher

Het für eine der nachstehenden heterocyclischen Gruppierungen

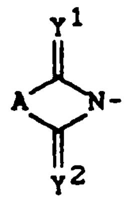 oder 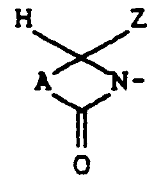 steht,

wobei

A für eine der nachstehenden Gruppierungen steht,

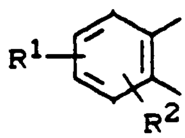, 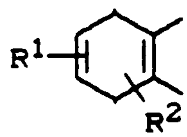, 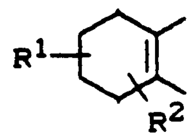, 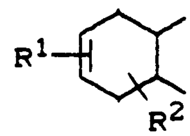,

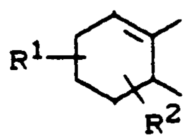, 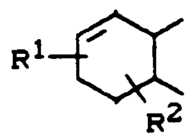, 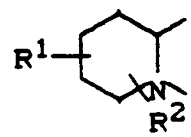, 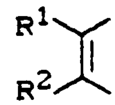,

wobei

$R^1$ und $R^2$ jeweils unabhängig voneinander für Wasserstoff, Fluor, Chlor, Brom oder für jeweils geradkettiges oder verzweigtes Alkyl oder Halogenalkyl mit jeweils 1 bis 3 Kohlenstoffatomen und im Fall des Halogenalkyl mit 1 bis 5 gleichen oder verschiedenen Halogenatomen, insbesondere Fluor oder Chlor, stehen,

$Y^1$ und $Y^2$ für Sauerstoff oder Schwefel stehen,

Z für Wasserstoff, Hydroxy oder Chlor steht,

R für jeweils gegebenenfalls verzweigtes und jeweils durch 1 bis 4 Sauerstoffatome unterbrochenes Alkyl, Alkenyl, Cycloalkyl, Cycloalkenyl, Cycloalkylalkyl oder Cycloalkenylalkyl mit jeweils bis zu 20 Kohlenstoffatomen steht und

X für Wasserstoff, Fluor, Chlor oder Brom steht, ausgenommen die Verbindungen in denen

Het für

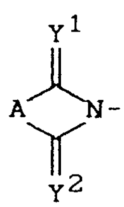 oder 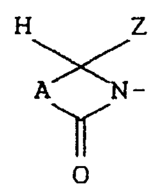 steht,

wobei

A für die Gruppierung

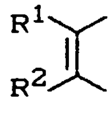 steht,

Z für Hydroxy steht,

X für Wasserstoff oder Halogen steht,

$R^1$ und $R^2$ jeweils unabhängig voneinander für Wasserstoff oder Alkyl stehen,

$Y^1$ für Sauerstoff oder Schwefel steht,

$Y^2$ für Sauerstoff steht und

R für Alkoxyalkyl, Oxiranyl alkyl oder für gegebenenfalls durch 1 oder 2 $C_1$-$C_4$-alkylsubstituierte Dioxolan-2-yl- und 1,3-Dioxan-2-yl-reste steht,

sowie die Verbindungen, in denen

Het für

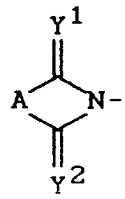
oder
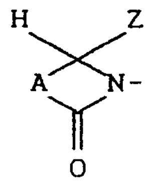
steht,

A für die Gruppierung

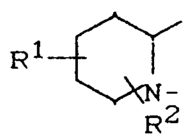
steht,

$R^1$ und $R^2$ jeweils unabhängig voneinander für Wasserstoff oder Alkyl stehen,

$Y^1$ für Sauerstoff oder Schwefel steht,

$Y^2$ für Sauerstoff oder Schwefel steht,

X für Sauerstoff oder Halogen steht,

Z für Hydroxy steht, und

R für Alkoxyalkyl Furanylalkyl, Pyranylalkyl und Oxiranyl alkyl steht,

sowie die Verbindungen, in denen

Het für

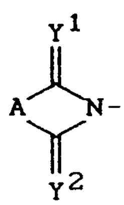
oder
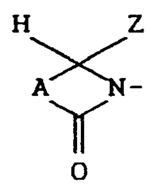
steht,

A für die Gruppierung

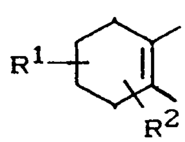
steht,

$R^1$ und $R^2$ jeweils unabhängig voneinander für Wasserstoff oder Alkyl stehen,

$Y^1$ für Sauerstoff oder Schwefel steht,

$Y^2$ für Sauerstoff oder Schwefel steht,

X für Wasserstoff oder Halogen steht,

Z für Hydroxy steht und

R für Alkoxyalkoxy,

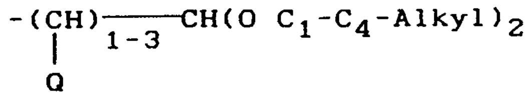,

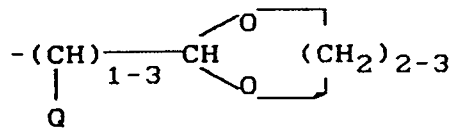

oder für durch wenigstens ein Sauerstoffatom unterbrochenes Cycloalkylalkyl oder Cycloalkenylalkyl steht und

Q für Wasserstoff oder $C_1$-$C_4$-Alkyl steht.

Die Erfindung betrifft insbesondere Verbindungen der Formel (I), in welcher

Het für eine der nachstehenden heterocyclischen Gruppierungen

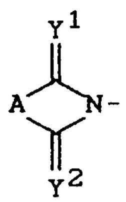 oder 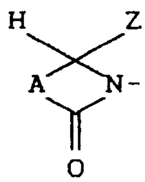 steht,

wobei

A für eine der nachstehenden Gruppierungen steht,

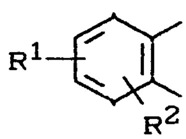, 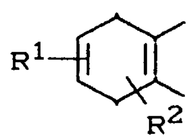, 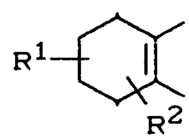, 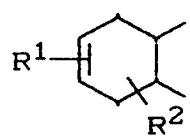,

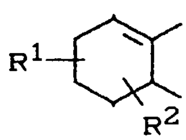, 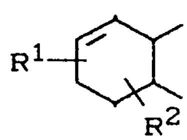, 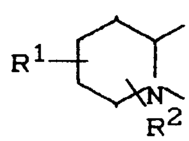, 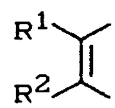,

wobei

$R^1$ und $R^2$ jeweils unabhängig voneinander für Wasserstoff, Methyl oder Trifluormethyl stehen,

$Y^1$ und $Y^2$ für Sauerstoff oder Schwefel stehen,

Z für Wasserstoff, Hydroxy oder Chlor steht,

R für jeweils gegebenenfalls verzweigtes Oxaalkyl, Oxaalkenyl oder Dioxaalkyl mit jeweils bis zu 10 Kohlenstoffatomen oder für Oxacycloalkyl-alkyl, Oxacycloalkenyl-alkyl oder Dioxacycloalkyl-alkyl mit jeweils bis zu 8 Kohlenstoffatomen im Oxacycloalkyl-, Oxacycloalkenyl- oder Dioxacycloalkylteil und bis zu 3 Kohlenstoffatomen im Alkylteil steht und

X für Wasserstoff, Chlor oder Brom steht, ausgenommen die Verbindungen in denen

Het für

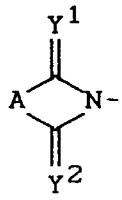 oder 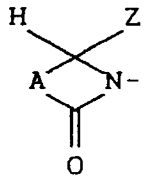 steht,

wobei

A für die Gruppierung

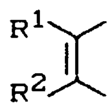 steht,

Z für Hydroxy steht,

X für Wasserstoff oder Halogen steht,

$R^1$ und $R^2$ jeweils unabhängig voneinander für Wasserstoff oder Alkyl stehen,

$Y^1$ für Sauerstoff oder Schwefel steht,

$Y^2$ für Sauerstoff steht und

R für Alkoxyalkyl, Oxiranyl alkyl oder für gegebenenfalls durch 1 oder 2 $C_1$-$C_4$-alkylsubstituierte Dioxolan-2-yl- und 1,3-Dioxan-2-yl-reste steht,

sowie die Verbindungen, in denen

Het für

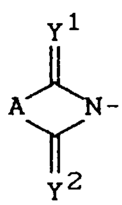 oder 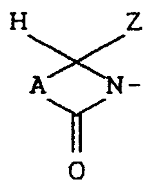 steht,

A für die Gruppierung

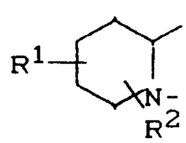 steht,

$R^1$ und $R^2$ jeweils unabhängig voneinander für Wasserstoff oder Alkyl stehen,

$Y^1$ für Sauerstoff oder Schwefel steht,

$Y^2$ für Sauerstoff oder Schwefel steht,

X für Sauerstoff oder Halogen steht,

Z für Hydroxy steht, und

R für Alkoxyalkyl Furanylalkyl, Pyranylalkyl und Oxiranyl alkyl steht,

sowie die Verbindungen, in denen

Het für

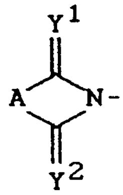 oder 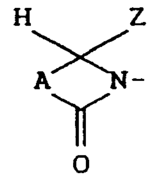 steht,

| | |
|---|---|
| A | für die Gruppierung |

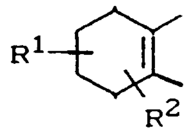 steht,

| | |
|---|---|
| $R^1$ und $R^2$ | jeweils unabhängig voneinander für Wasserstoff oder Alkyl stehen, |
| $Y^1$ | für Sauerstoff oder Schwefel steht, |
| $Y^2$ | für Sauerstoff oder Schwefel steht, |
| X | für Wasserstoff oder Halogen steht, |
| Z | für Hydroxy steht und |
| R | für Alkoxyalkoxy, |

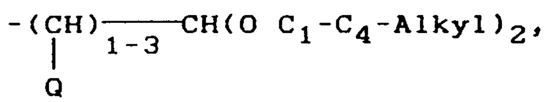

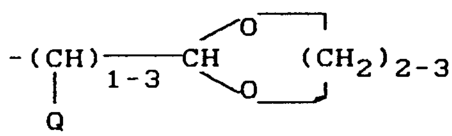

oder für durch wenigstens ein Sauerstoffatom unterbrochenes Cycloalkylalkyl oder Cycloalkenylalkyl steht und

| | |
|---|---|
| Q | für Wasserstoff oder $C_1$-$C_4$-Alkyl steht. |

Beispiele für die erfindungsgemäßen Verbindungen der Formel (I) sind in der nachstehenden Tabelle 1 aufgeführt - vgl. auch die Herstellungsbeispiele.

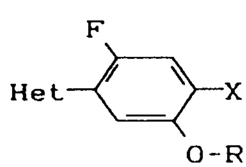 (I)

**Tabelle 1**: Beispiele für die Verbindungen der Formel (I)

| Het | X | R |
|---|---|---|
| 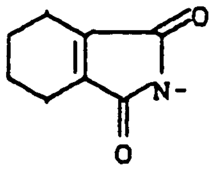 | H | $-CH_2CH_2-O-CH_2CH_2-O-CH_3$ |
| 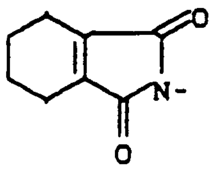 | H | $-CH_2CH_2-O-CH_2CH_2-O-C_2H_5$ |
| 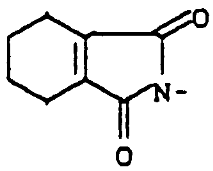 | F | $-CH_2CH_2-O-CH_2CH_2-O-CH_3$ |
| 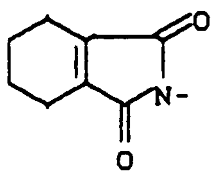 | F | $-CH_2CH_2-O-CH_2CH_2-O-C_2H_5$ |
| 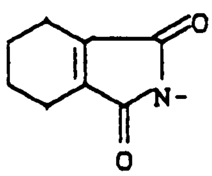 | Cl | $-CH_2CH_2-O-CH_2CH_2-O-CH_3$ |
| 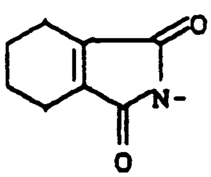 | Cl | $-CH_2CH_2-O-CH_2CH_2-O-C_2H_5$ |
| 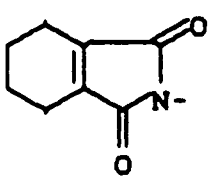 | Br | $-CH_2CH_2-O-CH_2CH_2-O-CH_3$ |

Tabelle 1 - Fortsetzung

| Het | X | R |
|---|---|---|
| 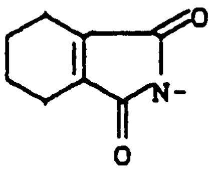 | Br | $-CH_2CH_2-O-CH_2CH_2-O-C_2H_5$ |
| 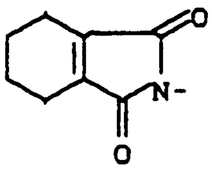 | Cl | $-CH_2CH_2-O-CH_2CH_2-O-C_3H_7$ |
| 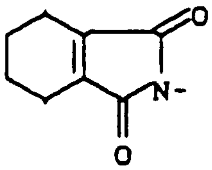 | Cl | $-CH_2CH_2-O-CH_2CH_2-O-C_4H_9$ |
| 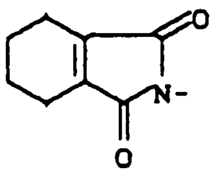 | Cl | 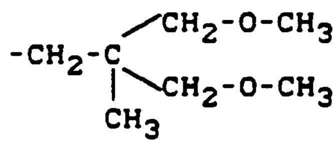 |
| 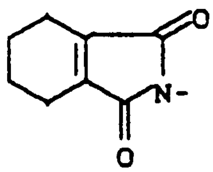 | Cl | 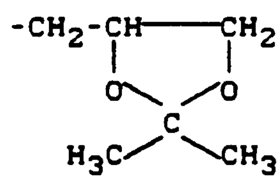 |
| 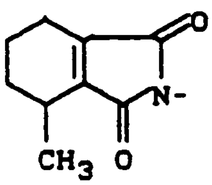 | F | $-CH_2CH_2-O-CH_2CH_2-O-CH_3$ |
| 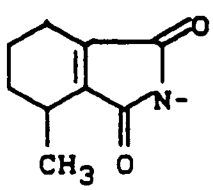 | H | $-CH_2CH_2-O-CH_2CH_2-O-C_2H_5$ |

<u>Tabelle 1</u> - Fortsetzung

| Het | X | R |
|---|---|---|
| 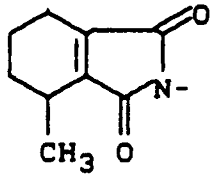 | Cl | $-CH_2CH_2-O-CH_2CH_2-O-CH_3$ |
| 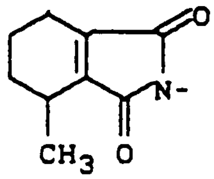 | F | $-CH_2CH_2-O-CH_2CH_2-O-C_4H_9$ |
| 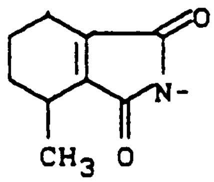 | Br | $-CH_2CH_2-O-CH_2CH_2-O-CH_3$ |
| 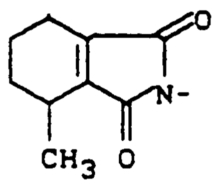 | Cl | 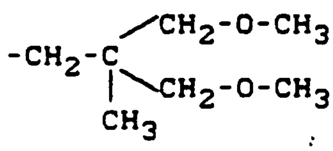 |
| 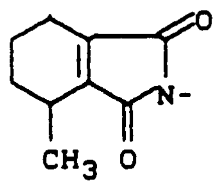 | Cl | 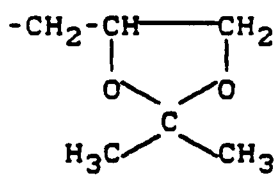 |
| 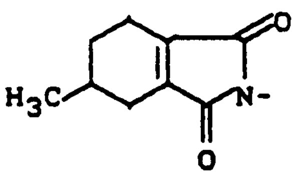 | F | $-CH_2CH_2-O-CH_2CH_2-O-CH_3$ |
| 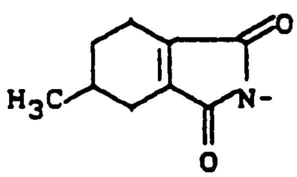 | H | $-CH_2CH_2-O-CH_2CH_2-O-C_2H_5$ |

Tabelle 1 - Fortsetzung

| Het | X | R |
|---|---|---|
| 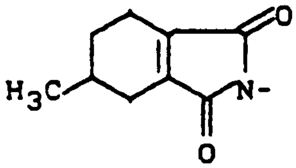 | Cl | $-CH_2CH_2-O-CH_2CH_2-O-C_2H_5$ |
| 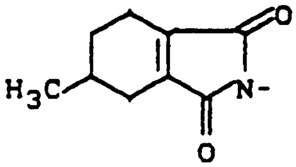 | F | $-CH_2CH_2-O-CH_2CH_2-O-C_3H_7$ |
| 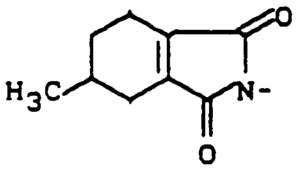 | Cl | $-CH_2CH_2-O-CH_2CH_2-O-C_4H_9$ |
| 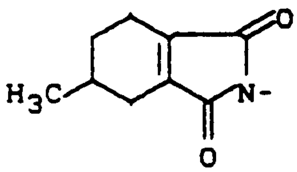 | Cl | 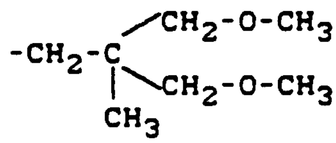 |
| 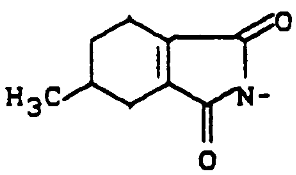 | Cl | 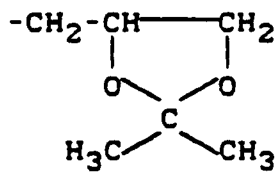 |
| 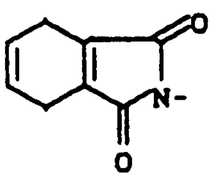 | F | $-CH_2CH_2-O-CH_2CH_2-O-CH_3$ |
| 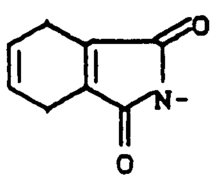 | H | $-CH_2CH_2-O-CH_2CH_2-O-CH_3$ |

**Tabelle 1** - Fortsetzung

| Het | X | R |
|---|---|---|
| 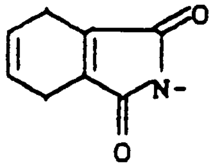 | Br | $-CH_2CH_2-O-CH_2CH_2-O-C_2H_5$ |
| 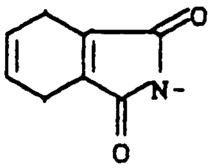 | Cl | $-CH_2CH_2-O-CH_2CH_2-O-CH_3$ |
| 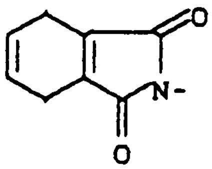 | Cl | $-CH_2CH_2-O-CH_2CH_2-O-C_2H_5$ |
| 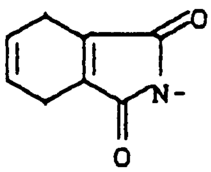 | Cl | 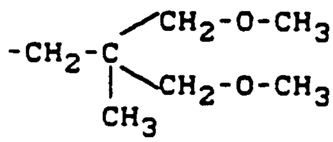 |
| 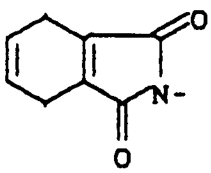 | Cl | 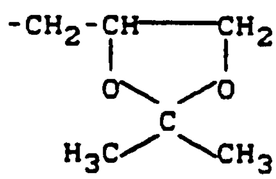 |
| 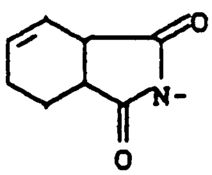 | F | $-CH_2CH_2-O-CH_2CH_2-O-CH_3$ |
| 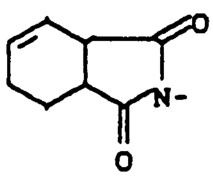 | H | $-CH_2CH_2-O-CH_2CH_2-O-C_2H_5$ |

Tabelle 1 - Fortsetzung

| Het | X | R |
|---|---|---|
| 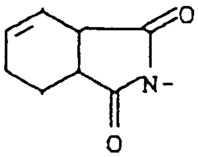 | Cl | $-CH_2CH_2-O-CH_2CH_2-O-CH_3$ |
| 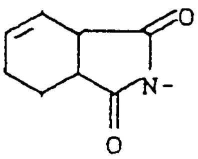 | Cl | $-CH_2CH_2-O-CH_2CH_2-O-C_2H_5$ |
| 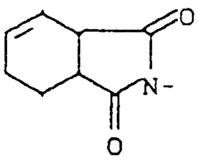 | Cl | $-CH_2CH_2-O-CH_2CH_2-O-C_4H_9$ |
| 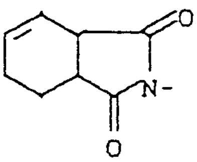 | Cl | 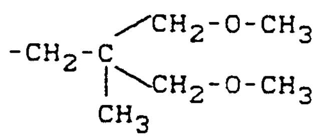 |
| 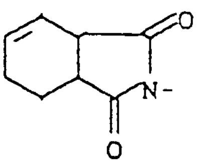 | Cl | 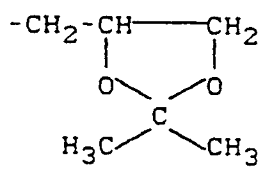 |
| 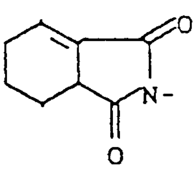 | F | $-CH_2CH_2-O-CH_2CH_2-O-CH_3$ |
| 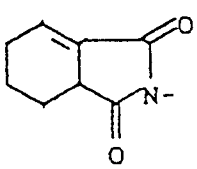 | H | $-CH_2CH_2-O-CH_2CH_2-O-C_2H_5$ |

Tabelle 1 - Fortsetzung

| Het | X | R |
|---|---|---|
| 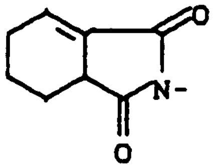 | Cl | $-CH_2CH_2-O-CH_2CH_2-O-CH_3$ |
| 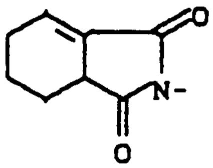 | Br | $-CH_2CH_2-O-CH_2CH_2-O-CH_3$ |
| 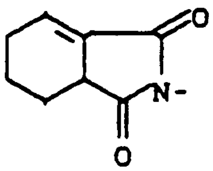 | Cl | $-CH_2CH_2-O-CH_2CH_2-O-C_2H_5$ |
| 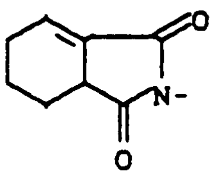 | Cl | 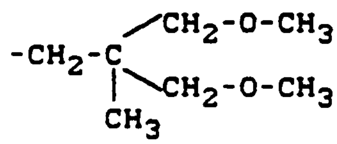 |
| 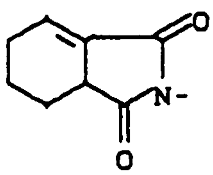 | Cl | 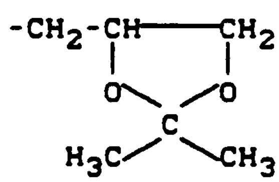 |
| 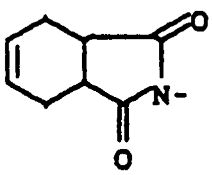 | F | $-CH_2CH_2-O-CH_2CH_2-O-CH_3$ |
| 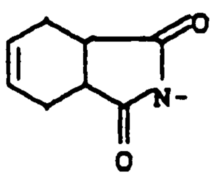 | H | $-CH_2CH_2-O-CH_2CH_2-O-C_2H_5$ |

Tabelle 1 - Fortsetzung

| Het | X | R |
|---|---|---|
| 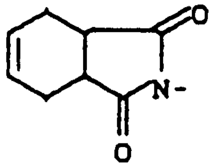 | Cl | $-CH_2CH_2-O-CH_2CH_2-O-CH_3$ |
| 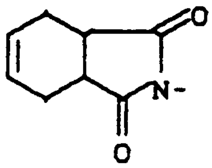 | Cl | $-CH_2CH_2-O-CH_2CH_2-O-C_2H_5$ |
| 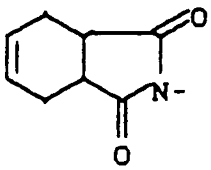 | Br | $-CH_2CH_2-O-CH_2CH_2-O-CH_3$ |
| 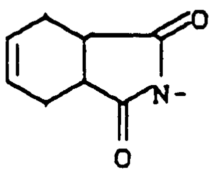 | Cl | 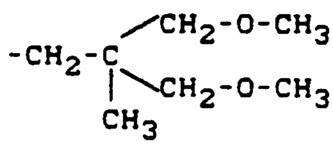 |
| 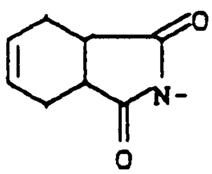 | Cl | 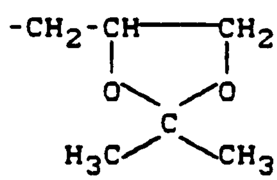 |
| 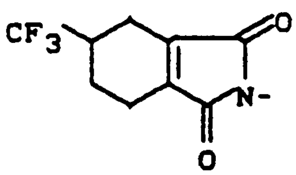 | F | $-CH_2CH_2-O-CH_2CH_2-O-CH_3$ |
| 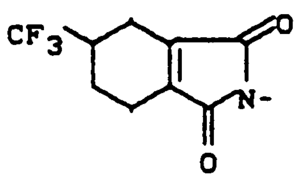 | H | $-CH_2CH_2-O-CH_2CH_2-O-C_2H_5$ |

Tabelle 1 - Fortsetzung

| Het | X | R |
|---|---|---|
| 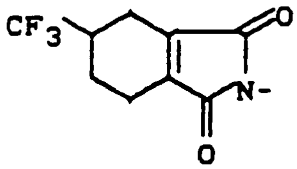 | Cl | $-CH_2CH_2-O-CH_2CH_2-O-CH_3$ |
| 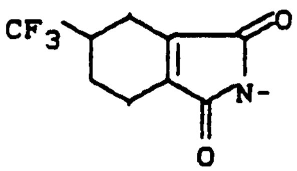 | Cl | $-CH_2CH_2-O-CH_2CH_2-O-C_2H_5$ |
| 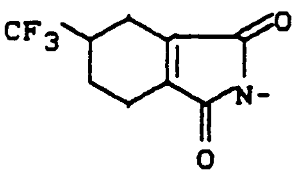 | Br | $-CH_2CH_2-O-CH_2CH_2-O-CH_3$ |
| 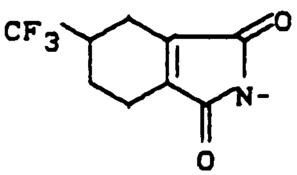 | Cl | 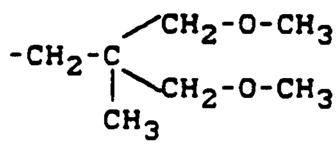 |
| 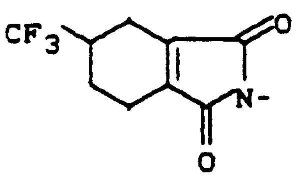 | Cl | 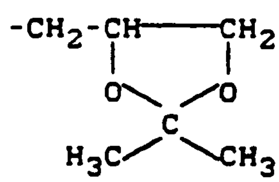 |
| 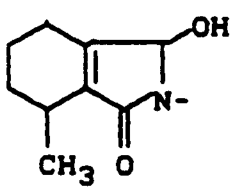 | F | $-CH_2CH_2-O-CH_2CH_2-O-CH_3$ |
| 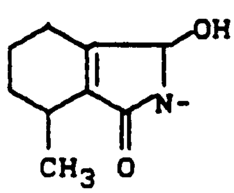 | H | $-CH_2CH_2-O-CH_2CH_2-O-C_2H_5$ |

**Tabelle 1** - Fortsetzung

| Het | X | R |
|---|---|---|
| 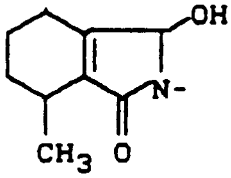 | Cl | $-CH_2CH_2-O-CH_2CH_2-O-CH_3$ |
| 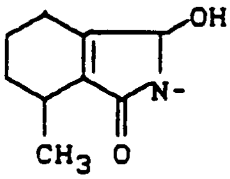 | Cl | $-CH_2CH_2-O-CH_2CH_2-O-C_2H_5$ |
| 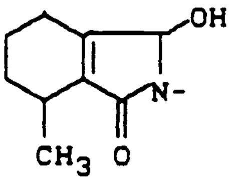 | Br | $-CH_2CH_2-O-CH_2CH_2-O-CH_3$ |
| 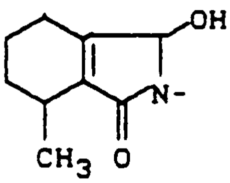 | Cl | 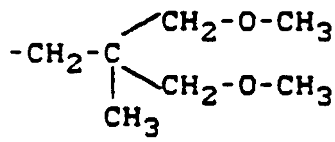 |
| 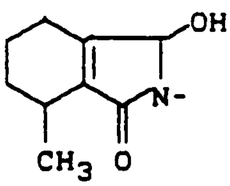 | Cl | 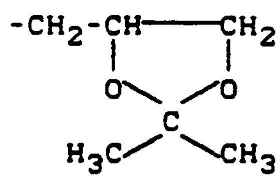 |
| 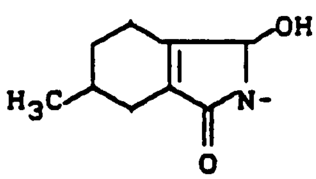 | F | $-CH_2CH_2-O-CH_2CH_2-O-CH_3$ |
| 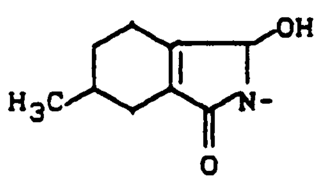 | H | $-CH_2CH_2-O-CH_2CH_2-O-C_2H_5$ |

Tabelle 1 - Fortsetzung

| Het | X | R |
|---|---|---|
| 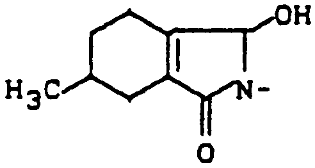 | Cl | $-CH_2CH_2-O-CH_2CH_2-O-CH_3$ |
| 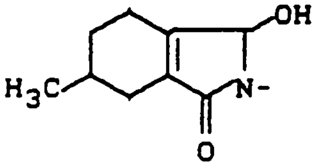 | Cl | $-CH_2CH_2-O-CH_2CH_2-O-C_2H_5$ |
| 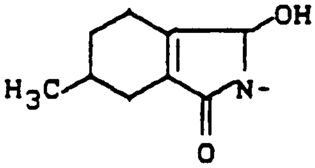 | Br | $-CH_2CH_2-O-CH_2CH_2-O-CH_3$ |
| 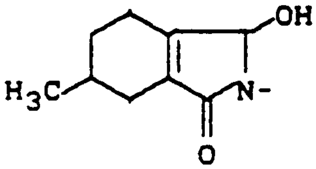 | Cl | 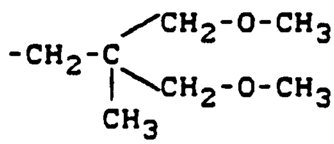 |
| 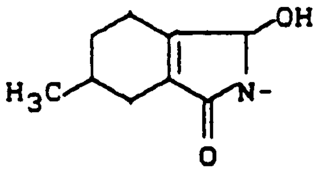 | Cl | 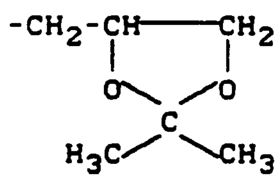 |
| 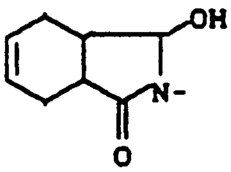 | F | $-CH_2CH_2-O-CH_2CH_2-O-CH_3$ |
| 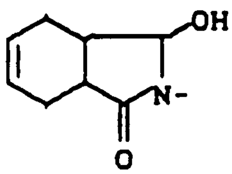 | H | $-CH_2CH_2-O-CH_2CH_2-O-C_2H_5$ |

Tabelle 1 - Fortsetzung

| Het | X | R |
|---|---|---|
| 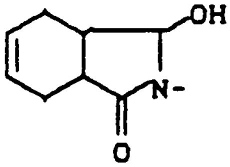 | Cl | $-CH_2CH_2-O-CH_2CH_2-O-CH_3$ |
| 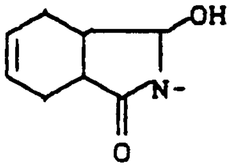 | Cl | $-CH_2CH_2-O-CH_2CH_2-O-C_2H_5$ |
| 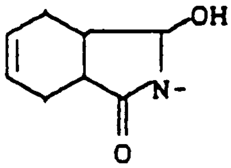 | Br | $-CH_2CH_2-O-CH_2CH_2-O-CH_3$ |
| 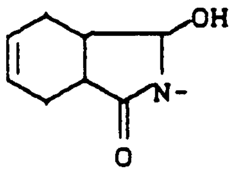 | Cl | 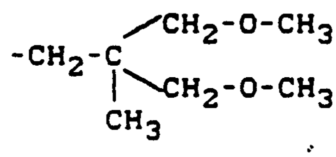 |
| 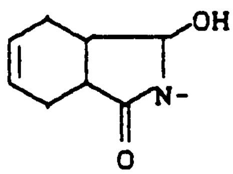 | Cl | 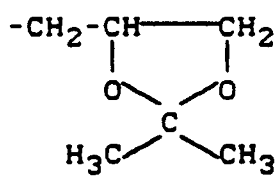 |
| 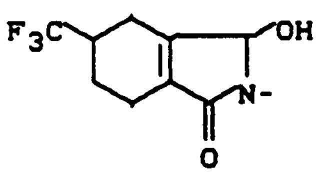 | F | $-CH_2CH_2-O-CH_2CH_2-O-CH_3$ |
| 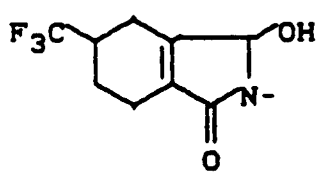 | H | $-CH_2CH_2-O-CH_2CH_2-O-C_2H_5$ |

Tabelle 1 - Fortsetzung

| Het | X | R |
|---|---|---|
| 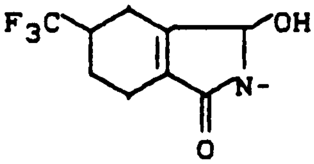 | Cl | $-CH_2CH_2-O-CH_2CH_2-O-CH_3$ |
| 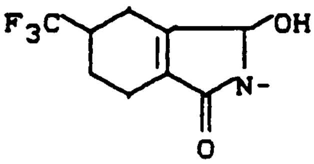 | Cl | $-CH_2CH_2-O-CH_2CH_2-O-C_2H_5$ |
| 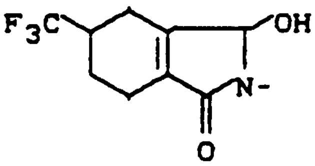 | Br | $-CH_2CH_2-O-CH_2CH_2-O-CH_3$ |
| 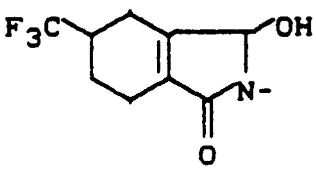 | Cl | 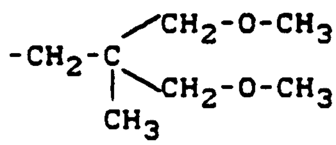 |
| 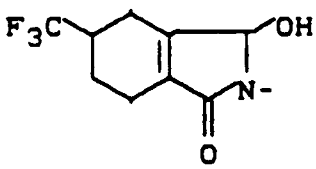 | Cl | 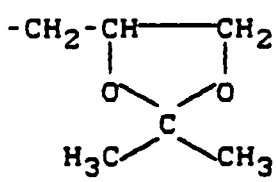 |
| 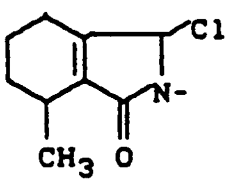 | F | $-CH_2CH_2-O-CH_2CH_2-O-C_2H_5$ |
| 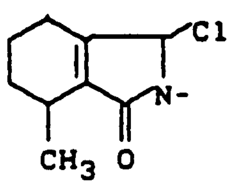 | H | $-CH_2CH_2-O-CH_2CH_2-O-CH_3$ |

Tabelle 1 - Fortsetzung

| Het | X | R |
|---|---|---|
| 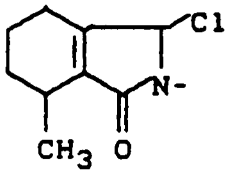 | Cl | $-CH_2CH_2-O-CH_2CH_2-O-CH_3$ |
| 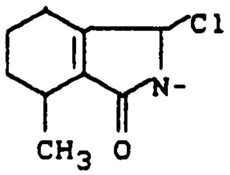 | Cl | $-CH_2CH_2-O-CH_2CH_2-O-C_2H_5$ |
| 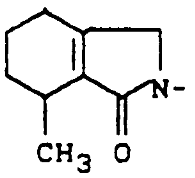 | F | $-CH_2CH_2-O-CH_2CH_2-O-C_2H_5$ |
| 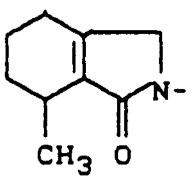 | Cl | $-CH_2CH_2-O-CH_2CH_2-O-C_2H_5$ |
| 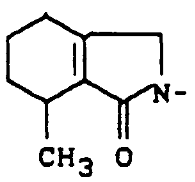 | Br | $-CH_2CH_2-O-CH_2CH_2-O-CH_3$ |
| 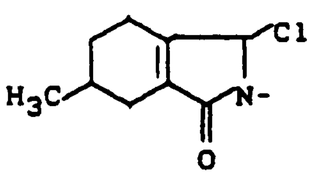 | F | $-CH_2CH_2-O-CH_2CH_2-O-C_2H_5$ |
| 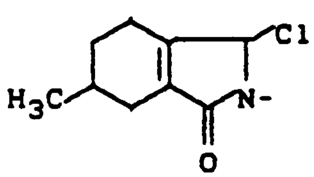 | Cl | $-CH_2CH_2-O-CH_2CH_2-O-CH_3$ |

Tabelle 1 - Fortsetzung

| Het | X | R |
|---|---|---|
| 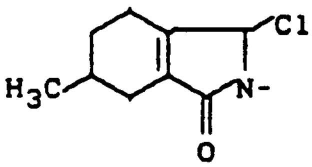 | Cl | $-CH_2CH_2-O-CH_2CH_2-O-C_2H_5$ |
| 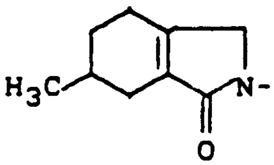 | F | $-CH_2CH_2-O-CH_2CH_2-O-C_2H_5$ |
| 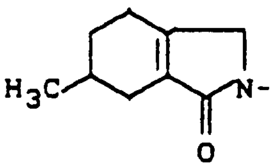 | Cl | $-CH_2CH_2-O-CH_2CH_2-O-C_2H_5$ |
| 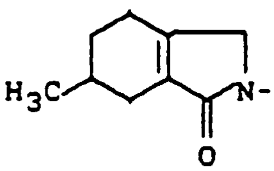 | Br | $-CH_2CH_2-O-CH_2CH_2-O-CH_3$ |
| 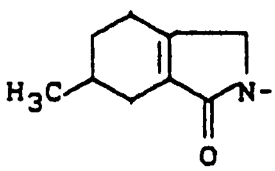 | Cl | 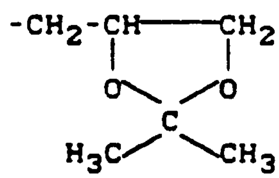 |
| 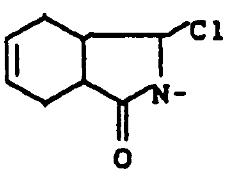 | F | $-CH_2CH_2-O-CH_2CH_2-O-C_2H_5$ |
| 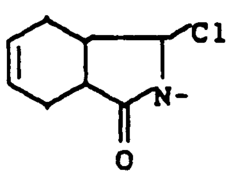 | H | $-CH_2CH_2-O-CH_2CH_2-O-C_4H_9$ |

Tabelle 1 - Fortsetzung

| Het | X | R |
| --- | --- | --- |
| 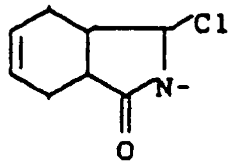 | Cl | $-CH_2CH_2-O-CH_2CH_2-O-CH_3$ |
| 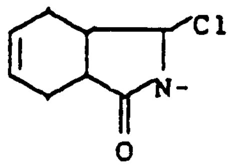 | Cl | $-CH_2CH_2-O-CH_2CH_2-O-C_2H_5$ |
| 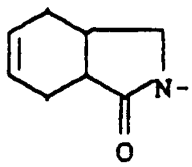 | F | $-CH_2CH_2-O-CH_2CH_2-O-C_4H_9$ |
| 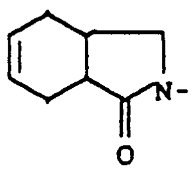 | Cl | $-CH_2CH_2-O-CH_2CH_2-O-CH_3$ |
| 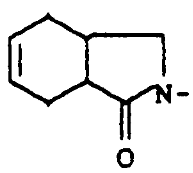 | Cl | $-CH_2CH_2-O-CH_2CH_2-O-C_2H_5$ |
| 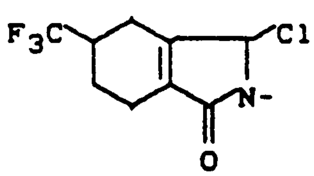 | F | $-CH_2CH_2-O-CH_2CH_2-O-C_3H_7$ |
| 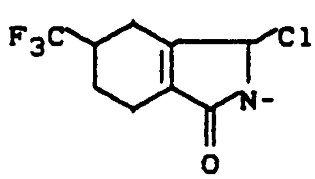 | H | $-CH_2CH_2-O-CH_2CH_2-O-C_4H_9$ |

Tabelle 1 - Fortsetzung

| Het | X | R |
|---|---|---|
| 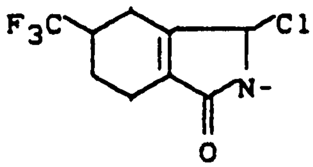 | Cl | $-CH_2CH_2-O-CH_2CH_2-O-CH_3$ |
| 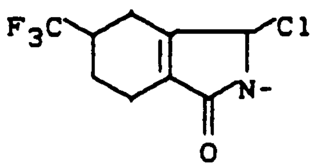 | Cl | $-CH_2CH_2-O-CH_2CH_2-O-C_2H_5$ |
| 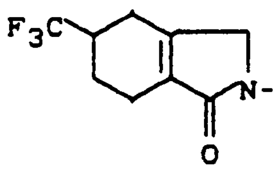 | F | $-CH_2CH_2-O-CH_2CH_2-O-C_2H_5$ |
| 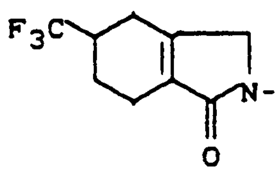 | Cl | $-CH_2CH_2-O-CH_2CH_2-O-CH_3$ |
| 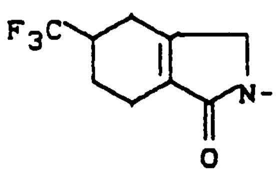 | Cl | $-CH_2CH_2-O-CH_2CH_2-O-C_2H_5$ |
| 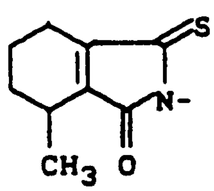 | F | $-CH_2CH_2-O-CH_2CH_2-O-CH_3$ |
| 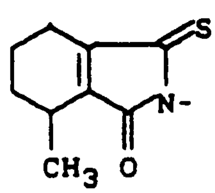 | H | $-CH_2CH_2-O-CH_2CH_2-O-CH_3$ |

Tabelle 1 - Fortsetzung

| Het | X | R |
|---|---|---|
| 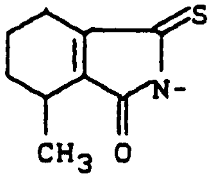 | Cl | $-CH_2CH_2-O-CH_2CH_2-O-CH_3$ |
| 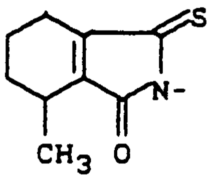 | Cl | $-CH_2CH_2-O-CH_2CH_2-O-C_2H_5$ |
| 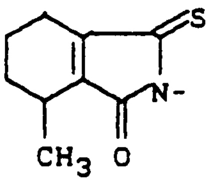 | Cl | $-CH_2CH_2-O-CH_2CH_2-O-C_3H_7$ |
| 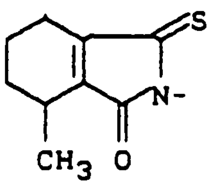 | Cl | 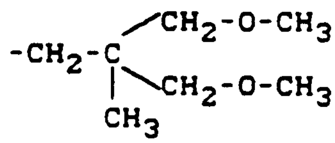 |
| 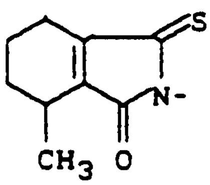 | Cl | 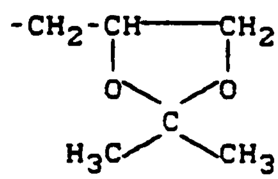 |
| 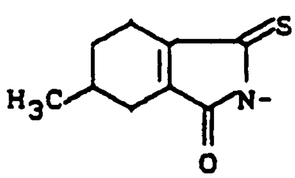 | F | $-CH_2CH_2-O-CH_2CH_2-O-C_2H_5$ |
| 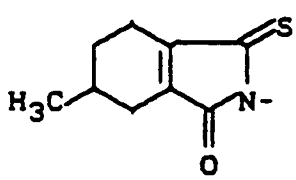 | H | $-CH_2CH_2-O-CH_2CH_2-O-C_4H_9$ |

Tabelle 1 - Fortsetzung

| Het | X | R |
|---|---|---|
| 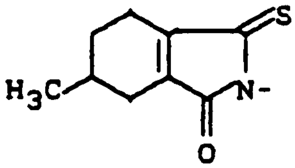 | Cl | $-CH_2CH_2-O-CH_2CH_2-O-CH_3$ |
| 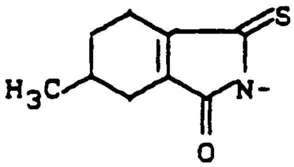 | Cl | $-CH_2CH_2-O-CH_2CH_2-O-C_2H_5$ |
| 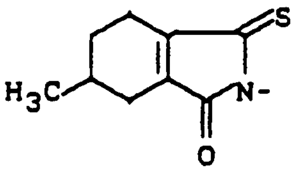 | Cl | $-CH_2CH_2-O-CH_2CH_2-O-C_3H_7$ |
| 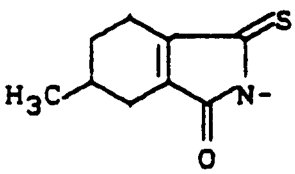 | Cl | 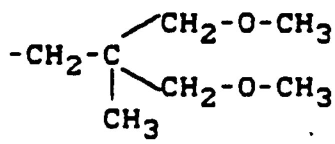 |
| 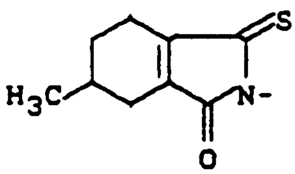 | Cl | 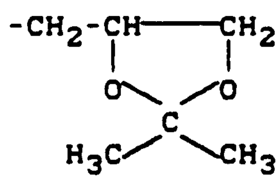 |
| 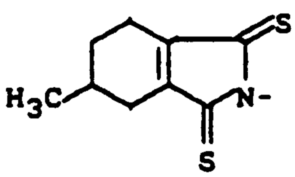 | F | $-CH_2CH_2-O-CH_2CH_2-O-C_3H_7$ |
| 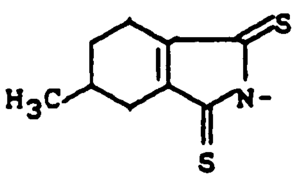 | H | $-CH_2CH_2-O-CH_2CH_2-O-C_4H_9$ |

Tabelle 1 - Fortsetzung

| Het | X | R |
|---|---|---|
| 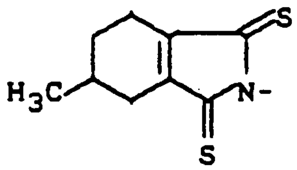 | Cl | $-CH_2CH_2-O-CH_2CH_2-O-CH_3$ |
| 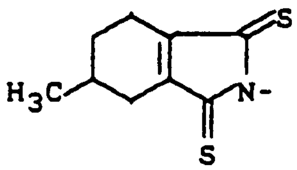 | Cl | $-CH_2CH_2-O-CH_2CH_2-O-C_2H_5$ |
| 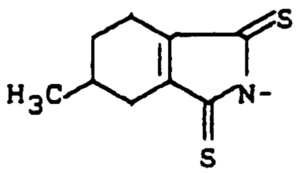 | Cl | $-CH_2CH_2-O-CH_2CH_2-O-C_3H_7$ |
| 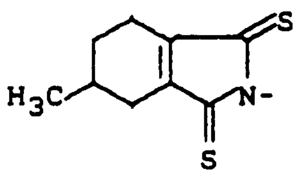 | Cl | 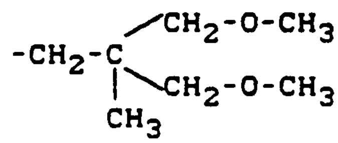 |
| 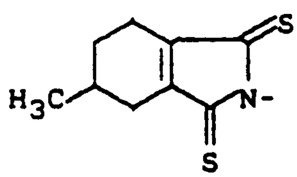 | Cl | 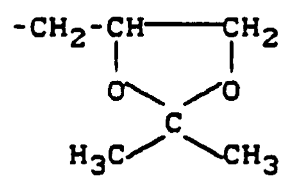 |
| 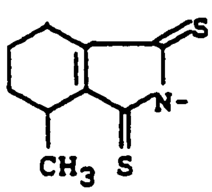 | F | $-CH_2CH_2-O-CH_2CH_2-O-C_2H_5$ |
| 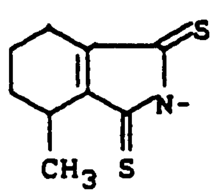 | H | $-CH_2CH_2-O-CH_2CH_2-O-C_4H_9$ |

Tabelle 1 - Fortsetzung

| Het | X | R |
|---|---|---|
| 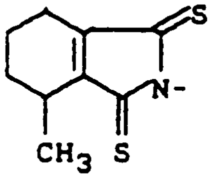 | Cl | $-CH_2CH_2-O-CH_2CH_2-O-CH_3$ |
| 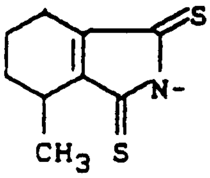 | Cl | $-CH_2CH_2-O-CH_2CH_2-O-C_2H_5$ |
| 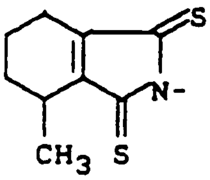 | Cl | $-CH_2CH_2-O-CH_2CH_2-O-C_3H_7$ |
| 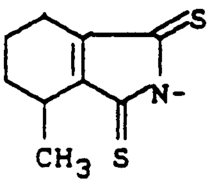 | Cl | 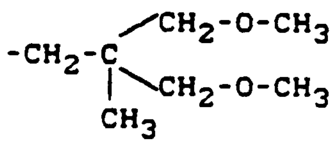 |
| 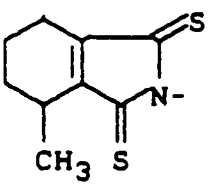 | Cl | 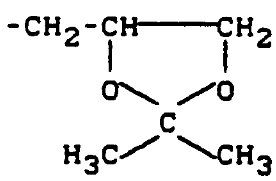 |
| 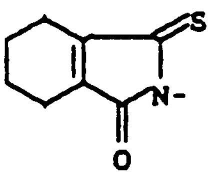 | F | $-CH_2CH_2-O-CH_2CH_2-O-C_2H_5$ |
| 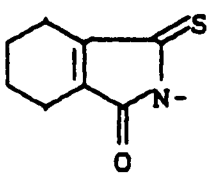 | H | $-CH_2CH_2-O-CH_2CH_2-O-C_3H_7$ |

<u>Tabelle 1</u> - Fortsetzung

| Het | X | R |
|---|---|---|
| 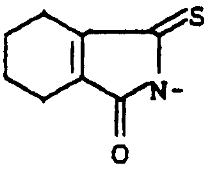 | Cl | $-CH_2CH_2-O-CH_2CH_2-O-CH_3$ |
| 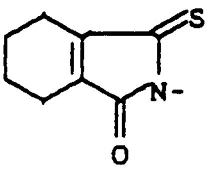 | Cl | $-CH_2CH_2-O-CH_2CH_2-O-C_2H_5$ |
| 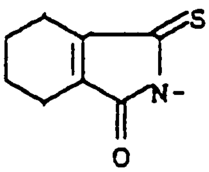 | Cl | $-CH_2CH_2-O-CH_2CH_2-O-C_3H_7$ |
| 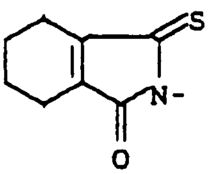 | Cl | 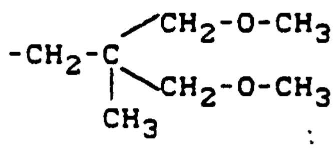 |
| 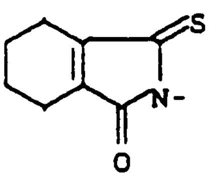 | Cl | 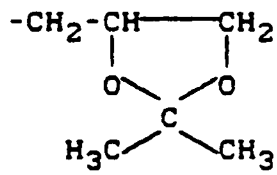 |
| 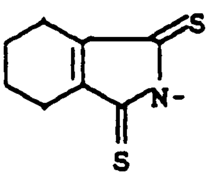 | Cl | $-CH_2CH_2-O-CH_2CH_2-O-CH_3$ |
| 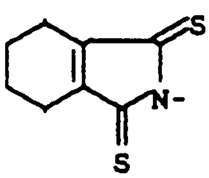 | Cl | $-CH_2CH_2-O-CH_2CH_2-O-C_2H_5$ |

Tabelle 1 - Fortsetzung

| Het | X | R |
|---|---|---|
| 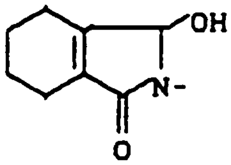 | H | $-CH_2CH_2-O-CH_2CH_2-O-C_2H_5$ |
| 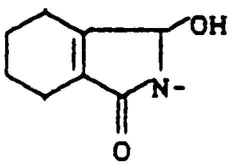 | F | $-CH_2CH_2-O-CH_2CH_2-O-C_4H_9$ |
| 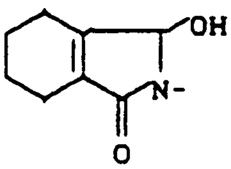 | Cl | $-CH_2CH_2-O-CH_2CH_2-O-CH_3$ |
| 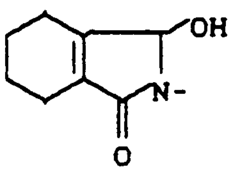 | Cl | $-CH_2CH_2-O-CH_2CH_2-O-C_2H_5$ |
| 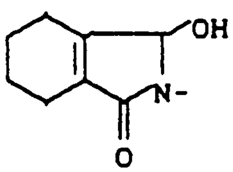 | Cl | $-CH_2CH_2-O-CH_2CH_2-O-C_3H_7$ |
| 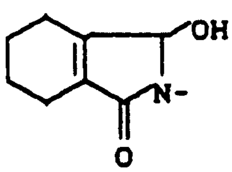 | Cl | 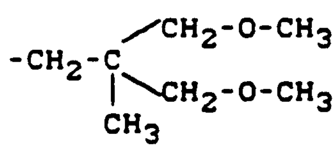 |
| 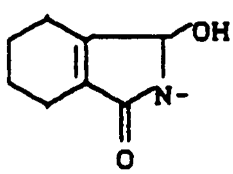 | Cl | 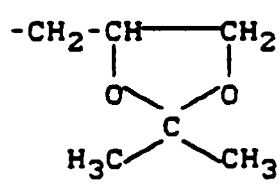 |

Tabelle 1 - Fortsetzung

| Het | X | R |
|---|---|---|
| 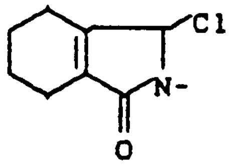 | H | $-CH_2CH_2-O-CH_2CH_2-O-C_4H_9$ |
| 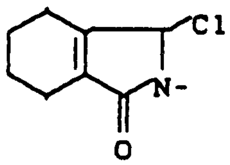 | H | $-CH_2CH_2-O-CH_2CH_2-O-C_2H_5$ |
| 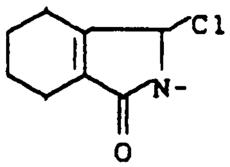 | F | $-CH_2CH_2-O-CH_2CH_2-O-C_2H_5$ |
| 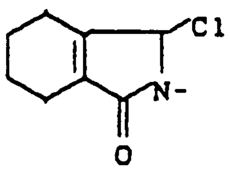 | Cl | $-CH_2CH_2-O-CH_2CH_2-O-CH_3$ |
| 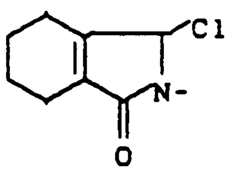 | Cl | $-CH_2CH_2-O-CH_2CH_2-O-C_2H_5$ |
| 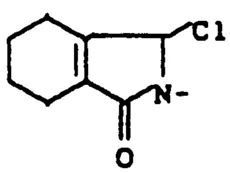 | Cl | $-CH_2CH_2-O-CH_2CH_2-O-C_3H_7$ |
| 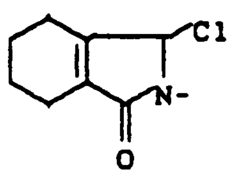 | Cl | 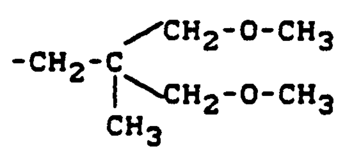 |

Tabelle 1 - Fortsetzung

| Het | X | R |
|---|---|---|
| 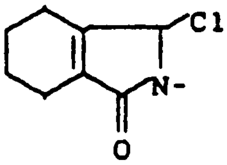 | Cl | 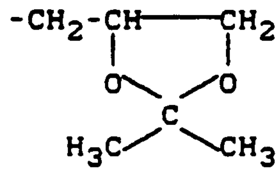 |
| 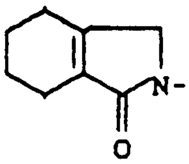 | H | $-CH_2CH_2-O-CH_2CH_2-O-C_3H_7$ |
| 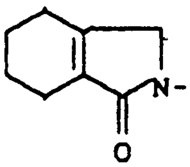 | F | $-CH_2CH_2-O-CH_2CH_2-O-C_4H_9$ |
| 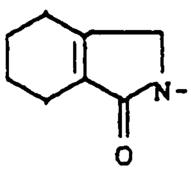 | Cl | $-CH_2CH_2-O-CH_2CH_2-O-CH_3$ |
| 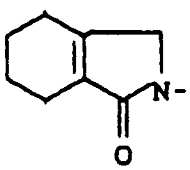 | Cl | $-CH_2CH_2-O-CH_2CH_2-O-C_2H_5$ |
| 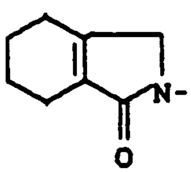 | Cl | 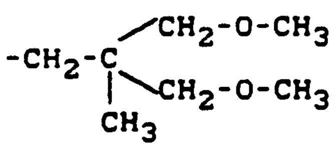 |
| 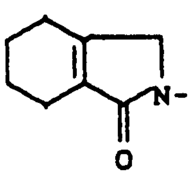 | Cl | 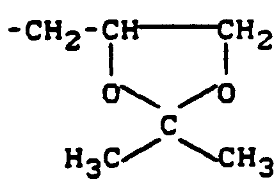 |

Tabelle 1 - Fortsetzung

| Het | X | R |
|---|---|---|
| 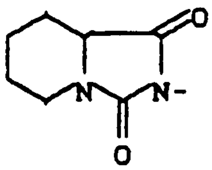 | H | $-CH_2CH_2-O-CH_2CH_2-O-C_2H_5$ |
| 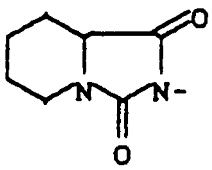 | H | $-CH_2CH_2-O-CH_2CH_2-O-C_4H_9$ |
| 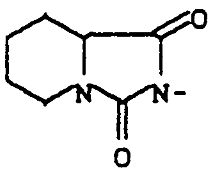 | F | $-CH_2CH_2-O-CH_2CH_2-O-C_2H_5$ |
| 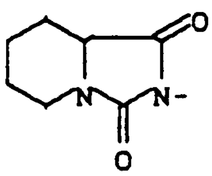 | F | $-CH_2CH_2-O-CH_2CH_2-O-CH_3$ |
| 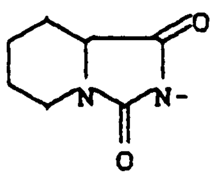 | Cl | $-CH_2CH_2-O-CH_2CH_2-O-CH_3$ |
| 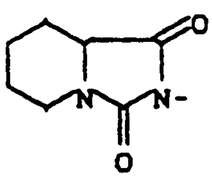 | Cl | $-CH_2CH_2-O-CH_2CH_2-O-C_2H_5$ |
| 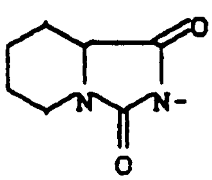 | Cl | $-CH_2CH_2-O-CH_2CH_2-O-C_3H_7$ |

Tabelle 1 - Fortsetzung

| Het | X | R |
|---|---|---|
| 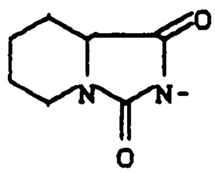 | Cl | $-CH_2CH_2-O-CH_2CH_2-O-C_4H_9$ |
| 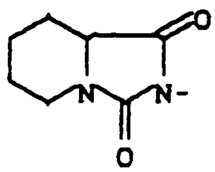 | Br | $-CH_2CH_2-O-CH_2CH_2-O-C_2H_5$ |
| 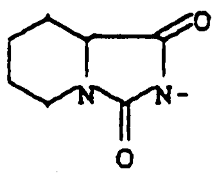 | Br | $-CH_2CH_2-O-CH_2CH_2-O-CH_3$ |
| 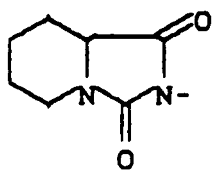 | Cl | 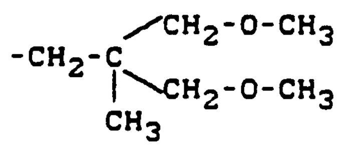 |
| 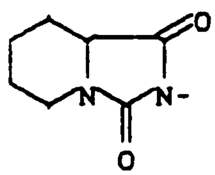 | Cl | 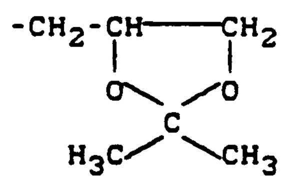 |
| 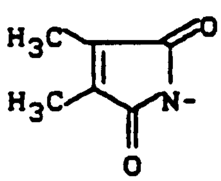 | H | $-CH_2CH_2-O-CH_2CH_2-O-C_2H_5$ |
| 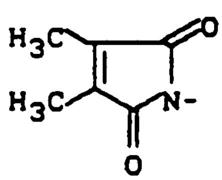 | F | $-CH_2CH_2-O-CH_2CH_2-O-C_2H_5$ |

<u>Tabelle 1</u> - Fortsetzung

| Het | X | R |
|---|---|---|
| 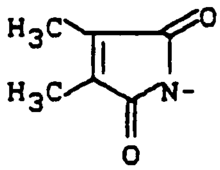 | Cl | $-CH_2CH_2-O-CH_2CH_2-O-CH_3$ |
| 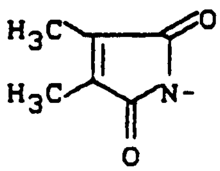 | Cl | $-CH_2CH_2-O-CH_2CH_2-O-C_2H_5$ |
| 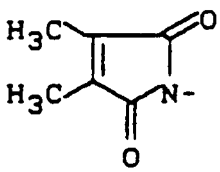 | Cl | $-CH_2CH_2-O-CH_2CH_2-O-C_3H_7$ |
| 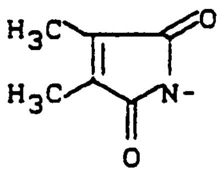 | Cl | $-CH_2CH_2-O-CH_2CH_2-O-C_4H_9$ |
| 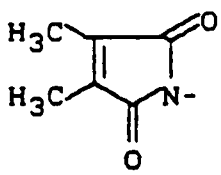 | Br | $-CH_2CH_2-O-CH_2CH_2-O-CH_3$ |
| 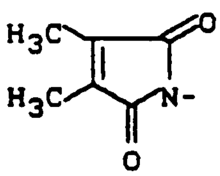 | Br | $-CH_2CH_2-O-CH_2CH_2-O-C_2H_5$ |
| 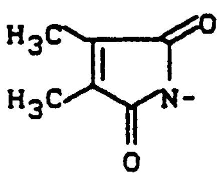 | Cl | 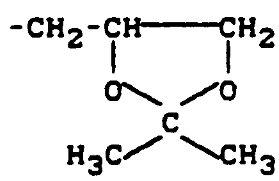 |

Tabelle 1 - Fortsetzung

| Het | X | R |
|---|---|---|
| 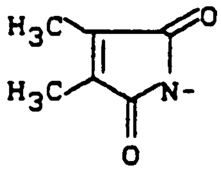 | Cl | 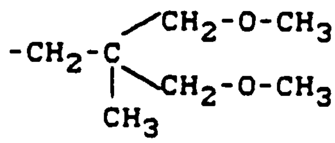 |
| 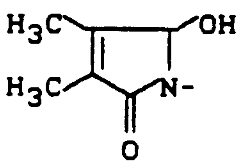 | H | $-CH_2CH_2-O-CH_2CH_2-O-C_2H_5$ |
| 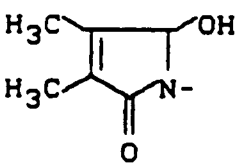 | F | $-CH_2CH_2-O-CH_2CH_2-O-C_2H_5$ |
| 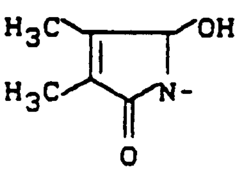 | Cl | $-CH_2CH_2-O-CH_2CH_2-O-CH_3$ |
| 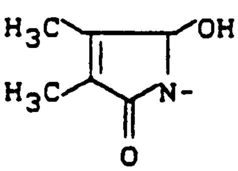 | Cl | $-CH_2CH_2-O-CH_2CH_2-O-C_2H_5$ |
| 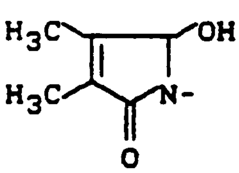 | Cl | $-CH_2CH_2-O-CH_2CH_2-O-C_3H_7$ |
| 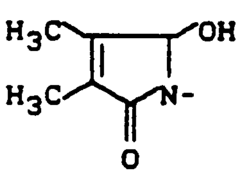 | Cl | $-CH_2CH_2-O-CH_2CH_2-O-C_4H_9$ |

<u>Tabelle 1</u> - Fortsetzung

| Het | X | R |
|---|---|---|
| 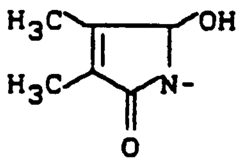 | Cl | 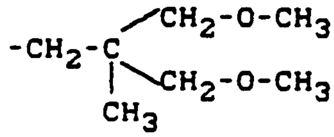 |
| 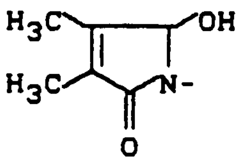 | Cl | 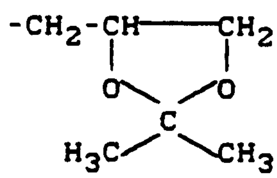 |
| 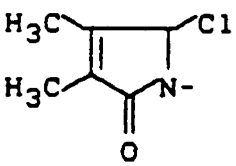 | H | $-CH_2CH_2-O-CH_2CH_2-O-CH_3$ |
| 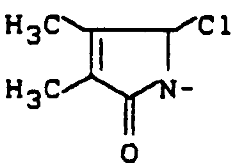 | F | $-CH_2CH_2-O-CH_2CH_2-O-C_4H_9$ |
| 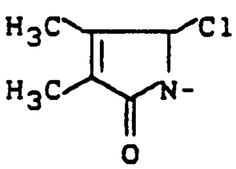 | Cl | $-CH_2CH_2-O-CH_2CH_2-O-CH_3$ |
| 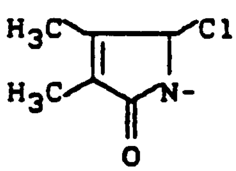 | Cl | $-CH_2CH_2-O-CH_2CH_2-O-C_2H_5$ |
| 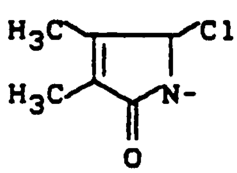 | Cl | $-CH_2CH_2-O-CH_2CH_2-O-C_4H_9$ |

Tabelle 1 - Fortsetzung

| Het | X | R |
|---|---|---|
| 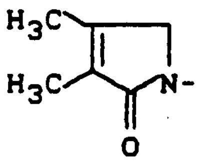 | H | $-CH_2CH_2-O-CH_2CH_2-O-C_4H_9$ |
| 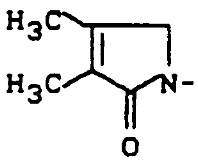 | F | $-CH_2CH_2-O-CH_2CH_2-O-C_2H_5$ |
| 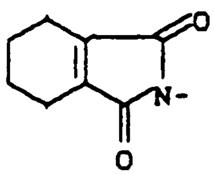 | Cl | $-CH_2-\underset{CH_3}{\underset{\vert}{CH}}-O-C_2H_5$ |
| 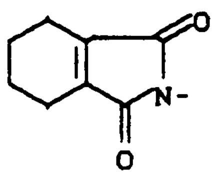 | Br | $-CH_2-\overset{CH_3}{\overset{\vert}{\underset{CH_3}{\underset{\vert}{C}}}}-O-C_2H_5$ |
| 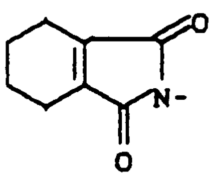 | H | $-CH_2-\underset{CH_3}{\underset{\vert}{CH}}-O-CH_2-CH_3-O-C_2H_5$ |
| 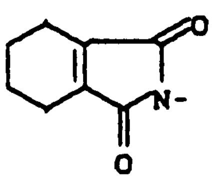 | Br | $-CH_2-\overset{CH_3}{\overset{\vert}{\underset{CH_3}{\underset{\vert}{C}}}}-O-CH(CH_3)_2$ |
| 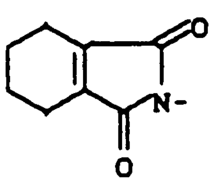 | H | $-CH_2-\overset{CH_3}{\overset{\vert}{\underset{CH_3}{\underset{\vert}{C}}}}-O-CH_3$ |

**Tabelle 1** - Fortsetzung

| Het | X | R |
|---|---|---|
| 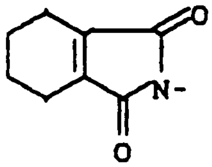 | Cl | 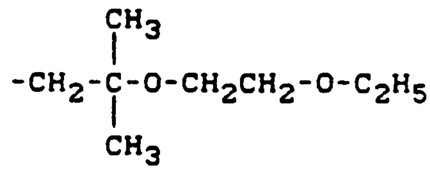 |
| 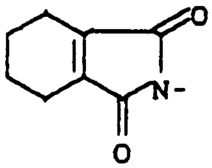 | Cl | 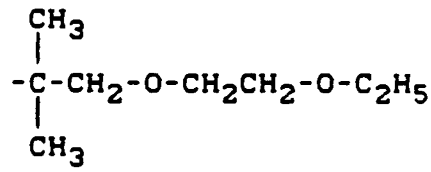 |
| 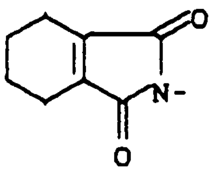 | Cl | 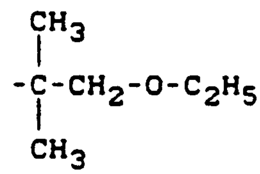 |
| 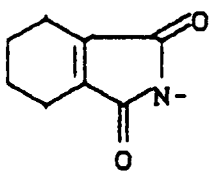 | Cl | 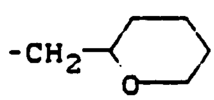 |
| 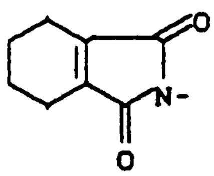 | Cl | 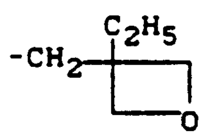 |
| 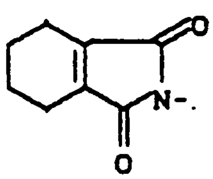 | Cl | 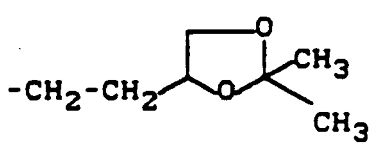 |
| 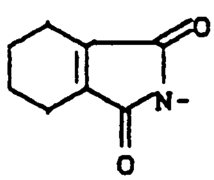 | Cl | $-CH_2-C(CH_2-O-CH_3)_3$ |

**Tabelle 1** - Fortsetzung

| Het | X | R |
|---|---|---|
| 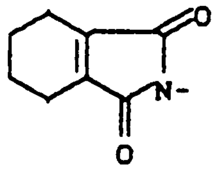 | Br | $-CH_2-C(CH_2-O-C_2H_5)_3$ |
| 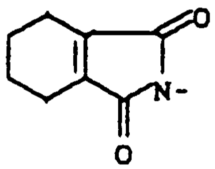 | F | 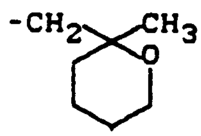 |
| 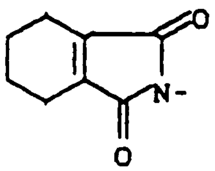 | F | 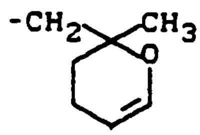 |
| 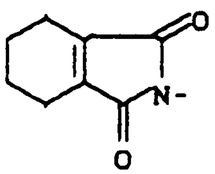 | Cl | 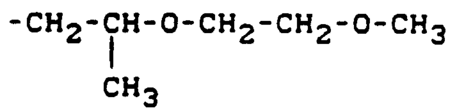 |
| 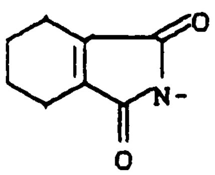 | Br | $-CH_2-CH-OCH_2CH_2-OCH_2CH_2-O-C_2H_5$ |
| 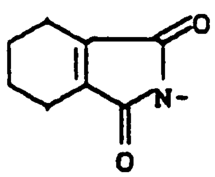 | Cl | 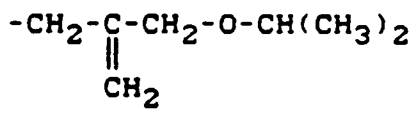 |
| 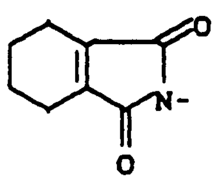 | Cl | 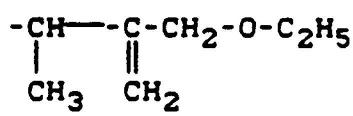 |

<u>Tabelle 1</u> - Fortsetzung

| Het | X | R |
|---|---|---|
| 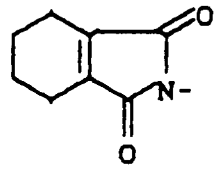 | Cl | 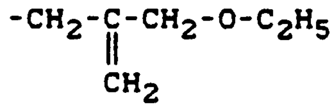 |
| 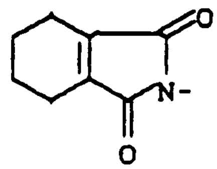 | H | 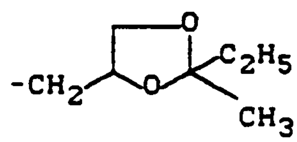 |
| 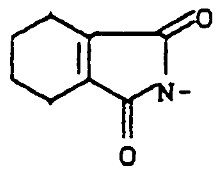 | Cl | 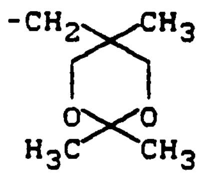 |
| 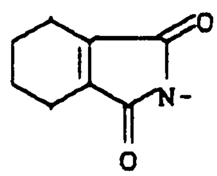 | Br | 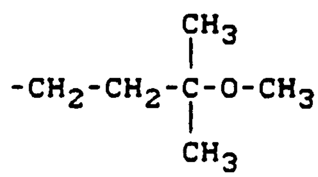 |
| 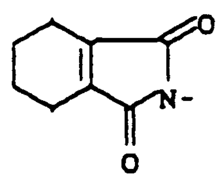 | F | 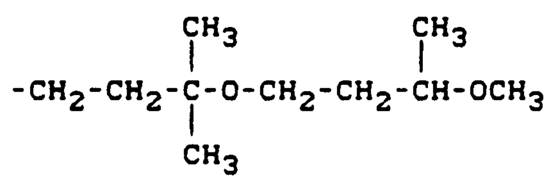 |
| 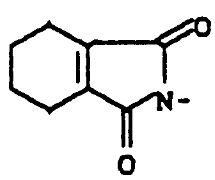 | Cl | 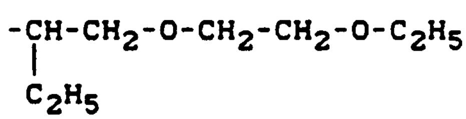 |
| 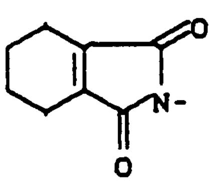 | F | 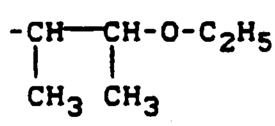 |

Tabelle 1 - Fortsetzung

| Het | X | R |
|---|---|---|
| 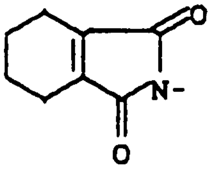 | Cl | $-CH-CH-O-CH_2-CH_2-O-C_2H_5$<br>$CH_3$ $CH_3$ |
| 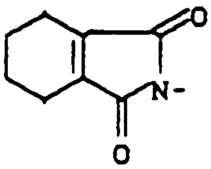 | Cl | $-CH_2-CH_2-O-CH-CH_2-O-C_2H_5$<br>$CH_3$ |
| 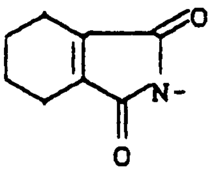 | Cl | $-CH_2-CH_2-CH-O-CH_3$<br>$CH_3$ |
| 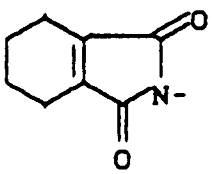 | Br | $-CH_2-CH-CH_2-O-CH_3$<br>$CH_3$ |
| 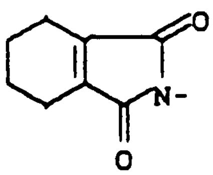 | Cl | $-CH_2-CH-O-(CH_2)_2-CH_3$<br>$CH_3$ |
| 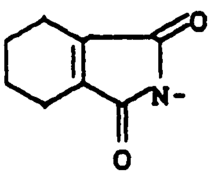 | Cl | $-CH_2-CH-O-(CH_2)_4-CH_3$<br>$CH_3$ |
| 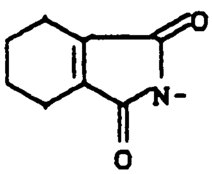 | Br | $-CH-CH_2-O-CH(CH_3)_2$<br>$CH_3$ |

Tabelle 1 - Fortsetzung

| Het | X | R |
|---|---|---|
| 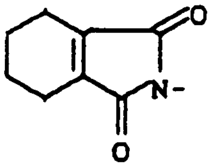 | F | $-CH-CH_2-O-C_2H_5$<br>\|<br>$CH_3$ |
| 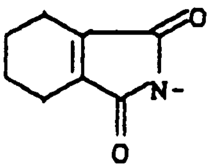 | Cl | $-O-CH-CH_2-O-C_2H_5$<br>\|<br>$C_2H_5$ |
| 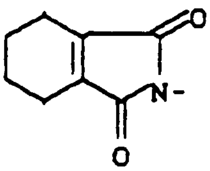 | Cl | $-CH-CH_2-O-CH_2-CH_2-O-C_2H_5$<br>\|<br>$CH_3$ |
| 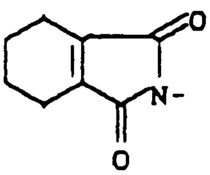 | Cl | $CH_3$<br>\|<br>$-CH_2-C-CH_2-O-CH_3$<br>\|<br>$CH_3$ |
| 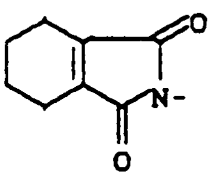 | Cl | $CH_3$<br>\|<br>$-CH_2-C-CH_2-O-C_2H_5$<br>\|<br>$CH_3$ |
| 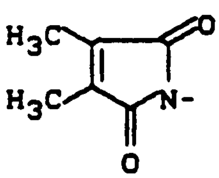 | Br | $-CH_2-CH_2-CH-O-CH_3$<br>\|<br>$CH_3$ |
| 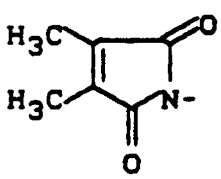 | Br | $-CH_2-CH-CH_2-O-CH_3$<br>\|<br>$CH_3$ |

Tabelle 1 - Fortsetzung

| Het | X | R |
|---|---|---|
| 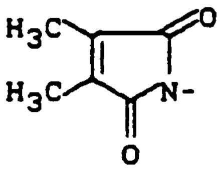 | Cl | $-CH_2-CH-O-(CH_2)_2-CH_3$<br>$CH_3$ |
| 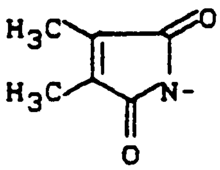 | Cl | $-CH_2-CH-O-(CH_2)_4-CH_3$ |
| 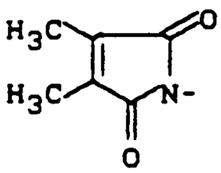 | Br | $-CH-CH_2-O-CH(CH_3)_2$<br>$CH_3$ |
| 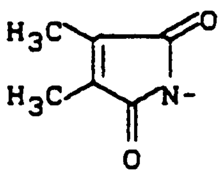 | F | $-CH-CH_2-O-C_2H_5$<br>$CH_3$ |
| 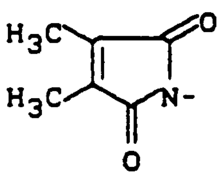 | Cl | $-CH-CH_2-O-C_2H_5$<br>$C_2H_5$ |
| 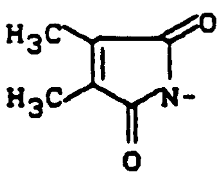 | Cl | $-CH-CH_2-O-CH_2-CH_2-O-C_2H_5$<br>$CH_3$ |
| 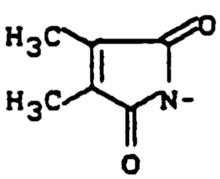 | Cl | $CH_3$<br>$-CH_2-C-CH_2-O-CH_3$<br>$CH_3$ |

Tabelle 1 - Fortsetzung

| Het | X | R |
|---|---|---|
| 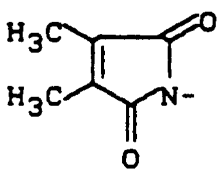 | Cl | 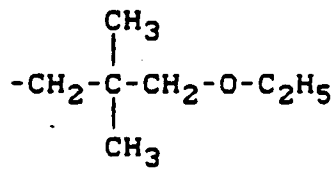 |
| 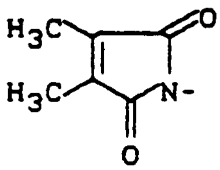 | H | 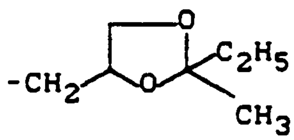 |
| 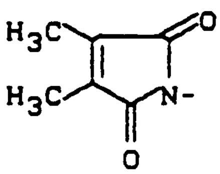 | Cl | 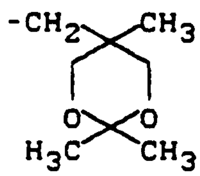 |
| 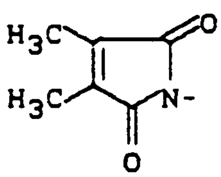 | Br | 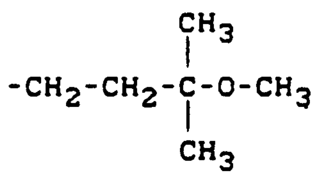 |
| 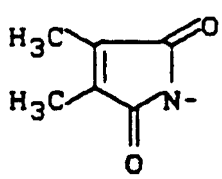 | F | 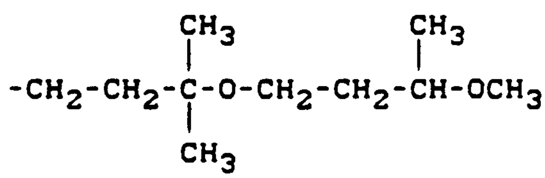 |
| 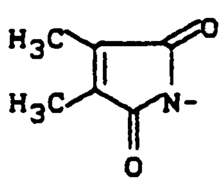 | Cl | 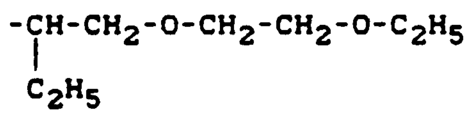 |
| 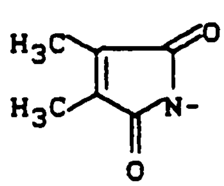 | F | 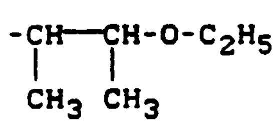 |

Tabelle 1 - Fortsetzung

| Het | X | R |
|---|---|---|
| 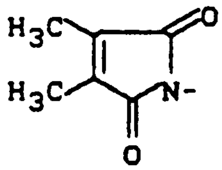 | Cl | 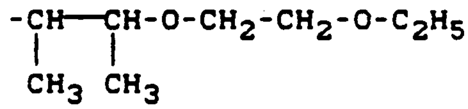 |
| 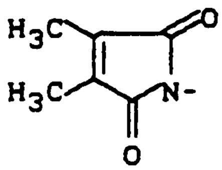 | Cl | 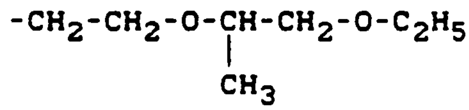 |
| 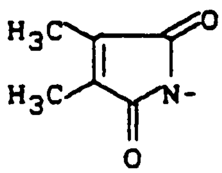 | Cl | 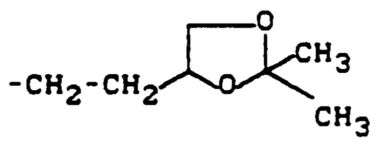 |
| 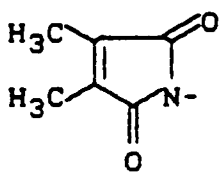 | Cl | $-CH_2-C(CH_2-O-CH_3)_3$ |
| 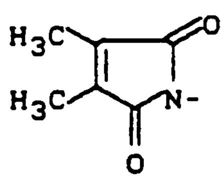 | Br | $-CH_2-C(CH_2-O-C_2H_5)_3$ |
| 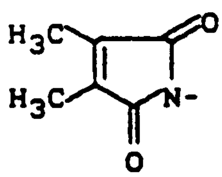 | F | 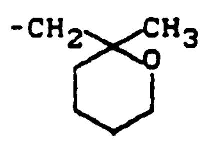 |
| 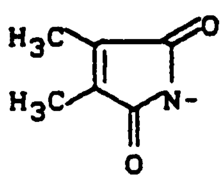 | F | 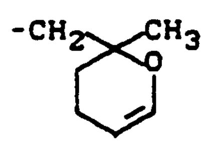 |

Tabelle 1 - Fortsetzung

| Het | X | R |
|---|---|---|
| 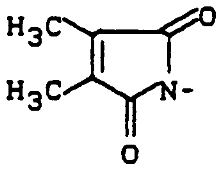 | Cl | 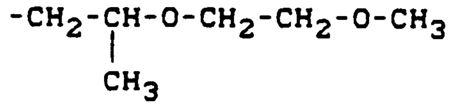 |
| 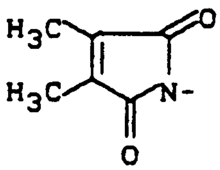 | Br | 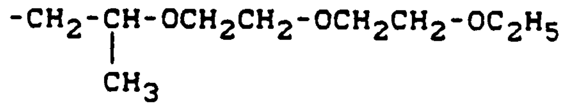 |
| 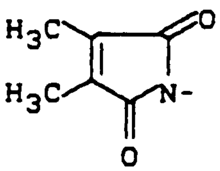 | Cl | 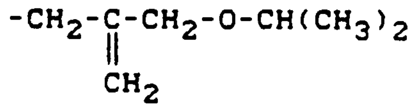 |
| 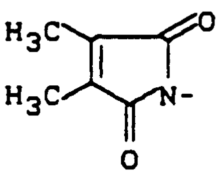 | Cl | 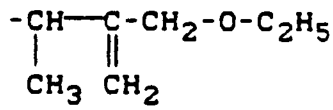 |
| 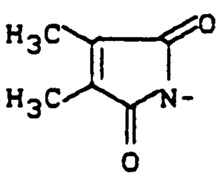 | Cl | 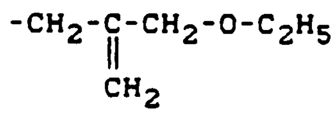 |
| 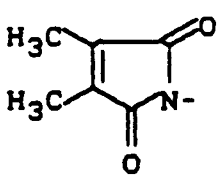 | Cl | 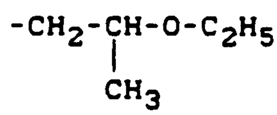 |
| 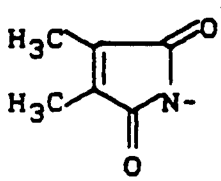 | Br | 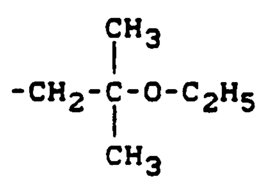 |

Tabelle 1 - Fortsetzung

| Het | X | R |
|---|---|---|
| 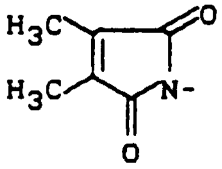 | H | 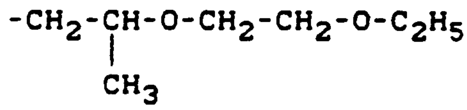 |
| 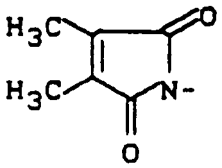 | Br | 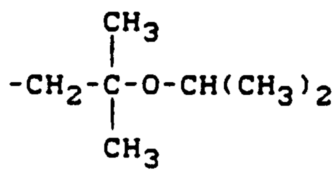 |
| 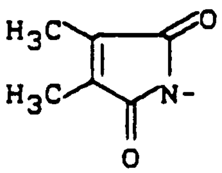 | H | 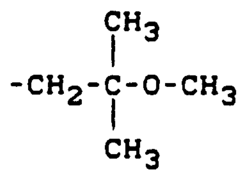 |
| 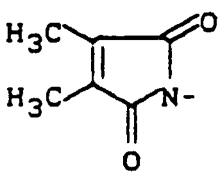 | Cl | 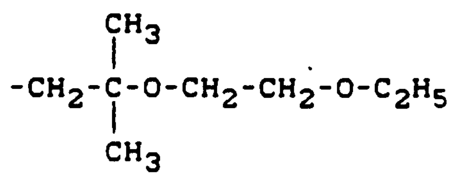 |
| 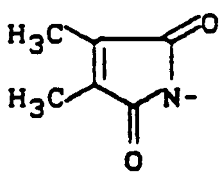 | Cl | 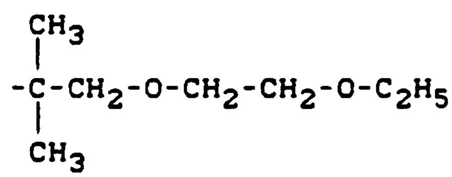 |
| 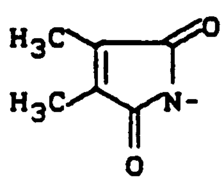 | Cl | 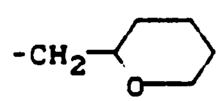 |

Tabelle 1 - Fortsetzung

| Het | X | R |
|---|---|---|
| 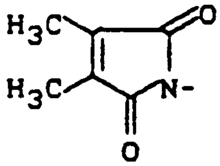 | Cl | 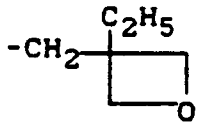 |
| 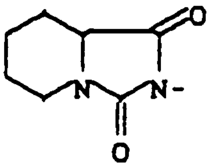 | Br | 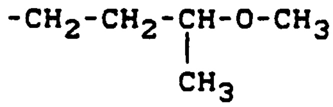 |
| 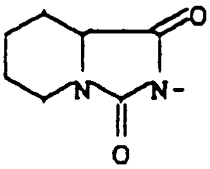 | Br | 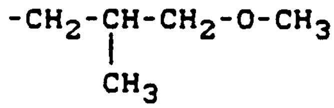 |
| 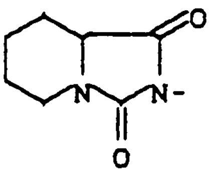 | Cl | 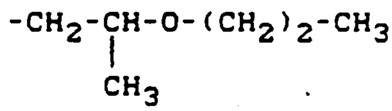 |
| 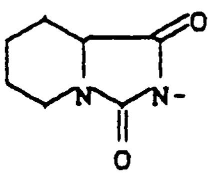 | Cl | 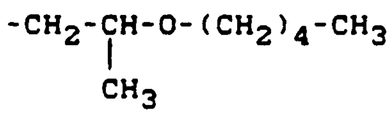 |
| 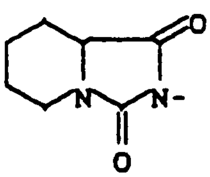 | Br | 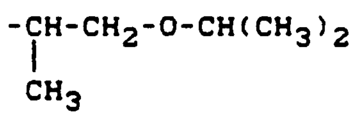 |
| 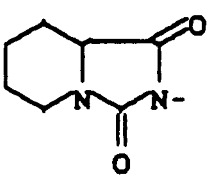 | F | 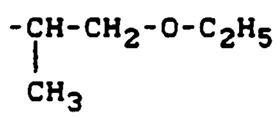 |

Tabelle 1 - Fortsetzung

| Het | X | R |
|---|---|---|
| 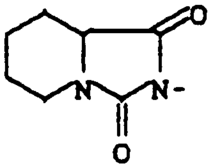 | Cl | $-CH-CH_2-O-C_2H_5$<br>$C_2H_5$ |
| 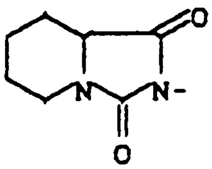 | Cl | $-CH-CH_2-O-CH_2-CH_2-O-C_2H_5$<br>$CH_3$ |
| 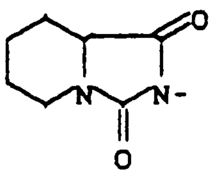 | Cl | $CH_3$<br>$-CH_2-C-CH_2-O-CH_3$<br>$CH_3$ |
| 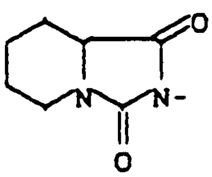 | Cl | $CH_3$<br>$-CH_2-C-CH_2-O-C_2H_5$<br>$CH_3$ |
| 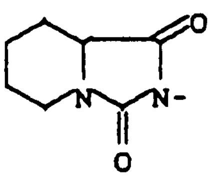 | H | 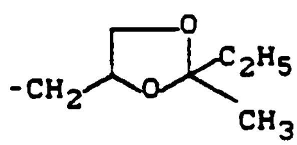 |
| 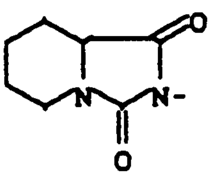 | Cl | 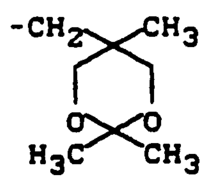 |
| 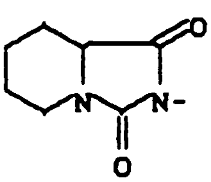 | Br | $CH_3$<br>$-CH_2-CH_2-C-O-CH_3$<br>$CH_3$ |

<u>Tabelle 1</u> - Fortsetzung

| Het | X | R |
|---|---|---|
| 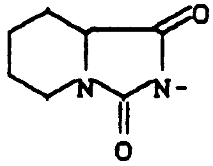 | F | 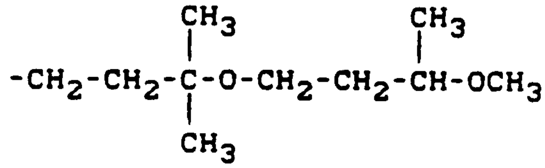 |
| 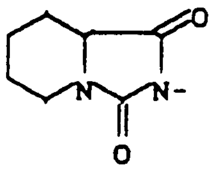 | Cl | 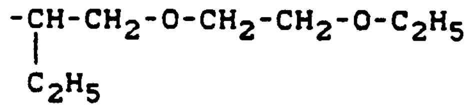 |
| 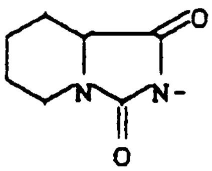 | F | 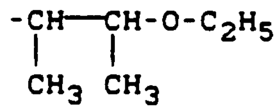 |
| 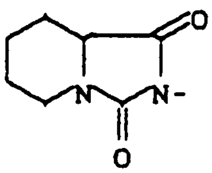 | Cl | 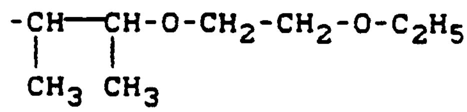 |
| 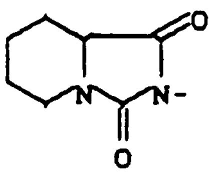 | Cl | 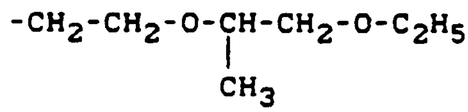 |
| 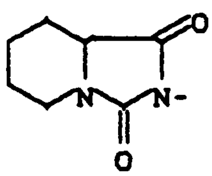 | Cl | 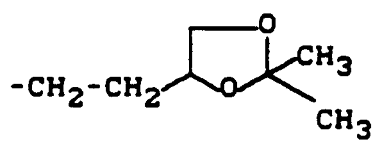 |
| 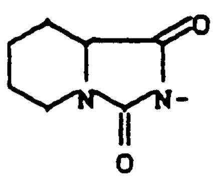 | Cl | $-CH_2-C(CH_2-O-CH_3)_3$ |

Tabelle 1 - Fortsetzung

| Het | X | R |
|---|---|---|
| 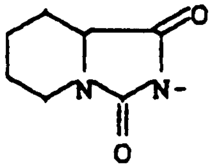 | Br | $-CH_2-C(CH_2-O-C_2H_5)_3$ |
| 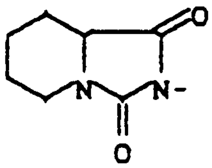 | F | 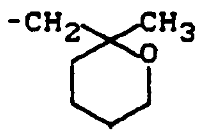 |
| 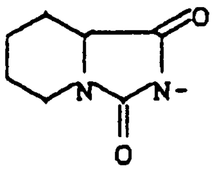 | F | 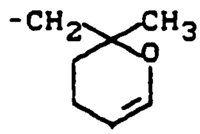 |
| 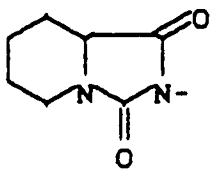 | Cl | $-CH_2-CH-O-CH_2-CH_2-O-CH_3$<br>$CH_3$ |
| 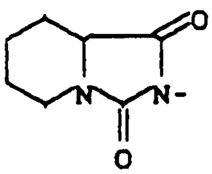 | Br | $-CH_2-CH-OCH_2CH_2-OCH_2CH_2-OC_2H_5$<br>$CH_3$ |
| 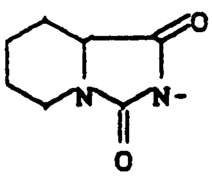 | Cl | $-CH_2-C-CH_2-O-CH(CH_3)_2$<br>$CH_2$ |
| 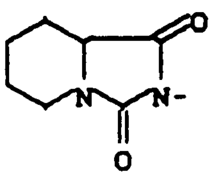 | Cl | $-CH-C-CH_2-O-C_2H_5$<br>$CH_3$ $CH_2$ |

<u>Tabelle 1</u> - Fortsetzung

| Het | X | R |
|---|---|---|
| 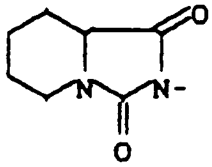 | Cl | 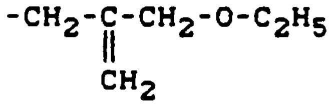 |
| 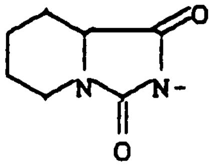 | Cl | 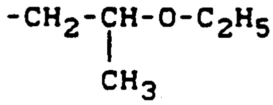 |
| 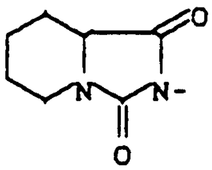 | Br | 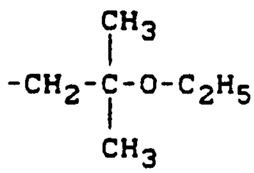 |
| 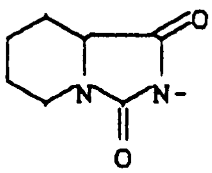 | H | 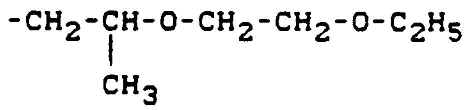 |
| 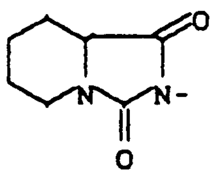 | Br | 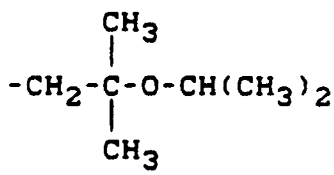 |
| 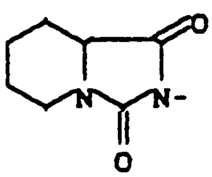 | H | 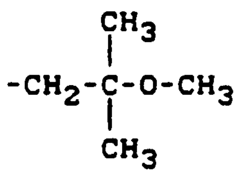 |
| 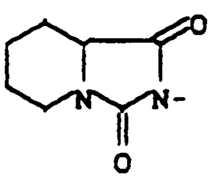 | Cl | 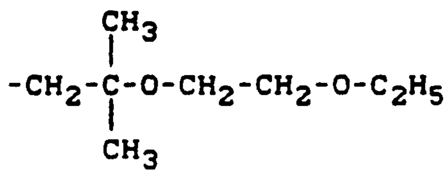 |

**Tabelle 1** - Fortsetzung

| Het | X | R |
|---|---|---|
| 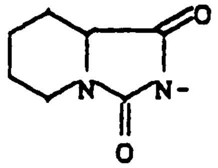 | Cl | 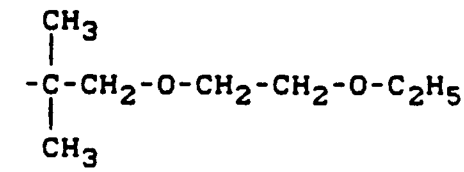 |
| 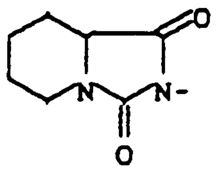 | Cl | 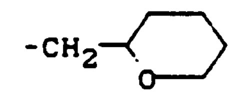 |
| 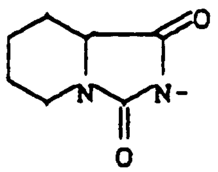 | Cl | 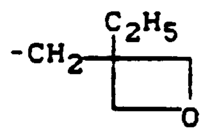 |

Verwendet man beispielsweise 3,4,5,6-Tetrahydrophthalsäureanhydrid und 2,4-Difluor-5-(2-(2-methoxy-ethoxy)-ethoxy)-anilin als Ausgangsstoffe, so kann der Reaktionsablauf beim erfindungsgemäßen Verfahren (a) durch das folgende Formelschema wiedergegeben werden:

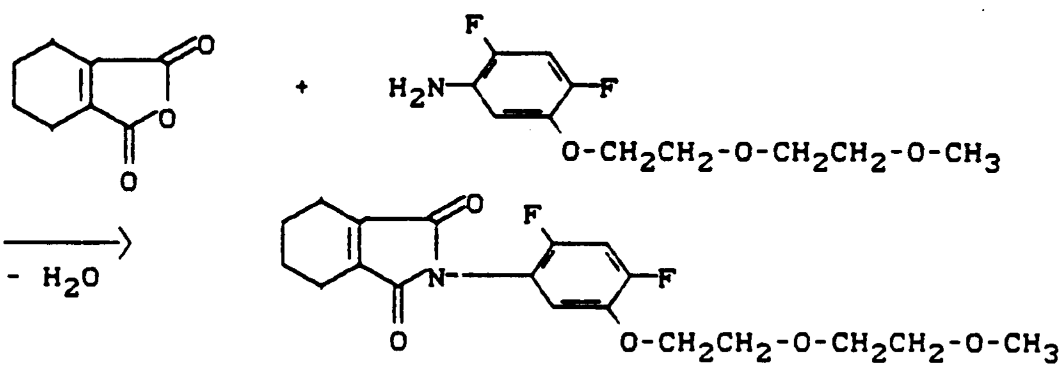


Verwendet man beispielsweise N-(4-Brom-2-fluor-5-(2-(2-ethoxy-ethoxy)-ethoxy)-phenyl)-dimethyl-maleinsäureimid und Natriumboranat als Ausgangsstoffe, so kann der Reaktionsablauf beim erfindungsgemäßen Verfahren (b) durch das folgende Formelschema wiedergegeben werden:

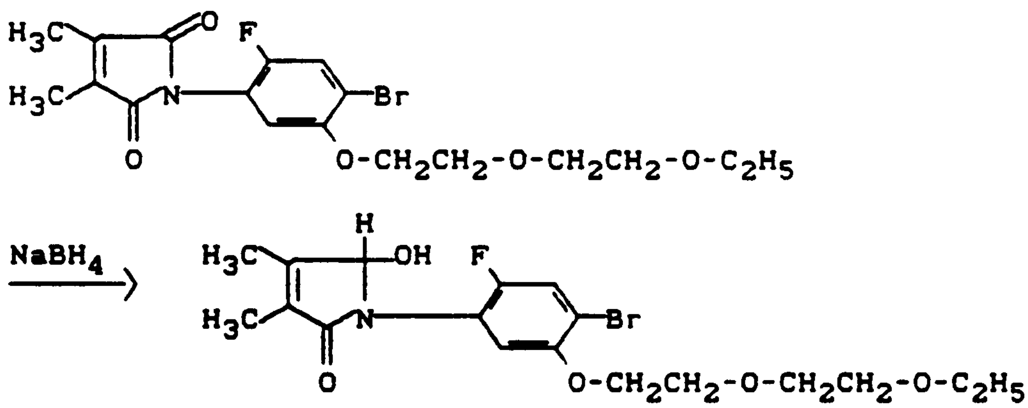


Verwendet man beispielsweise N-(4-Chlor-2-fluor-5-(2,2-bis-methoxymethyl-propyl)-phenyl)-3,4,5,6-tetrahydro phthalimid und Phosphor(V)-sulfid als Ausgangsstoffe, so kann der Reaktionsablauf beim erfindungsgemäßen Verfahren (c) durch das folgende Formelschema wiedergegeben werden:

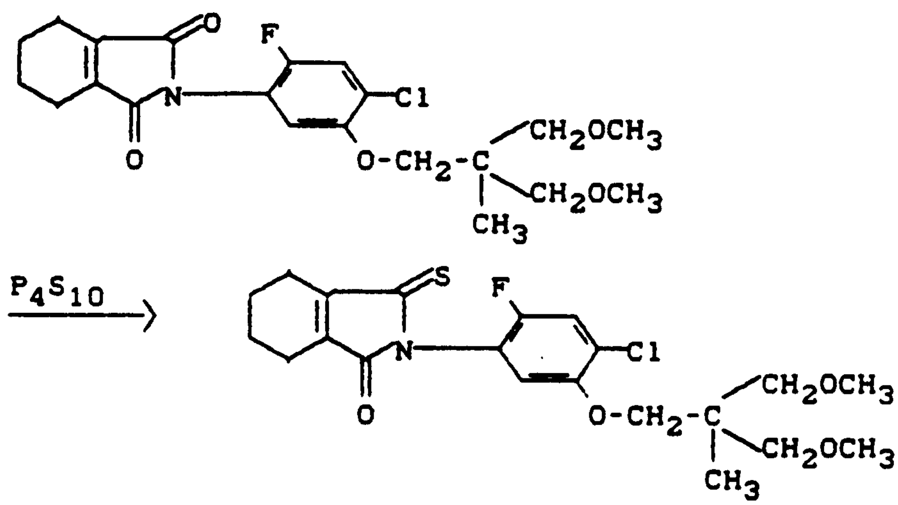


Verwendet man beispielsweise N-(4-Chlor-2-fluor-(5-(2-(2-propoxy-ethoxy)-ethoxy)-phenyl)-3,4-dimethyl-$\Delta^3$-pyrrolin-5-ol-2-on und Thionylchlorid als Ausgangsstoffe, so läßt sich der Reaktionsablauf beim erfindungsgemäßen Verfahren (d) durch das folgende Formelschema wiedergeben:

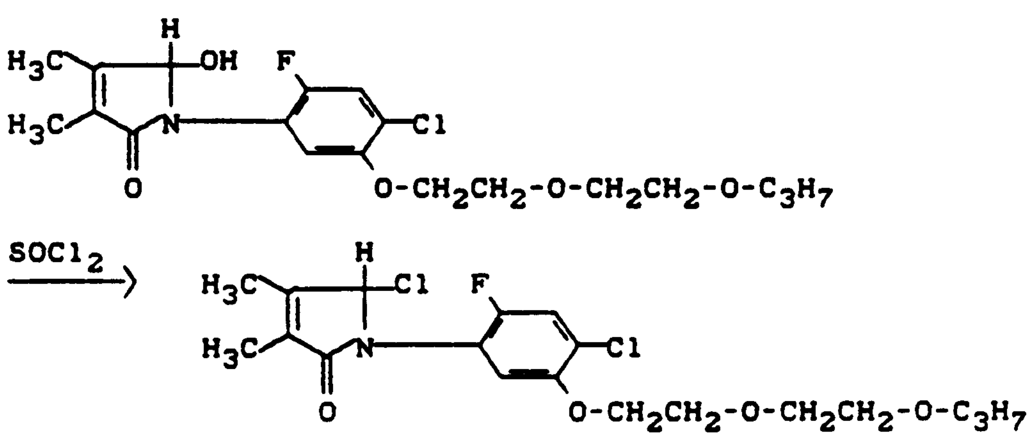


Verwendet man beispielsweise N-(2,4-Difluor-5-(2-(2-butoxy-ethoxy)-ethoxy)-phenyl)-5-chlor-3,4-dimethyl-$\Delta^3$-pyrrolin-2-on als Ausgangsverbindung, so kann der Reaktionsablauf beim erfindungsgemäßen

Verfahren (e) durch das folgende Formelschema wiedergegeben werden:

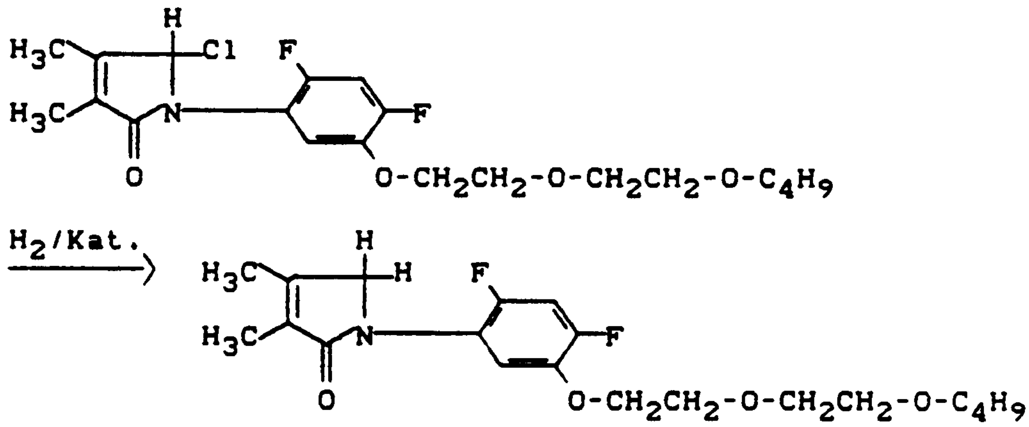


Verwendet man beispielsweise N-(2-Fluor-5-hydroxy-phenyl)-3,4,5,6-tetrahydro-phthalimid und Methansulfonsäure-(2-(2-methoxy-ethoxy)-ethyl)-ester als Ausgangsstoffe, so kann der Reaktionsablauf beim erfindungsgemäßen Verfahren (f) durch das folgende Formelschema wiedergegeben werden:

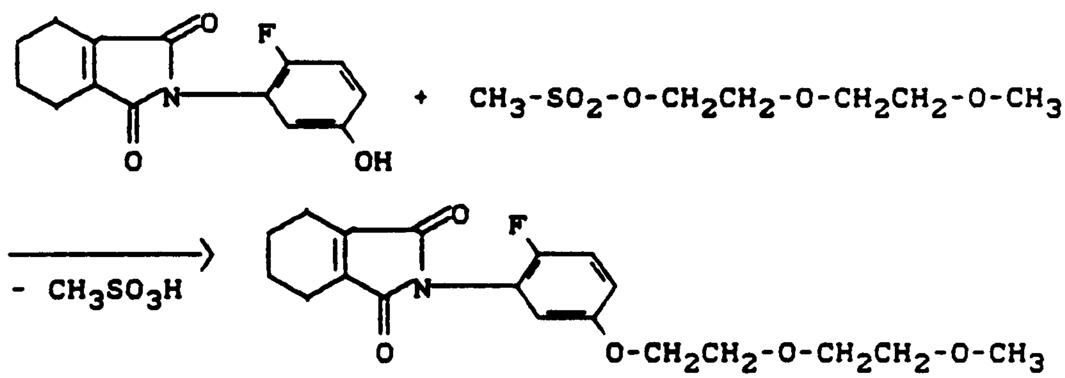


Verwendet man beispielsweise N-(2-Fluor-5-(2-(2-ethoxy-ethoxy)-ethoxy)-phenyl)-phthalimid und Chlor als Ausgangsstoffe, so kann der Reaktionsablauf beim erfindungsgemäßen Verfahren (g) durch das folgende Formelschema wiedergegeben werden:

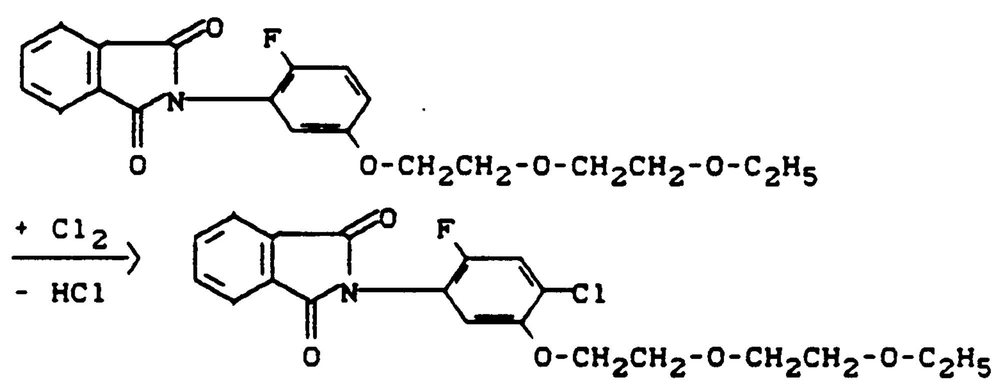


Verwendet man beispielsweise Chlorameisensäuremethylester, 4-Chlor-2-fluor-5-(2-(2-methoxy-ethoxy)-ethoxy)-anilin und Piperidin-2-carbonsäure-ethylester als Ausgangsstoffe, so kann der Reaktionsablauf beim erfindungsgemäßen Verfahren (h) durch das folgende Formelschema wiedergegeben werden:

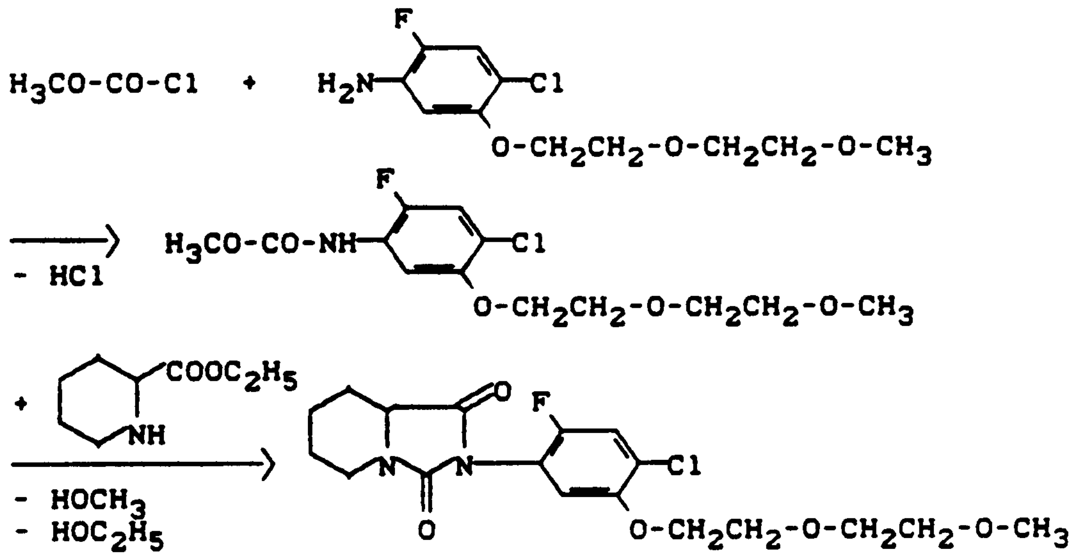


Die beim erfindungsgemäßen Verfahren (a) zur Herstellung von Verbindungen der Formel (I) als Ausgangsstoffe zu verwendenden cyclischen Anhydride sind durch die Formel (II) allgemein definiert.

In Formel (II) hat A vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für A angegeben wurde.

Als Beispiele für die Ausgangsstoffe der Formel (II) seien genannt:
Phthalsäureanhydrid, 3,4,5,6-Tetrahydro-phthalsäureanhydrid, 3-Methyl-, 4-Methyl-, 4-Trifluormethyl- und 3,3-Dimethyl-3,4,5,6-tetrahydro-phthalsäureanhydrid, 1,2,3,4-Tetrahydro-, 1,2,3,6-Tetrahydro- und 2,3,4,5-Tetrahydro-phthalsäureanhydrid, 3,6-Dihydro-phthalsäureanhydrid und Dimethylmaleinsäureanhydrid.

Die Ausgangsstoffe der Formel (II) sind bekannte organische Synthesechemikalien.

Die beim erfindungsgemäßen Verfahren (a) zur Herstellung von Verbindungen der Formel (I) weiter als Ausgangsstoffe zu verwendenden Arylamine sind durch die Formel (III) allgemein definiert.

In Formel (III) haben R und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R und X angegeben wurden.

Als Beispiele für die Ausgangsstoffe der Formel (III) seien genannt:
5-(2-(2-Methoxy-ethoxy)-ethoxy)-, 5-(2-(2-Ethoxy-ethoxy)-ethoxy)-, 5-(2-(2-Propoxy-ethoxy)-ethoxy)-, 5-(2-(2-Isopropoxy-ethoxy)-ethoxy)-, 5-(2-(2-Butoxy-ethoxy)-ethoxy)-, 5-(2-(2-Isobutoxy-ethoxy)-ethoxy)-, 5-(2-(2-sec-Butoxy-ethoxy)-ethoxy)-, 5-(2-(2-tert-Butoxy-ethoxy)-ethoxy)-, 5-(2,2-Bis-methoxymethyl-propoxy)- und 5-((2,2-Dimethyl-1,3-dioxolan-4-yl)-methoxy)-2-fluor-anilin, -2,4-difluor-anilin, -4-chlor-2-fluor-anilin und -4-brom-2-fluor-anilin.

Die Ausgangsstoffe der Formel (III) sind noch nicht aus der Literatur bekannt.

Man erhält die Verbindungen der allgemeinen Formel (III), wenn man

($\alpha$) Hydroxyarylamine der allgemeinen Formel (IX)

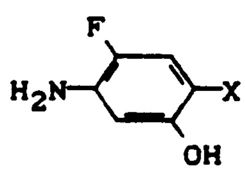 (IX)

in welcher

X die oben angegebene Bedeutung hat

mit Alkylierungsmitteln der allgemeinen Formel (V)

$X^1$ - R (V)

in welcher

R und $X^1$ die oben angegebenen Bedeutungen haben,
in Gegenwart eines Verdünnungsmittels, wie z. B. Aceton, Acetonitril, Dimethylformamid, Dimethylsulfoxid oder N-Methyl-pyrrolidon, gegebenenfalls in Gegenwart eines Säureakzeptors, wie z. B. Natrium- oder Kalium-carbonat, -hydrid oder -hydroxid, sowie gegebenenfalls zusätzlich in Gegenwart von Wasser, bei Temperaturen zwischen 0 °C und 100 °C umsetzt, oder wenn man
(ß) Nitrophenol-Derivate der allgemeinen Formel (X)

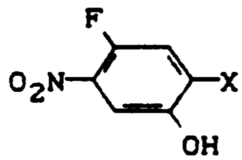
(X)

in welcher
X die oben angegebene Bedeutung hat
mit Alkylierungsmitteln der allgemeinen Formel (V)

$X^1$ - R (V)

in welcher
R und $X^1$ die oben angegebenen Bedeutungen haben,
nach der oben unter (α) angegebenen Methodik umsetzt und die so erhaltenen Verbindungen der allgemeinen Formel (XI)

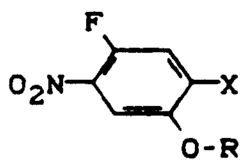
(XI)

in welcher
R und X die oben angegebenen Bedeutungen haben,
nach üblichen Methoden, beispielsweise mit Wasserstoff in Gegenwart eines Katalysators, wie z. B. Platin auf Aktivkohle, in Gegenwart eines Verdünnungsmittels, wie z. B. Ethanol, bei Temperaturen zwischen 0 °C und 100 °C reduziert, oder wenn man
(γ) Fluorphenol-Derivate der allgemeinen Formel (XII)

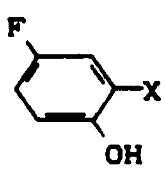
(XII)

in welcher
X die oben angegebene Bedeutung hat,
mit Alkylierungsmitteln der allgemeinen Formel (V)

$X^1$ - R (V)

in welcher
R und $X^1$ die oben angegebenen Bedeutungen haben,
nach der oben unter (α) angegebenen Methode umsetzt und die so erhaltenen Verbindungen der Formel (XIII)

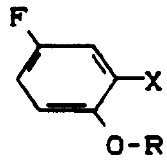

(XIII)

in welcher

R und X die oben angegebenen Bedeutungen haben,

mit Salpetersäure zu den Verbindungen der Formel (XI) nitriert und anschließend nach üblichen Methoden (siehe (ß)) reduziert.

Die Nitrierung kann durchgeführt werden in anorganischen Säuren, wie Schwefelsäure oder Salpetersäure, aber auch in organischen Lösungsmitteln, vorzugsweise Halogenkohlenwasserstoffen, wie Methylenchlorid, mit oder ohne Zusatz von Salzen der salpetrigen Säure, mit oder ohne Zusatz von Harnstoff oder Amidosulfonsäure, bei Temperaturen von -30 °C bis +60 °C, vorzugsweise -10 °C bis +30 °C.

Die als Ausgangsstoffe benötigten Phenole der Formel (XII), die Hydroxyarylamine der Formel (IX) und die Nitrophenol-Derivate der Formel (X) sind bereits bekannt (vgl. EP-A 61 741).

Als Beispiele hierfür seien genannt:

2-Chlor-4-fluorphenol und 4-Fluorphenol, 2-Fluor-3-hydroxy-anilin und -nitrobenzol, 4-Chlor-2-fluor-3-hydroxy-anilin und -nitrobenzol sowie 4-Brom-2-fluor-3-hydroxy-anilin und -nitrobenzol.

Die weiter als Ausgangsstoffe benötigten Alkylierungsmittel sind durch die Formel (V) allgemein definiert.

In Formel (V) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde und $X^1$ steht vorzugsweise für Methylsulfonyl, Phenylsulfonyl oder Tolylsulfonyl.

Als Beispiele für die Ausgangsstoffe der Formel (V) seien genannt:

Methansulfonsäure-, Benzolsulfonsäure- und p-Toluolsulfonsäure-2-(2-methoxy-ethoxy)-ethylester, -2-(2-ethoxy-ethoxy)-ethylester, -2-(2-propoxy-ethoxy)-ethylester, -2-(2-butoxy-ethoxy)-ethylester, -2-(2-isopropoxy-ethoxy)-ethylester,-2-(2-isobutoxy-ethoxy)-ethylester, -2-(2-sec-butoxy-ethoxy)-ethylester, -2-(2-tert-butoxy-ethoxy)-ethylester, -2,2-bis-methoxymethyl-propylester und -(2,2-dimethyl-1,3-dioxolan-4-yl)-methylester.

Die Alkylierungsmittel der Formel (V) sind bekannt und/oder können nach an sich bekannten Verfahren, beispielsweise durch Umsetzung von geeigneten Sulfonsäurechloriden, wie z. B. Methansulfonsäurechlorid, mit geeigneten Hydroxyverbindungen (HOR) hergestellt werden.

Die bei den erfindungsgemäßen Verfahren (b) und (c) zur Herstellung von Verbindungen der Formel (I) als Ausgangsstoffe zu verwendenden substituierten Arylimide sind durch die Formel (Ia) allgemein definiert.

In Formel (Ia) haben A, R und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R und X angegeben wurden.

Beispiele für die Ausgangsstoffe der Formel (Ia) sind Tabelle 1 (oben) zu entnehmen.

Die substituierten Arylimide der Formel (Ia) sind neue, erfindungsgemäße Verbindungen; sie können nach dem erfindungsgemäßen Verfahren (a) hergestellt werden.

Die beim erfindungsgemäßen Verfahren (d) zur Herstellung von Verbindungen der Formel (I) als Ausgangsstoffe zu verwendenden N-Aryl-Stickstoffheterocyclen sind durch die Formel (Ib) allgemein definiert.

In Formel (Ib) haben A, R und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R und X angegeben wurden.

Beispiele für die Ausgangsstoffe der Formel (Ib) sind Tabelle 1 (oben) zu entnehmen.

Die N-Aryl-Stickstoffheterocyclen der Formel (Ib) sind neue, erfindungsgemäße Verbindungen; sie können nach dem erfindungsgemäßen Verfahren (b) hergestellt werden.

Die beim erfindungsgemäßen Verfahren (e) zur Herstellung von Verbindungen der Formel (I) als Ausgangsstoffe zu verwendenden N-Aryl-Stickstoffheterocyclen sind durch die Formel (Ic) allgemein definiert.

In Formel (Ic) haben A, R und X vorzugweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, R und X angegeben wurden.

Beispiele für die Ausgangsstoffe der Formel (Ic) sind Tabelle 1 (oben) zu entnehmen.

Die N-Aryl-Stickstoffheterocyclen der Formel (Ic) sind neue, erfindungsgemäße Verbindungen; sie können nach dem erfindungsgemäßen Verfahren (d) hergestellt werden.

Die beim erfindungsgemäßen Verfahren (f) zur Herstellung von Verbindungen der Formel (I) als Ausgangsstoffe zu verwendenden Hydroxyarylimide sind durch die Formel (IV) allgemein definiert.

In Formel (IV) haben A und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A und X angegeben wurden.

Als Beispiele für die Ausgangsstoffe der Formel (IV) seien genannt:
N-(2-Fluor-5-hydroxy-phenyl)-, N-(2,4-Difluor-5-hydroxy-phenyl)-, N-(4-chlor-2-fluor-5-hydroxy-phenyl)-phthalsäureimid, N-(2-Fluor-5-hydroxy-phenyl)-, N-(2,4-Difluor-5-hydroxy-phenyl)-, N-(4-Chlor-2-fluor-5-hydroxy-phenyl)- und N-(4-Brom-2-fluor-5-hydroxy-phenyl)-3,4,5,6-tetrahydrophthalimid, N-(2-Fluor-5-hydroxy-phenyl)-, N-(2,4-Difluor-5-hydroxy-phenyl)-, N-(4-Chlor-2-fluor-5-hydroxy-phenyl)- und N-(2-Fluor-4-brom-5-hydroxy-phenyl)-, -3-methyl-, -4-methyl-, -4-trifluormethyl- und -3,3-dimethyl-3,4,5,6-tetrahydrophthalimid, N-(2-Fluor-5-hydroxy-phenyl)-, N-(2,4-Difluor-5-hydroxy-phenyl)-, N-(4-Chlor-2-fluor-3-hydroxy-phenyl)- und N-(4-Brom-2-fluor-5-hydroxy-phenyl)-3,6-dihydro-phthalimid, sowie N-(2-Fluor-5-hydroxy-phenyl)-, N-(2,4-Difluor-5-hydroxy-phenyl-, N-(4-Chlor-2-fluor-5-hydroxy-phenyl)- und N-(4-Brom-2-fluor-5-hydroxy-phenyl)-dimethylmaleinsäureimid.

Die Ausgangsstoffe der Formel (IV) sind bekannt und/oder können nach an sich bekannten Verfahren hergestellt werden (vgl. EP-A 61 741).

Die beim erfindungsgemäßen Verfahren (f) zur Herstellung von Verbindungen der Formel (I) weiter als Ausgangsstoffe zu verwendenden Alkylierungsmittel sind durch die Formel (V) allgemein definiert.

In Formel (V) hat R vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R angegeben wurde und $X^1$ steht vorzugsweise für Methylsulfonyl, Phenylsulfonyl oder Tolylsulfonyl.

Beispiele für die Ausgangsstoffe der Formel (V) wurden bereits oben im Zusammenhang mit der Beschreibung der Ausgangsstoffe für das erfindungsgemäße Verfahren (a) genannt.

Die beim erfindungsgemäßen Verfahren (g) zur Herstellung von Verbindungen der Formel (I) als Ausgangsstoffe zu verwendenden substituierten Arylimide sind durch die Formel (Id) allgemein definiert.

In Formel (Id) haben A und R vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A und R angegeben wurden.

Beispiele für die Ausgangsstoffe der Formel (Id) sind Tabelle 1 (oben) zu entnehmen.

Die substituierten Arylimide der Formel (Id) sind neue, erfindungsgemäße Verbindungen, sie können nach dem erfindungsgemäßen Verfahren (a) hergestellt werden.

Die beim erfindungsgemäßen Verfahren (h) zur Herstellung von Verbindungen der Formel (I) als Ausgangsstoffe zu verwendenden Arylamine sind durch die Formel (III) allgemein definiert.

In Formel (III) haben R und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R und X angegeben wurden.

Beispiele für die Ausgangsstoffe der Formel (III) und Verfahren zu ihrer Herstellung wurden bereits oben im Zusammenhang mit der Beschreibung der Ausgangsstoffe für das erfindungsgemäße Verfahren (a) angegeben.

Die beim erfindungsgemäßen Verfahren (h) weiter als Ausgangsstoffe zu verwendenden Chlorameisensäureester sind durch die Formel (VI) allgemein definiert. In Formel (VI) steht $R^3$ vorzugsweise für Methyl, Benzyl oder Phenyl.

Als Beispiele für die Ausgangsstoffe der Formel (VI) seien genannt:
Chlorameisensäure-methylester, -benzylester und -phenylester.

Die Chlorameisensäureester der Formel (VI) sind bekannte organische Synthesechemikalien.

Die beim erfindungsgemäßen Verfahren (h) ferner als Ausgangsstoffe zu verwendenden Piperidin-2-carbonsäureester sind durch die Formel (VIII) allgemein definiert.

In Formel (VIII) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurde und $R^4$ steht vorzugsweise für Methyl oder Ethyl.

Als Beispiele für die Ausgangsstoffe der Formel (VIII) seien genannt:
Piperidin-2-carbonsäure-methylester und -ethylester.

Die Ausgangsstoffe der Formel (VIII) sind bekannte organische Synthesechemikalien.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Carbonsäuren wie Ameisensäure, Essigsäure oder Propionsäure, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (a) kann gegebenenfalls in Gegenwart eines geeigneten Reaktionshilfsmittels durchgeführt werden. Vorzugsweise verwendet man anorganische oder organische Säuren wie beispielsweise Essigsäure oder p-Toluolsulfonsäure, Anhydride wie beispielsweise Acetanhydrid oder Säurechloride wie Acetylchlorid als Reaktionshilfsmittel. Es ist auch möglich andere übliche wasserabspaltende Mittel wie beispielsweise N,N′-Dicyclohexylcarbodiimid oder übliche Acylierungskatalysatoren, wie beispielsweise 4-(N,N-Dimethylamino)-pyridin als Reaktionshilfsmittel zu verwenden.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 180 °C, vorzugsweise bei Temperaturen zwischen 50 °C und 150 °C.

Zur Durchführung des erfindungsgemäßen Verfahrens (a) setzt man pro Mol an Anhydrid der Formel (II) im allgemeinen 1.0 bis 1.5 Mol, vorzugsweise 1.0 bis 1.2 Mol an Amin der Formel (III) und gegebenenfalls 0.01 bis 1.2 Mol, vorzugsweise 0.1 bis 1.0 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Als Reduktionsmittel zur Durchführung des erfindungsgemäßen Verfahrens (b) kommen alle üblichen für derartige Reduktionsreaktionen verwendbaren Reduktionsmittel infrage. Vorzugsweise verwendet man komplexe Hydride, wie beispielsweise Natriumborhydrid, Natriumcyanoborhydrid, Lithiumborhydrid oder Lithiumaluminiumhydrid.

Als Verdünnungsmittel zur Durchführung des erfindungsgemäßen Verfahrens (b) kommen in Abhängigkeit vom verwendeten Reduktionsmittel alle üblichen organischen oder anorganischen Lösungsmittel in Frage. Vorzugsweise verwendet man Ether, wie Diethylether, Dioxan oder Tetrahydrofuran oder Alkohole, wie Methanol, Ethanol oder Propanol.

Die Reaktionstemperaturen können bei der Durchführung des erfindungsgemäßen Verfahrens (b) in Abhängigkeit vom verwendeten Reduktionsmittel in einem größeren Bereich variiert werden. Im allgemeinen arbeitet man bei Temperaturen zwischen - 20 °C und + 150 °C, vorzugsweise bei Temperaturen zwischen 0 °C und 120 °C.

Zur Durchführung des erfindungsgemäßen Verfahrens (b) setzt man pro Mol an substituiertem Arylimid der Formel (Ia) im allgemeinen 0.1 bis 2.0 Mol, vorzugsweise 0.25 bis 1.5 Mol an Reduktionsmittel ein.
Die Reaktionsdurchführung, Aufarbeitung und Isolierung der Reaktionsprodukte erfolgt nach allgemein üblichen Methoden (vgl. auch die Herstellungsbeispiele).

Als Schwefelungsmittel zur Durchführung des erfindungsgemäßen Verfahrens (c) kommen alle üblichen für derartige Schwefelungsreaktionen verwendbaren Schwefelungsmittel in Betracht. Vorzugsweise verwendet man Phosphor-Schwefel-Verbindungen, wie beispielsweise Phosphor(V)-sulfid ($P_4S_{10}$) oder das sogenannte Lawesson-Reagenz (2,4-Bis-(4-methoxy-phenyl)-1,3-dithia-phosphetan-2,4-disulfid.

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 180 °C, vorzugsweise bei Temperaturen zwischen 20 °C und 150 °C.

Zur Durchführung des erfindungsgemäßen Verfahrens (c) setzt man je Mol Arylimid der Formel (Ia) im allgemeinen zwischen 0,2 und 2,0 Mol, vorzugsweise zwischen 0,5 und 1,5 Mol, Schwefelungsmittel ein.

Die Reaktionsdurchführung, Aufarbeitung und Isolierung der Reaktionsprodukte erfolgt nach an sich bekannter Verfahrensweise (vgl. Bull. Soc. Chim. Belg. 87 (1978), 223 - 228). Im allgemeinen erhält man

hierbei Gemische aus einfach und zweifach geschwefelten Produkten, die sich nach üblichen Trennmethoden (z. B. Chromatographie oder Kristallisation) auftrennen lassen.

Als Verdünnungsmittel zur Durchführung des erfindungsgemäßen Verfahrens (d) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Nitrile wie Acetonitril oder Propionitril.

Das erfindungsgemäße Verfahren (d) kann gegebenenfalls in Gegenwart eines geeigneten Reaktionshilfsmittels durchgeführt werden. Als solche kommen insbesondere organische Amine oder Amide infrage. Vorzugsweise verwendet man Pyridin, Dimethylanilin oder Dimethylformamid.

Die Reaktionstemperaturen können bei der Durchführung des erfindungsgemäßen Verfahrens (d) in einem größeren Bereich variiert werden. Im allgemeinen arbeitet man bei Temperaturen zwischen 0 °C und 120 °C, vorzugsweise bei Temperaturen zwischen 20 °C und 80 °C.

Zur Durchführung des erfindungsgemäßen Verfahrens (d) setzt man pro Mol an N-Aryl-Stickstoffheterocyclus der Formel (Ib) im allgemeinen 1.0 bis 5.0 Mol, vorzugsweise 1.0 bis 2.0 Mol an Thionylchlorid und gegebenenfalls 0.1 bis 2.0 Mol, vorzugsweise 0.5 bis 1.5 Mol an Reaktionshilfsmittel ein.

Die Reaktionsdurchführung, Aufarbeitung und Isolierung der Reaktionsprodukte erfolgt nach allgemein üblichen Methoden.

Als Katalysatoren zur Durchführung des erfindungsgemäßen Verfahrens (e) kommen alle üblichen Hydrierkatalysatoren in Frage. Vorzugsweise verwendet man Edelmetallkatalysatoren, wie beispielsweise Platin, Platinoxid, Palladium oder Ruthenium gegebenenfalls auf einem geeignetem Träger wie beispielsweise Kohlenstoff oder Siliciumdioxid.

Als Verdünnungsmittel zur Durchführung des erfindungsgemäßen Verfahrens (e) kommen inerte organische Lösungsmittel in Frage. Hierzu gehören insbesondere aliphatische oder alicyclische gegebenenfalls halogenierte Kohlenwasserstoffe, wie beispielsweise Benzin, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether oder Alkohole wie Methanol, Ethanol oder Propanol.

Das erfindungsgemäße Verfahren (e) kann gegebenenfalls in Gegenwart eines geeigneten Säurebindemittels durchgeführt werden. Vorzugsweise verwendet man Alkalimetallcarbonate wie Natriumcarbonat oder Kaliumcarbonat oder organische Basen wie Pyridin oder Lutidin.

Die Reaktionstemperaturen können bei der Durchführung des erfindungsgemäßen Verfahrens (e) in einem größeren Bereich variiert werden. Im allgemeinen arbeitet man bei Temperaturen zwischen -20 °C und 100 °C, vorzugsweise bei Temperaturen zwischen 0 °C und 50 °C.

Das erfindungsgemäße Verfahren kann bei Normaldruck oder unter erhöhtem Druck durchgeführt werden. Vorzugsweise arbeitet man unter Normaldruck.

Zur Durchführung des erfindungsgemäßen Verfahrens (e) setzt man pro Mol an N-Aryl-Stickstoffheterocyclus der Formel (Ic) im allgemeinen 1.0 bis 3.0 Mol, vorzugsweise 1.0 bis 1.5 Mol an Wasserstoff und gegebenenfalls 1.0 bis 3.0 Mol, vorzugsweise 1.0 bis 1.5 Mol an Säurebindemittel ein.
Die Reaktionsdurchführung, Aufarbeitung und Isolierung der Reaktionsprodukte erfolgt nach allgemein üblichen Methoden.

Das erfindungsgemäße Verfahren (f) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (f) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (f) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (f) wird im allgemeinen bei Normaldruck durchgeführt.

Zur Durchführung des erfindungsgemäßen Verfahrens (f) setzt man je Mol Hydroxyarylimid der Formel (IV) im allgemeinen zwischen 1 und 2 Mol, vorzugsweise zwischen 1,1 und 1,5 Mol, Alkylierungsmittel der Formel (V) ein.

Die Reaktionsdurchführung, Aufarbeitung und Isolierung der Reaktionsprodukte erfolgt nach allgemein üblichen Methoden.

Als Halogenierungsmittel zur Durchführung des erfindungsgemäßen Verfahrens (g) kommen die üblichen für die Halogenierung von aromatischen Verbindungen verwendbaren Halogenierungsmittel in Betracht. Vorzugsweise verwendet man elementare Halogene, wie Chlor oder Brom, oder Halogenverbindungen, wie Sulfurylchlorid.

Verfahren (g) wird gegebenenfalls unter Verwendung von Katalysatoren durchgeführt. Als solche kommen vorzugsweise saure bzw. elektrophile Halogenverbindungen, wie z. B. Hydrogenchlorid, Hydrogenbromid, Aluminiumchlorid, Aluminiumbromid, Eisen(III)-chlorid oder Eisen(III)-bromid in Betracht.

Das erfindungsgemäße Verfahren (g) wird vorzugsweise unter Verwendung von Verdünnungsmitteln durchgeführt. Es kommen vor allem diejenigen organischen Solventien in Betracht, die bereits oben für Verfahren (f) angegeben wurden, daneben jedoch auch Essigsäure und/oder Wasser.

Die Reaktionstemperaturen können bei dem erfindungsgemäßen Verfahren (g) in einem größeren Bereich variiert werden. Im allgemeinen arbeitet man bei Temperaturen zwischen 0 °C und 150 °C, vorzugsweise bei Temperaturen zwischen 20 °C und 120 °C.

Verfahren (g) wird im allgemeinen bei Normaldruck durchgeführt.

Zur Durchführung des erfindungsgemäßen Verfahrens (g) setzt man je Mol Ausgangsverbindung der Formel (Id) im allgemeinen zwischen 1 und 5 Mol, vorzugsweise zwischen 1 und 3 Mol, Halogenierungsmittel ein.

Die Reaktionsdurchführung, Aufarbeitung und Isolierung der Reaktionsprodukte erfolgt nach allgmein üblichen Methoden.

Das erfindungsgemäße Verfahren (h) wird vorzugsweise unter Verwendung von Verdünnungsmitteln durchgeführt. Es kommen vor allem diejenigen organischen Solventien in Betracht, die bereits oben für Verfahren (f) angegeben wurden, in der zweiten Stufe außerdem vorzugsweise auch Alkohole, wie Methanol, Ethanol oder Isopropanol.

Das erfindungsgemäße Verfahren (h) wird vorzugsweise in Gegenwart eines Säureakzeptors durchgeführt. Es kommen als solche vorwiegend diejenigen Säurebindemittel in Betracht, die bereits oben für Verfahren (f) angegeben wurden.

Die Reaktionstemperaturen können bei dem erfindungsgemäßen Verfahren (h) in einem größeren Bereich variiert werden. Im allgemeinen arbeitet man bei Temperaturen zwischen -20 °C und +150 °C, vorzugsweise bei Temperaturen zwischen 0 °C und 100 °C.

Das erfindungsgemäße Verfahren (h) wird im allgemeinen bei Normaldruck durchgeführt.

Zur Durchführung des erfindungsgemäßen Verfahrens (h) setzt man je Mol Arylamin der Formel (III) im allgemeinen zwischen 0,8 und 1,5 Mol, vorzugsweise zwischen 1,0 und 1,2 Mol, Chlorameisensäureester der Formel (VI) und je Mol Arylurethan der Formel (VII) im allgemeinen zwischen 0,8 und 1,5 Mol, vorzugsweise zwischen 1,0 und 1,2 Mol, Piperidin-2-carbonsäureester der Formel (VIII) ein.

Die Reaktionsdurchführung, Aufarbeitung und Isolierung der Reaktionsprodukte erfolgt nach allgemein üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern im Vorauflauf- und im Nachauflauf-Verfahren.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl,

Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)buttersäure (2,4-DB); 2,4-Dichlorphenoxypropionsäure (2,4-DP); 2-[[[[[(4,6-Dimethoxypyrimidin-2-yl)-amino]-carbonyl]-amino]-sulfonyl]-methyl]-benzoesäuremethylester (BENSULFURON); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl-oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoe säure oder deren Methylester (METSULFURON); S-Ethyl-N,N-hexamethylen-thiolcarbamat (MOLINATE); 4-(Di-n-propylamino)-3,5-dinitrobenzolsulfonamid (ORYZALIN); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) und 3,5,6-Trichlor-2-pyridyloxyessigsäure (TRICLOPYR). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

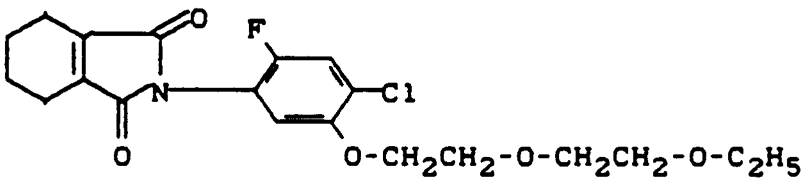

(Verfahren (a))

Eine Mischung aus 6,4 g (0,023 Mol) 4-Chlor-2-fluor-5-(2-(2-ethoxy-ethoxy)-ethoxy)-anilin, 3,7 g (0,024 Mol) 3,4,5,6-Tetrahydrophthalsäureanhydrid und 60 ml Essigsäure wird 5 Stunden bei 80 °C gerührt. Nach dem Erkalten wird die Reaktionsmischung mit Wasser verdünnt und mit Methylenchlorid extrahiert. Die organische Phase wird mit Natriumbicarbonat-Lösung gewaschen, über Magnesiumsulfat getrocknet und im Vakuum eingeengt. Das so erhaltene dunkle Öl wird einer säulenchromatographischen Reinigung an Kieselgel mit dem Laufmittel Cyclohexan/Essigester (1 : 1) unterworfen.

Man erhält 4,8 g (50,7 % der Theorie) N-[4-Chlor-2-fluor-5-[2-(2-ethoxy-ethoxy)-ethoxy]-phenyl]-3,4,5,6-tetrahydrophthalimid als farblose Kristalle vom Schmelzpunkt 61 °C.

Beispiel 2

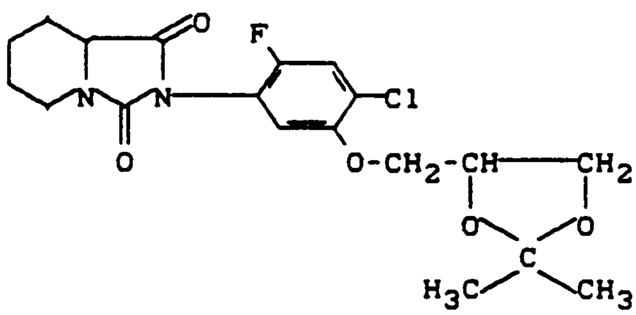

(Verfahren (h))

Eine Lösung von 1,4 ml (11 mMol) Chlorameisensäurephenylester in 15 ml Methylenchlorid und 15 ml 1N-Natronlauge werden simultan zu einer gerührten und auf 0 °C bis 5 °C gekühlten Lösung von 3,05 g (11 mMol) 4-Chlor-2-fluor-5-((2,2-dimethyl-1,3-dioxolan-4-yl)-methoxy)-anilin in 20 ml Methylenchlorid gegeben. Nach 30 Minuten Rühren wird die organische Phase abgetrennt und die wäßrige Phase wird mit Methylenchlorid nachextrahiert; die organischen Phasen werden vereinigt, mit Natriumsulfat getrocknet und filtriert. Vom Filtrat wird das Lösungsmittel im Wasserstrahlvakuum abdestilliert, der Rückstand in 20 ml Propanol aufgenommen, mit 1,73 g (11 mMol) Piperidin-2-carbonsäureethylester versetzt und das Gemisch 6 Stunden unter Rückfluß zum Sieden erhitzt. Nach Einengen wird der Rückstand in Methylenchlorid aufgenommen, diese Lösung mit 1N-Natronlauge gewaschen, mit Natriumsulfat getrocknet und filtriert. Das Filtrat wird eingeengt und der Rückstand durch Säulenchromatographie (an Kieselgel mit Cyclohexan/Essigsäureethylester, 2 : 3) gereinigt.

Man erhält 1,85 g (41 % der Theorie) 2-(4-Chlor-2-fluor-5-((2,2-dimethyl-1,3-dioxolan-4-yl)-methoxy)-phenyl)-5,6,7,8-tetrahydroimidazo[1,5a]-pyridin-1,3(2H, 8aH)-dion als Öl.

$^1$H-NMR (200 MHz, $CDCl_3$, $\delta$): 1,40 und 1,46 (2s, 6H, 2x $CH_3$); 4,48 (m, 1H, -OCH<);

6,88 (d, 1H, 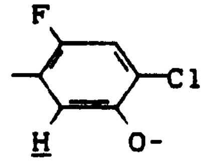 );

7,29 (d, 1H, 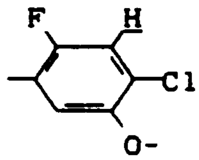 ).

Analog Beispiel 1 oder 2 und/oder entsprechend der allgemeinen Beschreibung der erfindungsgemäßen Herstellungsverfahren können die in der nachstehenden Tabelle 2 aufgeführten Verbindungen der Formel (I) erhalten werden.

**Tabelle 2:** Beispiele für die Verbindungen der Formel (I)

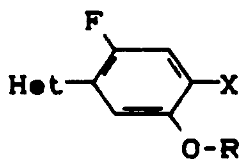 (I)

| Bsp.-Nr. | Het | X | R | Physikal. Daten (Schmp. °C) |
|---|---|---|---|---|
| 3 | 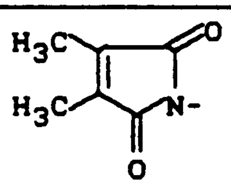 | Cl | $-CH_2CH_2-O-CH_2CH_2-O-C_2H_5$ | |
| 4 | 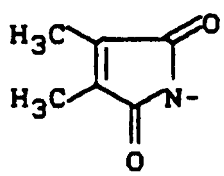 | Cl | $-CH_2CH_2-O-CH_2CH_2-O-C_4H_9$ | |

**Tabelle 2** - Fortsetzung

| Bsp.-Nr. | Het | X | R | Physikal. Daten (Schmp. °C) |
|---|---|---|---|---|
| 5 | 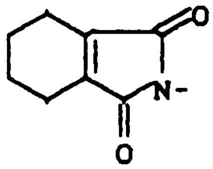 | Cl | $-CH_2CH_2-O-CH_2CH_2-O-C_4H_9$ | |
| 6 | 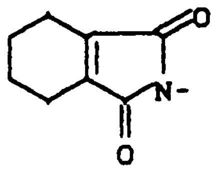 | Cl | $-CH_2CH_2-O-CH_2CH_2-O-CH_3$ | |
| 7 | 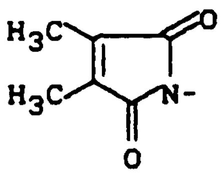 | Cl | $-CH_2CH_2-O-CH_2-CH_2-O-CH_3$ | |
| 8 | 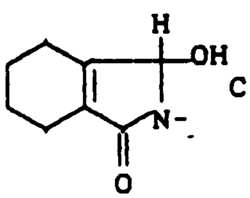 | Cl | $-CH_2CH_2-O-CH_2CH_2-O-C_2H_5$ | |
| 9 | 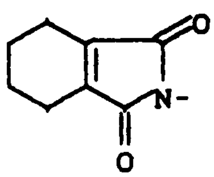 | Cl | 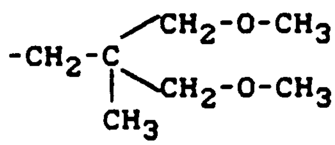 | 75 |
| 10 | 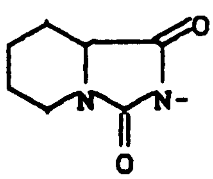 | Cl | 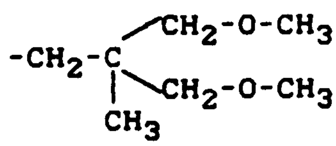 | 78 |
| 11 | 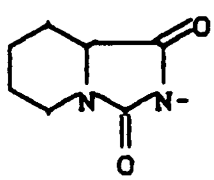 | Cl | $-CH_2CH_2-O-CH_2CH_2-O-CH_3$ | Öl |

Ausgangsstoffe der Formel (III)

Beispiel (III-1)

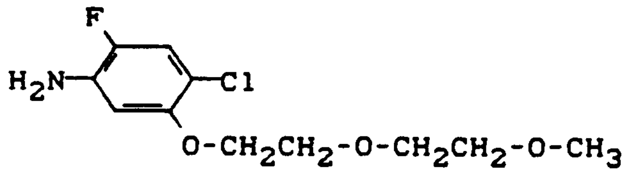

20 g (0,124 Mol) 4-Chlor-2-fluor-5-hydroxy-anilin werden in 140 ml N-Methylpyrrolidon gelöst und nacheinander mit 10,2 g (0,186 Mol) Kaliumhydroxid in 12 ml Wasser sowie 35,6 g (0,130 Mol) p-Toluolsulfonsäure-2-(2-methoxy-ethoxy)-ethylester versetzt. Es wird 18 Stunden bei Raumtemperatur gerührt und danach die Hauptmenge des Lösungsmittels im Vakuum entfernt. Der ölige Rückstand wird mit 200 ml Wasser versetzt und das sich abscheidende Öl mit Dichlormethan extrahiert. Die organische Phase wird abgetrennt, gewaschen, getrocknet und im Vakuum eingeengt.

Als Rückstand verbleiben 30,5 g (93,3 % der Theorie) 4-Chlor-2-fluor-5-[2-(2-methoxy-ethoxy)-ethoxy]-anilin als Öl.

$^1$H-NMR ($CDCl_3$): $\delta$ = 6,98 ppm (1H, d).

Beispiel (III-2)

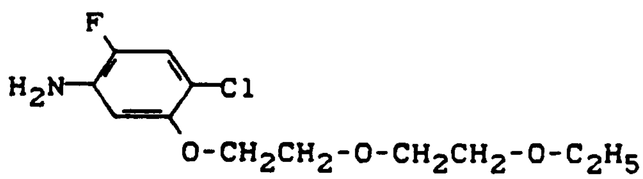

5,5 g (0,018 Mol) 2-Chlor-4-fluor-5-nitro-[2-(2-ethoxy-ethoxy)-ethyl]-phenol werden in 100 ml Ethanol gelöst, mit 1,0 g 5 %igem Platin auf Aktivkohle versetzt und bei 40 °C mit Wasserstoff unter Normaldruck hydriert. Nach beendeter Reaktion wird der Katalysator abfiltriert und das Lösungsmittel im Vakuum entfernt.

Man erhält 4,5 g (90,7 % der Theorie) 4-Chlor-2-fluor-5-[2-(2-ethoxy-ethoxy)-ethoxy]-anilin als Öl.

$^1$H-NMR ($CDCl_3$): $\delta$ = 7,00 ppm (1H, d).

Ausgangsstoffe der Formel (XI)

Beispiel (XI-1)

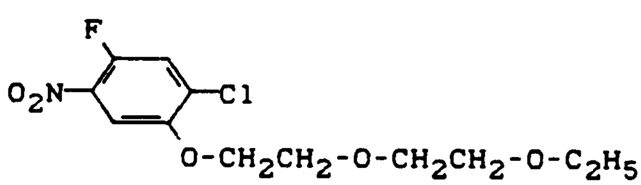

5 g (0,026 Mol) 2-Chlor-4-fluor-5-nitro-phenol werden in 10 ml Dimethylsulfoxid gelöst und nacheinander mit 0,8 g (0,0267 Mol) Natriumhydrid (80 %ig) und 7,7 g (0,0267 Mol) p-Toluolsulfonsäure-2-(2-ethoxy-ethoxy)-ethylester versetzt. Man erwärmt die Mischung 8 Stunden auf 80 °C, trägt danach auf Wasser aus und extrahiert das ausgefallene Öl mit Dichlormethan. Die organische Phase wird gewaschen, getrocknet und eingeengt.

Man erhält 5,9 g (72 % der Theorie) 4-Chlor-2-fluor-5-[2-(2-ethoxy-ethoxy)-ethoxy]-nitrobenzol.

$^1$H-NMR ($CDCl_3$): $\delta$ = 7,70 ppm (1H,d).

Anwendungsbeispiele

In den folgenden Anwendungsbeispielen wird die nachstehend aufgeführte Verbindung als Vergleichssubstanz herangezogen:

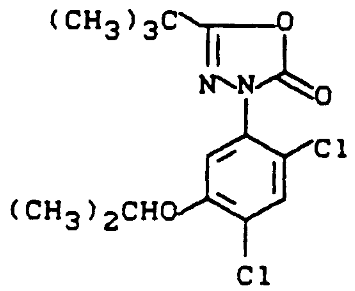

(A)

5-tert-Butyl-3-(2,4-dichlor-5-isopropoxy-phenyl)-1,3,4-oxadiazol-2-on (Oxadiazone/®Ronstar)
- bekannt aus US-P 3 835 862.

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigt beispielsweise die Verbindung gemäß Herstellungsbeispiel (1) eine deutliche Überlegenheit in der Wirksamkeit gegenüber der bekannten Verbindung (A).

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigt beispielsweise die Verbindung gemäß Herstellungsbeispiel (1) eine deutliche Überlegenheit in der Wirksamkeit gegenüber der bekannten Verbindung (A).

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

| | |
|---|---|
| 0 | kein Austrocknen der Blätter, kein Blattfall |
| + | leichtes Austrocknen der Blätter, geringer Blattfall |
| + + | starkes Austrocknen der Blätter, starker Blattfall |
| + + + | sehr starkes Austrocknen der Blätter, sehr starker Blattfall |

Eine deutliche Überlegenheit im Vergleich zur unbehandelten Kontrolle zeigt in diesem Test beispielsweise die Verbindung gemäß Herstellungsbeispiel: (1).

**Patentansprüche**

**1.** Substituierte N-Aryl-Stickstoffheterocyclen der allgemeinen Formel (I)

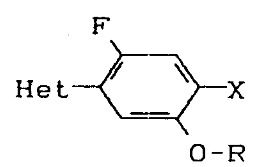

(I)

in welcher
Het für eine der nachstehenden heterocyclischen Gruppierungen

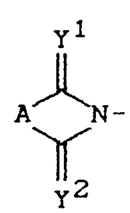

oder

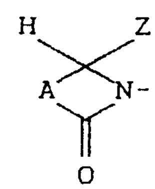

steht,

wobei
A für eine der nachstehenden Gruppierungen steht,

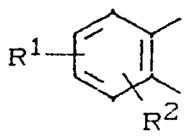 , 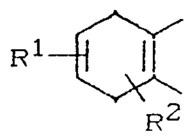 , 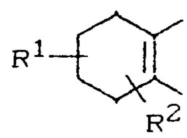 , 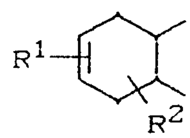 ,

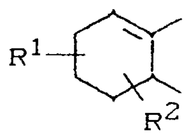 , 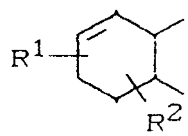 , 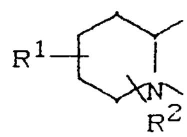 , 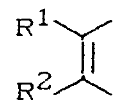 ,

wobei

$R^1$ und $R^2$ jeweils unabhängig voneinander für Wasserstoff, Halogen, Halogenalkyl oder Alkyl stehen,

$Y^1$ und $Y^2$ jeweils für Sauerstoff oder Schwefel stehen und

Z für Wasserstoff, Hydroxy oder Chlor steht,

R für jeweils gegebenenfalls verzweigtes und jeweils durch wenigstens 1 Sauerstoffatom unterbrochenes Alkyl, Alkenyl, Cycloalkyl, Cycloalkenyl, Cycloalkylalkyl oder Cycloalkenylalkyl steht und

X für Wasserstoff oder Halogen steht, ausgenommen die Verbindungen in denen

Het für

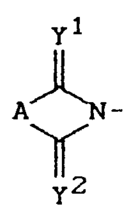 oder 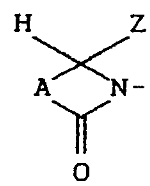 steht,

wobei

A für die Gruppierung

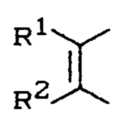 steht,

Z für Hydroxy steht,

X für Wasserstoff oder Halogen steht,

$R^1$ und $R^2$ jeweils unabhängig voneinander für Wasserstoff oder Alkyl stehen,

$Y^1$ für Sauerstoff oder Schwefel steht,

$Y^2$ für Sauerstoff steht und

R für Alkoxyalkyl, Oxiranyl alkyl oder für gegebenenfalls durch 1 oder 2 $C_1$-$C_4$-alkylsubstituierte Dioxolan-2-yl- und 1,3-Dioxan-2-yl-reste steht,

sowie die Verbindungen, in denen

Het für

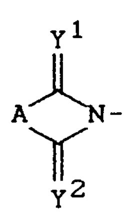 oder 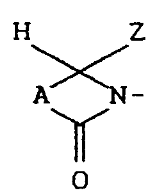 steht,

A für die Gruppierung

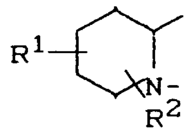 steht,

$R^1$ und $R^2$ jeweils unabhängig voneinander für Wasserstoff oder Alkyl stehen,
$Y^1$ für Sauerstoff oder Schwefel steht,
$Y^2$ für Sauerstoff oder Schwefel steht,
X für Sauerstoff oder Halogen steht,
Z für Hydroxy steht, und
R für Alkoxyalkyl, Furanylalkyl, Pyranylalkyl und Oxiranyl alkyl steht,
sowie die Verbindungen, in denen
Het für

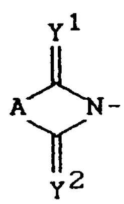 oder 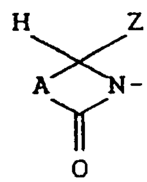 steht,

A für die Gruppierung

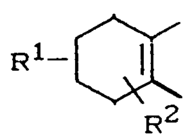 steht,

$R^1$ und $R^2$ jeweils unabhängig voneinander für Wasserstoff oder Alkyl stehen,
$Y^1$ für Sauerstoff oder Schwefel steht,
$Y^2$ für Sauerstoff oder Schwefel steht,
X für Wasserstoff oder Halogen steht,
Z für Hydroxy steht und
R für Alkoxyalkoxy,

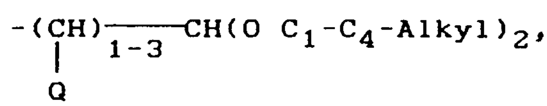

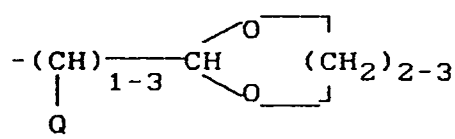

oder für durch wenigstens ein Sauerstoffatom unterbrochenes Cycloalkylalkyl oder Cycloalkenylalkyl steht und
Q für Wasserstoff oder $C_1$-$C_4$-Alkyl steht.

**2.** Verbindungen der allgemeinen Formel (I) gemäß Anspruch 1, dadurch gekennzeichnet, daß darin
Het für eine der nachstehenden heterocyclischen Gruppierungen

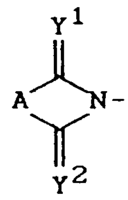 oder 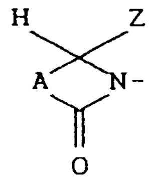 steht,

wobei

A für eine der nachstehenden Gruppierungen steht,

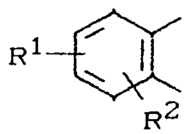 , 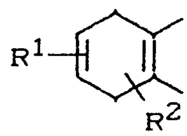 , 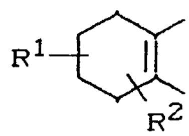 , 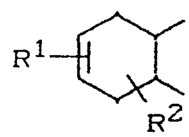 ,

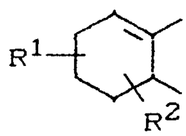 , 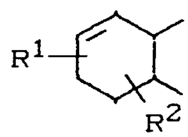 , 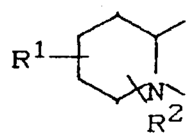 , 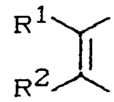 ,

wobei

$R^1$ und $R^2$ jeweils unabhängig voneinander für Wasserstoff, Fluor, Chlor, Brom oder für jeweils geradkettiges oder verzweigtes Alkyl oder Halogenalkyl mit jeweils 1 bis 3 Kohlenstoffatomen und im Fall des Halogenalkyl mit 1 bis 5 gleichen oder verschiedenen Halogenatomen, insbesondere Fluor oder Chlor, stehen,

$Y^1$ und $Y^2$ für Sauerstoff oder Schwefel stehen,

Z für Wasserstoff, Hydroxy oder Chlor steht,

R für jeweils gegebenenfalls verzweigtes und jeweils durch 1 bis 4 Sauerstoffatome unterbrochenes Alkyl, Alkenyl, Cycloalkyl, Cycloalkenyl, Cycloalkylalkyl oder Cycloalkenylalkyl mit jeweils bis zu 20 Kohlenstoffatomen steht und

X für Wasserstoff, Fluor, Chlor oder Brom steht, ausgenommen die Verbindungen in denen

Het für

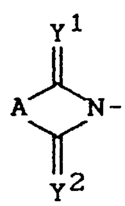 oder 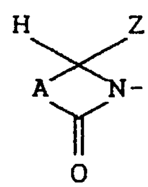 steht,

wobei

A für die Gruppierung

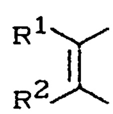 steht,

Z für Hydroxy steht,

X für Wasserstoff oder Halogen steht,

$R^1$ und $R^2$ jeweils unabhängig voneinander für Wasserstoff oder Alkyl stehen,

| | |
|---|---|
| $Y^1$ | für Sauerstoff oder Schwefel steht, |
| $Y^2$ | für Sauerstoff steht und |
| R | für Alkoxyalkyl, Oxiranyl alkyl oder für gegebenenfalls durch 1 oder 2 $C_1$-$C_4$-alkylsubstituierte Dioxolan-2-yl- und 1,3-Dioxan-2-yl-reste steht, |
| | sowie die Verbindungen, in denen |
| Het | für |

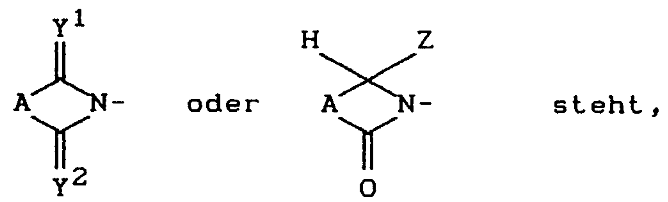

| | |
|---|---|
| A | für die Gruppierung |

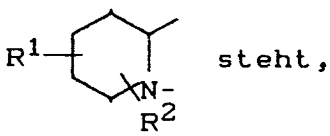

| | |
|---|---|
| $R^1$ und $R^2$ | jeweils unabhängig voneinander für Wasserstoff oder Alkyl steht, |
| $Y^1$ | für Sauerstoff oder Schwefel steht, |
| $Y^2$ | für Sauerstoff oder Schwefel steht, |
| X | für Sauerstoff oder Halogen steht, |
| Z | für Hydroxy steht, und |
| R | für Alkoxyalkyl, Furanylalkyl, Pyranylalkyl und Oxiranyl alkyl steht, |
| | sowie die Verbindungen, in denen |
| Het | für |

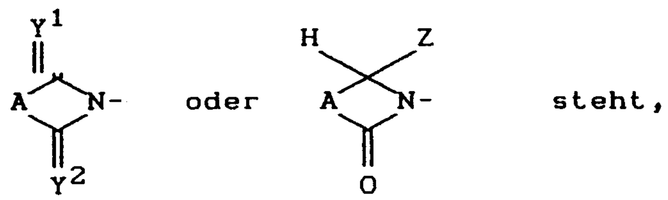

| | |
|---|---|
| A | für die Gruppierung |

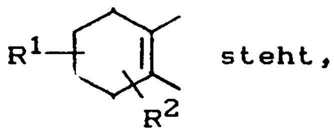

| | |
|---|---|
| $R^1$ und $R^2$ | jeweils unabhängig voneinander für Wasserstoff oder Alkyl stehen, |
| $Y^1$ | für Sauerstoff oder Schwefel steht, |
| $Y^2$ | für Sauerstoff oder Schwefel steht, |
| X | für Wasserstoff oder Halogen steht, |
| Z | für Hydroxy steht und |
| R | für Alkoxyalkoxy, |

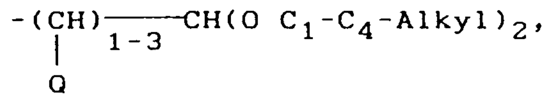

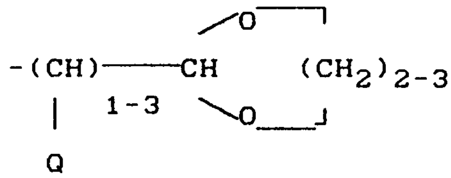 oder für

durch wenigstens ein Sauerstoffatom unterbrochenes Cycloalkylalkyl oder Cycloalkenylalkyl steht und

Q für Wasserstoff oder $C_1$-$C_4$-Alkyl steht.

**3.** Verbindungen der allgemeinen Formel (I) gemäß Anspruch 1, dadurch gekennzeichnet, daß darin

Het für eine der nachstehenden heterocyclischen Gruppierungen

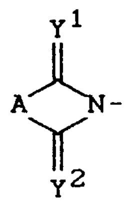 oder 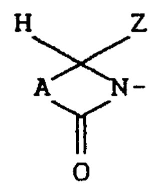 steht,

wobei

A für eine der nachstehenden Gruppierungen steht,

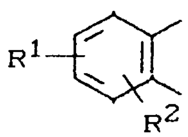 , 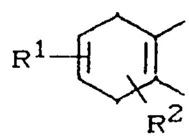 , 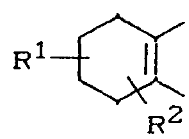 , 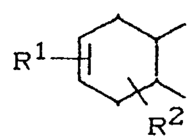 ,

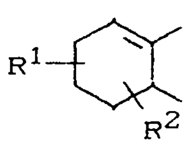 , 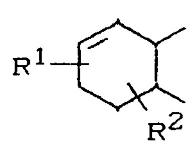 , 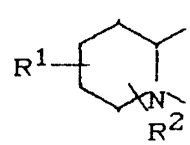 , 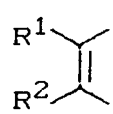 ,

wobei

$R^1$ und $R^2$ jeweils unabhängig voneinander für Wasserstoff, Methyl oder Trifluormethyl stehen,

$Y^2$ und $Y^2$ für Sauerstoff oder Schwefel stehen,

Z für Wasserstoff, Hydroxy oder Chlor steht,

R für jeweils gegebenenfalls verzweigtes Oxaalkyl, Oxaalkenyl oder Dioxaalkyl mit jeweils bis zu 10 Kohlenstoffatomen oder für Oxacycloalkyl-alkyl, Oxacycloalkenyl-alkyl, oder Dioxacycloalkyl-alkyl mit jeweils bis zu 8 Kohlenstoffatomen im Oxacycloalkyl-, Oxacycloalkenyl- oder Dioxacycloalkylteil und bis zu 3 Kohlenstoffatomen im Alkylteil steht und

X für Wasserstoff, Chlor oder Brom steht, ausgenommen die Verbindungen in denen

Het für

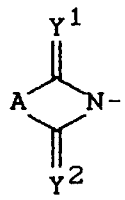 oder 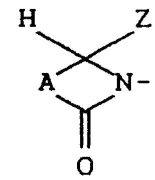 steht,

wobei

A für die Gruppierung

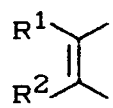 steht,

Z für Hydroxy steht,

X für Wasserstoff oder Halogen steht,

$R^1$ und $R^2$ jeweils unabhängig voneinander für Wasserstoff oder Alkyl stehen,

$Y^1$ für Sauerstoff oder Schwefel steht,

$Y^2$ für Sauerstoff steht und

R für Alkoxyalkyl, Oxiranyl alkyl oder für gegebenenfalls durch 1 oder 2 $C_1$-$C_4$-alkylsubstituierte Dioxolan-2-yl- und 1,3-Dioxan-2-yl-reste steht,

sowie die Verbindungen, in denen

Het für

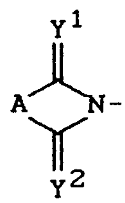 oder 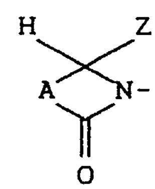 steht,

A für die Gruppierung

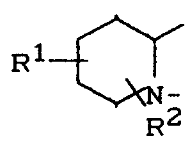 steht,

$R^1$ und $R^2$ jeweils unabhängig voneinander für Wasserstoff oder Alkyl steht,

$Y^1$ für Sauerstoff oder Schwefel steht,

$Y^2$ für Sauerstoff oder Schwefel steht,

X für Sauerstoff oder Halogen steht,

Z für Hydroxy steht, und

R für Alkoxyalkyl, Furanylalkyl, Pyranylalkyl und Oxiranyl alkyl steht,

sowie die Verbindungen, in denen

Het für

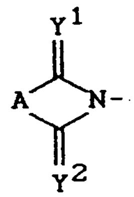 oder 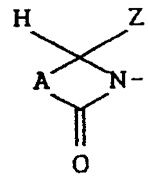 steht,

A für die Gruppierung

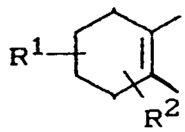 steht,

$R^1$ und $R^2$ jeweils unabhängig voneinander für Wasserstoff oder Alkyl stehen,
$Y^1$ für Sauerstoff oder Schwefel steht,
$Y^2$ für Sauerstoff oder Schwefel steht,
X für Wasserstoff oder Halogen steht,
Z für Hydroxy steht und
R für Alkoxyalkoxy,

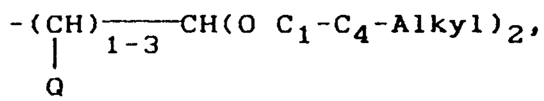

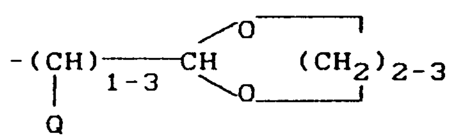

oder für durch wenigstens ein Sauerstoffatom unterbrochenes Cycloalkylalkyl oder Cycloalkenylalkyl steht und
Q für Wasserstoff oder $C_1$-$C_4$-Alkyl steht.

**4.** Verfahren zur Herstellung von substituierten N-Aryl-Stickstoffheterocyclen der allgemeinen Formel (I) gemäß Anspruch 1, dadurch gekennzeichnet, daß man
(a) für den Fall, daß Het für die Gruppierung

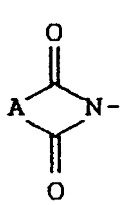 steht und

A, R und X die in Anspruch 1 angegebenen Bedeutungen haben,
cyclische Anhydride der allgemeinen Formel (II)

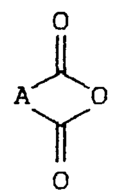

(II)

in welcher

A die in Anspruch 1 angegebene Bedeutung hat,

mit Arylaminen der allgemeinen Formel (III)

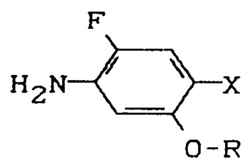

(III)

in welcher

R und X die in Anspruch 1 angegebenen Bedeutungen haben,

gegebenenfalls in Gegenwart eines Verdünnungsmittels und gegebenenfalls in Gegenwart eines Reaktionshilfsmittels umsetzt, oder daß man

(b) für den Fall, daß Het für die Gruppierung

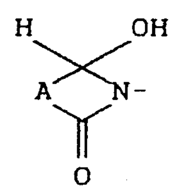

steht und

A, R und X die in Anspruch 1 angegebenen Bedeutungen haben,

substituierte Arylimide der allgemeinen Formel (Ia)

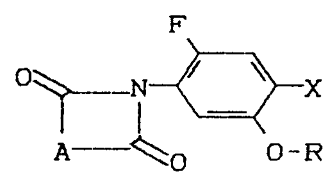

(Ia)

in welcher

A, R und X die in Anspruch 1 angegebenen Bedeutungen haben,

mit einem Reduktionsmittel, gegebenenfalls in Gegenwart eines Verdünnungsmittels umsetzt, oder daß man

(c) für den Fall, daß Het für die Gruppierung

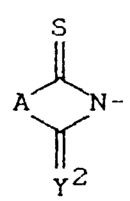

steht und

A, R, X und $Y^2$ die in Anspruch 1 angegebenen Bedeutungen haben, substituierte Arylimide der allgemeinen Formel (Ia)

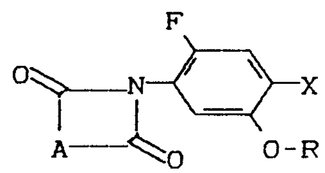

(Ia)

in welcher

A, R und X die in Anspruch 1 angegebenen Bedeutungen haben,

mit einem Schwefelungsmittel, gegebenenfalls in Gegenwart eines Verdünnungsmittel umsetzt, oder daß man

(d) für den Fall, daß Het für die Gruppierung

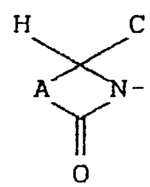

steht und

A, R und X die in Anspruch 1 angegebenen Bedeutungen haben, N-Aryl-Stickstoffheterocyclen der allgemeinen Formel (Ib)

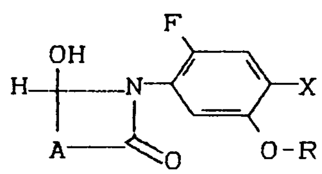

(Ib)

in welcher

A, R und X die in Anspruch 1 angegebenen Bedeutungen haben,

mit Thionylchlorid, gegebenenfalls in Gegenwart eines Verdünnungsmittels und gegebenenfalls in Gegenwart eines Reaktionshilfsmittels umsetzt, oder daß man

(e) für den Fall, daß Het für die Gruppierung

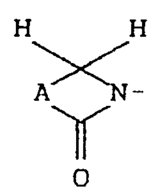

steht und

A, R und X die in Anspruch 1 angegebenen Bedeutungen haben, N-Aryl-Stickstoffheterocyclen der allgemeinen Formel (Ic)

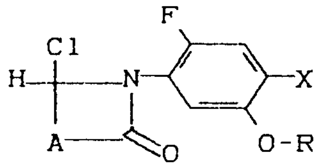

(Ic)

in welcher
A, R und X die in Anspruch 1 angegebenen Bedeutungen haben,
mit Wasserstoff, in Gegenwart eines Katalysators sowie gegebenenfalls in Gegenwart eines Verdünnungsmittels und gegebenenfalls in Gegenwart eines Säurebindemittels umsetzt, oder daß man
(f) für den Fall, daß Het für die Gruppierung

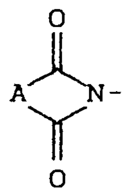

steht und

A, R und X die in Anspruch 1 angegebenen Bedeutungen haben,
Hydroxyarylimide der allgemeinen Formel (IV)

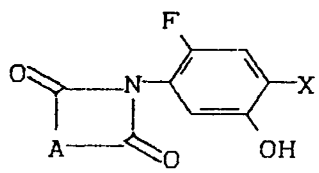

(IV)

in welcher
A und X die in Anspruch 1 angegebenen Bedeutungen haben,
mit Alkylierungsmitteln der allgemeinen Formel

$X^1$ - R (V)

in welcher
R die in Anspruch 1 angegebene Bedeutung hat und
$X^1$ für eine nucleophile Abgangsgruppe steht,
gegebenenfalls in Gegenwart eines Säureakzeptors und gegebenenfalls in Gegenwart eines Verdünnungsmittels umsetzt, oder daß man
(g) für den Fall, daß Het für die Gruppierung

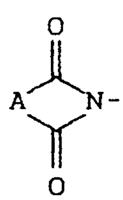

steht und

A und R die in Aspruch 1 angegebenen Bedeutungen haben sowie
X für Halogen steht,
substituierte Arylimide der allgemeinen Formel (Id)

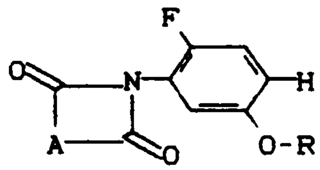

(Id')

in welcher

A und R die in Anspruch 1 angegebenen Bedeutungen haben,

mit einem Halogenierungsmittel, gegebenenfalls in Gegenwart eines Katalysators und gegebenenfalls in Gegenwart eines Verdünnungsmittels umsetzt, oder daß man

(h) für den Fall, daß Het für die Gruppierung

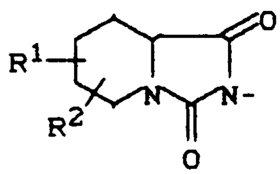

steht und

R, $R^1$, $R^2$ und X die in Anspruch 1 angegebenen Bedeutungen haben

Arylamine der allgemeinen Formel (III)

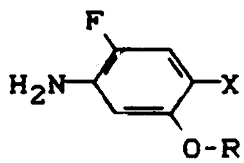

(III)

in welcher

R und X die in Anspruch 1 angegebenen Bedeutungen haben,

mit Chlorameisensäureestern der allgemeinen Formel (VI)

$R^3O$ - CO - Cl (VI)

in welcher

$R^3$ für Niederalkyl, Benzyl oder Phenyl steht,

gegebenenfalls in Gegenwart eines Säureakzeptors und gegebenenfalls in Gegenwart eines Verdünnungsmittels umsetzt und die hierbei gebildeten

Arylurethane der allgemeinen Formel (VII)

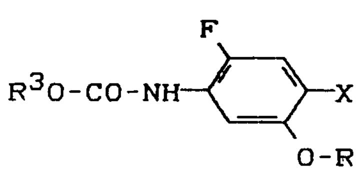

(VII)

in welcher

R, $R^3$ und X die in Anspruch1 angegebene Bedeutung haben

mit Piperidin-2-carbonsäureestern der allgemeinen Formel (VIII)

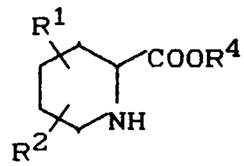 (VIII)

in welcher

$R^1$ und $R^2$ die in Anspruch 1 angegebenen Bedeutungen haben und

$R^4$ für Niederalkyl steht,

gegebenenfalls in Gegenwart eines Verdünnungsmittels umsetzt, ausgenommen die Verbindungen in denen

Het für

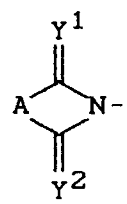 oder 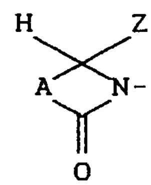 steht,

wobei

A für die Gruppierung

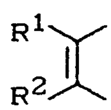 steht,

Z für Hydroxy steht,

X für Wasserstoff oder Halogen steht,

$R^1$ und $R^2$ jeweils unabhängig voneinander für Wasserstoff oder Alkyl stehen,

$Y^1$ für Sauerstoff oder Schwefel steht,

$Y^2$ für Sauerstoff steht und

R für Alkoxyalkyl, Oxiranyl alkyl oder für gegebenenfalls durch 1 oder 2 $C_1$-$C_4$-alkylsubstituierte Dioxolan-2-yl- und 1,3-Dioxan-2-yl-reste steht,

sowie die Verbindungen, in denen

Het für

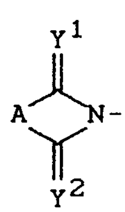 oder 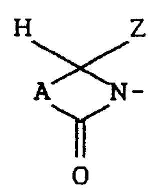 steht,

A für die Gruppierung

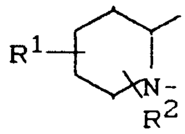 steht,

| | |
|---|---|
| $R^1$ und $R^2$ | jeweils unabhängig voneinander für Wasserstoff oder Alkyl steht, |
| $Y^1$ | für Sauerstoff oder Schwefel steht, |
| $Y^2$ | für Sauerstoff steht, |
| X | für Sauerstoff oder Halogen steht, |
| Z | für Hydroxy steht, und |
| R | für Alkoxyalkyl, Furanylalkyl, Pyranylalkyl und Oxiranyl alkyl steht, |
| | sowie die Verbindungen, in denen |
| Het | für |

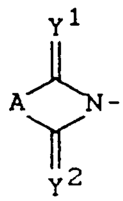 oder 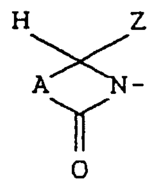 steht,

| | |
|---|---|
| A | für die Gruppierung |

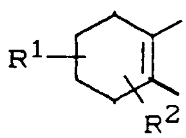 steht,

| | |
|---|---|
| $R^1$ und $R^2$ | jeweils unabhängig voneinander für Wasserstoff oder Alkyl stehen, |
| $Y^1$ | für Sauerstoff oder Schwefel steht, |
| $Y^2$ | für Sauerstoff oder Schwefel steht, |
| X | für Wasserstoff oder Halogen steht, |
| Z | für Hydroxy steht und |
| R | für Alkoxyalkoxy, |

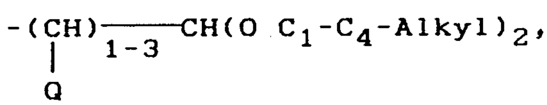

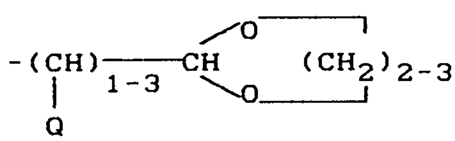

oder durch wenigstens ein Sauerstoffatom unterbrochenes Cycloalkylalkyl oder Cycloalkenylalkyl steht und

| | |
|---|---|
| Q | für Wasserstoff oder $C_1$-$C_4$-Alkyl steht. |

**5.** Herbizide und pflanzenwuchsregulierende Mittel, gekennzeichnet durch einen Gehalt an mindestens einem N-Aryl-Stickstoffheterocyclus der Formel (I) gemäß den Ansprüchen 1 bis 4.

6. Verfahren zur Bekämpfung von Unkräutern, dadurch gekennzeichnet, daß man N-Aryl-Stickstoffheterocyclen der Formel (I) gemäß den Ansprüchen 1 bis 4 auf die Unkräuter und/oder ihren Lebensraum einwirken läßt.

7. Verwendung von N-Aryl-Stickstoffheterocyclen der Formel (I) gemäß den Ansprüchen 1 bis 4 zur Bekämpfung von Unkräutern und/oder als Pflanzenwachstumsregulatoren.

8. Verfahren zur Herstellung von herbiziden und/oder pflanzenwuchsregulierenden Mitteln, dadurch gekennzeichnet, daß man N-Aryl-Stickstoffheterocyclen der Formel (I) gemäß den Ansprüchen 1 bis 4 mit Streckmitteln und/oder oberflächenaktiven Substanzen vermischt.

9. Arylamine der allgemeinen Formel (III)

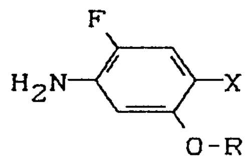

(III)

in welcher

R für jeweils gegebenenfalls verzweigtes und jeweils durch wenigstens 1 Sauerstoffatom unterbrochenes Alkyl, Alkenyl, Cycloalkyl, Cycloalkenyl, Cycloalkylalkyl oder Cycloalkenylalkyl steht und

X für Wasserstoff oder Halogen steht, ausgenommen die Verbindungen in denen

R für Alkoxyalkyl,

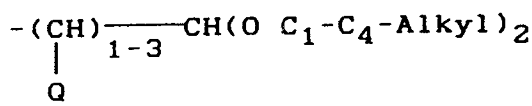,

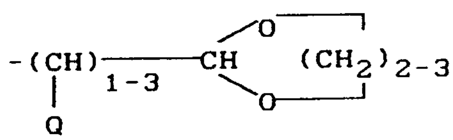,

durch 1 oder 2 alkylsubstituiertes und durch wenigstens ein Sauerstoffatom unterbrochenes Cycloalkylalkyl oder Cycloalkenylalkyl steht und

Q für Wasserstoff oder $C_1$-$C_4$-Alkyl steht

**Claims**

1. Substituted N-aryl nitrogen heterocycles of the general formula (I)

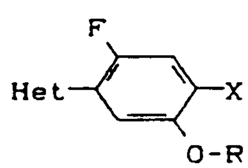

(I)

in which

Het represents one of the heterocyclic groups below

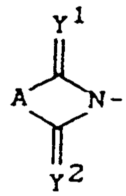 or 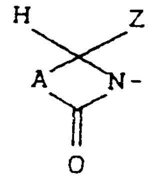

where

A represents one of the groups below

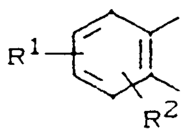 , 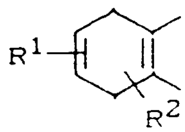 , 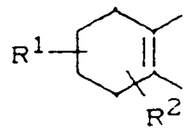 , 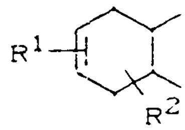 ,

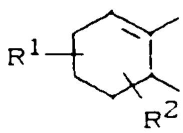 , 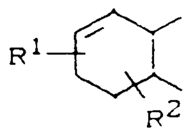 , 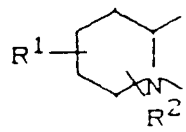 , 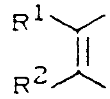 ,

where

$R^1$ and $R^2$ in each case independently of one another represent hydrogen, halogen, halogenoalkyl or alkyl,

$Y^1$ and $Y^2$ in each case represent oxygen or sulphur and

Z represents hydrogen, hydroxyl or chlorine,

R represents alkyl, alkenyl, cycloalkyl, cycloalkenyl, cycloalkylalkyl or cycloalkenylalkyl, in each case optionally branched and in each case interrupted by at least 1 oxygen atom, and

X represents hydrogen or halogen, excluding the compounds in which

Het represents

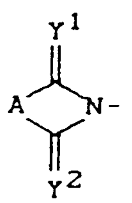 or 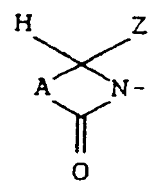

where

A represents the group

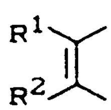

Z represents hydroxyl,

X represents hydrogen or halogen,

$R^1$ and $R^2$ in each case independently of one another represent hydrogen or alkyl,

$Y^1$ represents oxygen or sulphur,

$Y^2$ represents oxygen and

R represents alkoxyalkyl, oxiranylalkyl or dioxolan-2-yl and 1,3-dioxan-2-yl radicals optionally substituted by 1 or 2 $C_1$-$C_4$-alkyl, and the compounds in which

Het represents

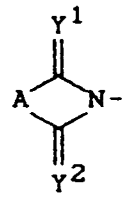

or

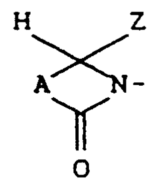

where

A represents the group

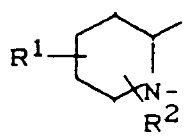

$R^1$ and $R^2$ in each case independently of one another represent hydrogen or alkyl,

$Y^1$ represents oxygen or sulphur,

$Y^2$ represents oxygen or sulphur,

X represents oxygen or halogen,

Z represents hydroxyl, and

R represents alkoxyalkyl, furanylalkyl, pyranylalkyl and oxiranylalkyl,

and the compounds in which

Het represents

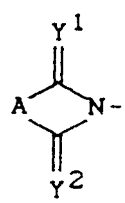

or

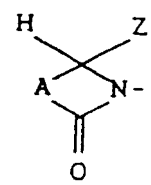

where

A represents the group

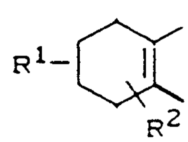

$R^1$ and $R^2$ in each case independently of one another represent hydrogen or alkyl,

$Y^1$ represents oxygen or sulphur,

$Y^2$ represents oxygen or sulphur,

X represents hydrogen or halogen,

Z represents hydroxyl and

R represents alkoxyalkoxy,

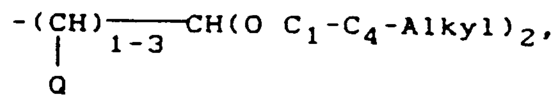

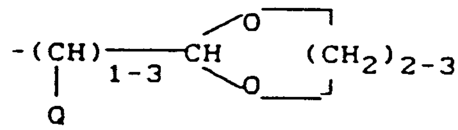

or cycloalkylalkyl or cycloalkenylalkyl interrupted by at least one oxygen atom and

Q represents hydrogen or $C_1$-$C_4$-alkyl.

**2.** Compounds of the general formula (I) according to Claim 1, characterised in that, in this formula,

Het represents one of the heterocyclic groups below

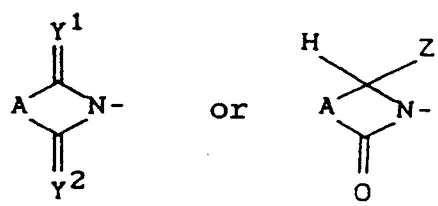

or

where

A represents one of the groups below

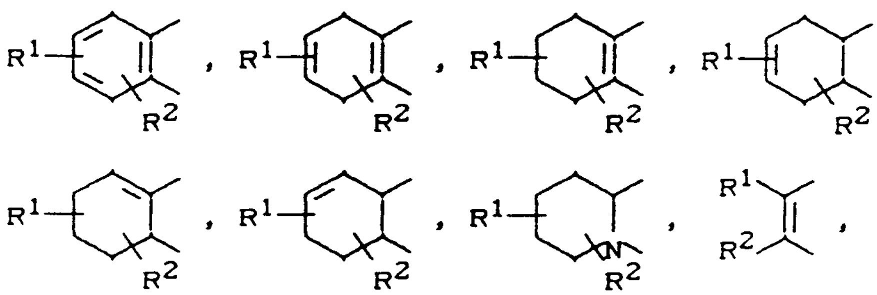

, , , , , , ,

where

$R^1$ and $R^2$ in each case independently of one another represent hydrogen, fluorine, chlorine, bromine or in each case straight-chain or branched alkyl or halogenoalkyl, in each case having 1 to 3 carbon atoms and, in the case of halogenoalkyl, having 1 to 5 identical or different halogen atoms, in particular fluorine or chlorine,

$Y^1$ and $Y^2$ represent oxygen or sulphur,

Z represents hydrogen, hydroxyl or chlorine,

R represents alkyl, alkenyl, cycloalkyl, cycloalkenyl, cycloalkylalkyl or cycloalkenylalkyl, in each case having up to 20 carbon atoms, in each case optionally branched and in each case interrupted by 1 to 4 oxygen atoms, and

X represents hydrogen, fluorine, chlorine or bromine excluding the compounds in which

Het represents

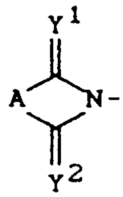

or

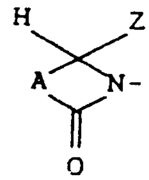

where

A represents the group

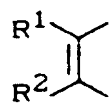

Z represents hydroxyl,

X represents hydrogen or halogen,

$R^1$ and $R^2$ in each case independently of one another represent hydrogen or alkyl,

$Y^1$ represents oxygen or sulphur,

$Y^2$ represents oxygen and

R represents alkoxyalkyl, oxiranylalkyl or dioxolan-2-yl and 1,3-dioxan-2-yl radicals optionally substituted by 1 or 2 $C_1$-$C_4$-alkyl,

and the compounds in which

Het represents

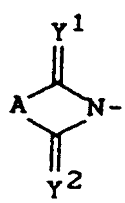

or

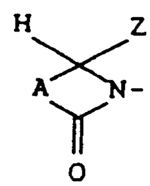

where

A represents the group

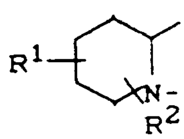

$R^1$ and $R^2$ in each case independently of one another represent hydrogen or alkyl,

$Y^1$ represents oxygen or sulphur,

$Y^2$ represents oxygen or sulphur,

X represents oxygen or halogen,

Z represents hydroxyl, and

R represents alkoxyalkyl, furanylalkyl, pyranylalkyl and oxiranylalkyl,

and the compounds in which

Het represents

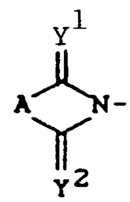 or 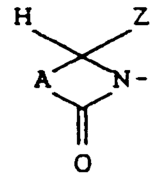

A represents the group

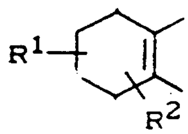

where

$R^1$ and $R^2$ in each case independently of one another represent hydrogen or alkyl,
$Y^1$ represents oxygen or sulphur,
$Y^2$ represents oxygen or sulphur,
X represents hydrogen or halogen,
Z represents hydroxyl and
R represents alkoxyalkoxy,

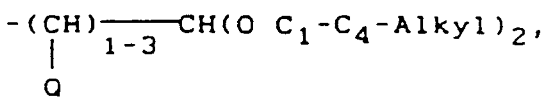

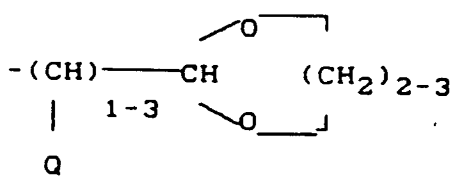 or

cycloalkylalkyl or cycloalkenylalkyl interrupted by at least one oxygen atom and
Q represents hydrogen or $C_1$-$C_4$-alkyl.

**3.** Compounds of the general formula (I) according to Claim 1, characterised in that, in this formula,
Het represents one of the heterocyclic groups below

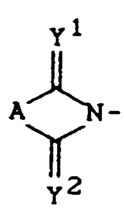 or 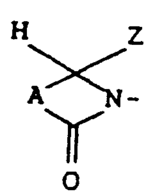

where
A represents one of the groups below

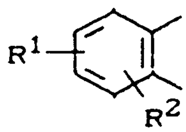, 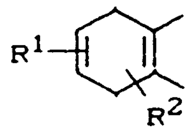, 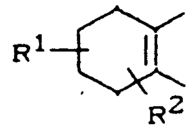, 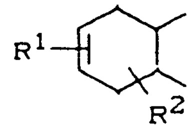,

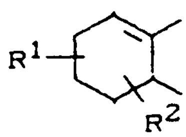, 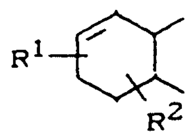, 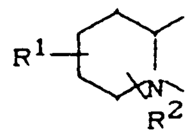, 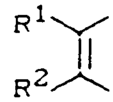,

wherein

| | |
|---|---|
| $R^1$ and $R^2$ | in each case independently of one another represent hydrogen, methyl or trifluoromethyl, |
| $Y^1$ and $Y^2$ | represent oxygen or sulphur, |
| Z | represents hydrogen, hydroxyl or chlorine, |
| R | represents oxaalkyl, oxaalkenyl or dioxaalkyl, in each case having up to 10 carbon atoms and in each case optionally branched, or oxacycloalkyl-alkyl, oxacycloalkenyl-alkyl or dioxacycloalkyl-alkyl, in each case having up to 8 carbon atoms in the oxacycloalkyl moiety, oxacycloalkenyl moiety or dioxacycloalkyl moiety and up to 3 carbon atoms in the alkyl moiety, and |
| X | represents hydrogen, chlorine or bromine excluding the compounds in which |
| Het | represents |

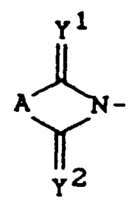 or 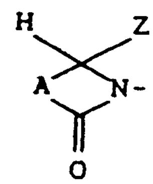

where

| | |
|---|---|
| A | represents the group |

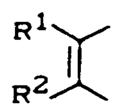

| | |
|---|---|
| Z | represents hydroxyl, |
| X | represents hydrogen or halogen, |
| $R^1$ and $R^2$ | in each case independently of one another represent hydrogen or alkyl, |
| $Y^1$ | represents oxygen or sulphur, |
| $Y^2$ | represents oxygen and |
| R | represents alkoxyalkyl, oxiranylalkyl or dioxolan-2-yl and 1,3-dioxan-2-yl radicals optionally substituted by 1 or 2 $C_1$-$C_4$-alkyl, |
| | and the compounds in which |
| Het | represents |

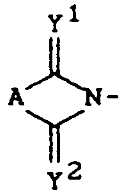 or 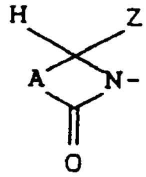

where

A represents the group

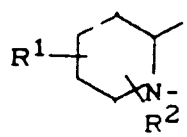

$R^1$ and $R^2$ in each case independently of one another represent hydrogen or alkyl,
$Y^1$ represents oxygen or sulphur,
$Y^2$ represents oxygen or sulphur,
X represents oxygen or halogen,
Z represents hydroxyl, and
R represents alkoxyalkyl, furanylalkyl, pyranylalkyl and oxiranylalkyl,
and the compounds in which
Het represents

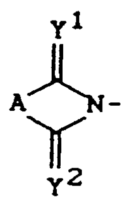 or 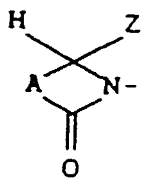

where

A represents the group

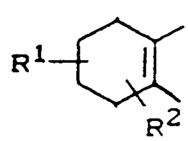

$R^1$ and $R^2$ in each case independently of one another represent hydrogen or alkyl,
$Y^1$ represents oxygen or sulphur,
$Y^2$ represents oxygen or sulphur,
X represents hydrogen or halogen,
Z represents hydroxyl and
R represents alkoxyalkoxy,

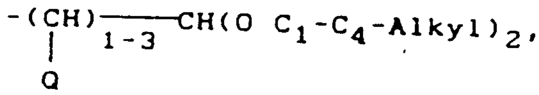

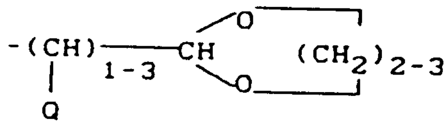

or

cycloalkylalkyl or cycloalkenylalkyl interrupted by at least one oxygen atom and

Q represents hydrogen or $C_1$-$C_4$-alkyl.

**4.** Process for the preparation of substituted N-aryl nitrogen heterocycles of the general formula (I) according to Claim 1, characterised in that

(a) in the event that Het represents the group

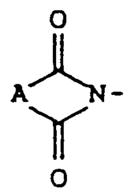

and

A, R and X have the meanings mentioned in Claim 1,

cyclic anhydrides of the general formula (II)

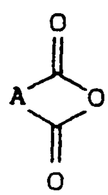

(II)

in which

A has the meaning mentioned in Claim 1,

are reacted with arylamines of the general formula (III)

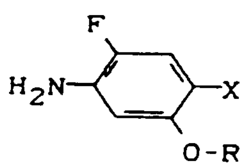

(III)

in which

R and X have the meanings mentioned in Claim 1,

if appropriate in the presence of a diluent and if appropriate in the presence of a reaction auxiliary, or in that

(b) in the event that Het represents the group

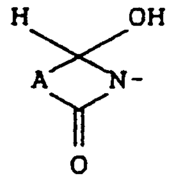

and

A, R and X have the meanings mentioned in Claim 1,

substituted arylimides of the general formula (Ia)

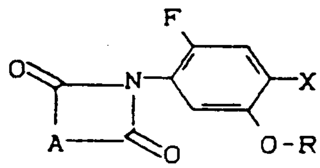

(Ia)

in which

A, R and X have the meanings mentioned in Claim 1,

are reacted with a reducing agent, if appropriate in the presence of a diluent, or in that

(c) in the event that Het represents the group

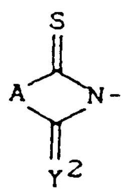

and

A, R, X and $Y^2$ have the meanings mentioned in Claim 1,

substituted arylimides of the general formula (Ia)

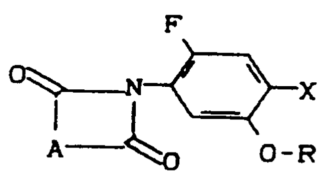

(Ia)

in which

A, R and X have the meanings mentioned in Claim 1,

are reacted with a sulphurising agent, if appropriate in the presence of a diluent, or in that

(d) in the event that Het represents the group

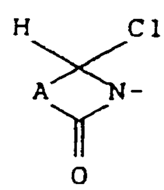

and

A, R and X have the meanings mentioned in Claim 1,
N-aryl nitrogen heterocycles of the general formula (Ib)

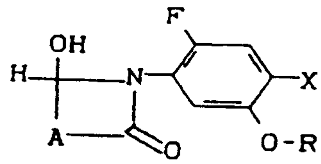

(Ib)

in which

A, R and X have the meanings mentioned in Claim 1,

are reacted with thionyl chloride, if appropriate in the presence of a diluent and if appropriate in the presence of a reaction auxiliary, or in that

(e) in the event that Het represents the group

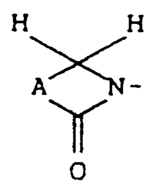

and

A, R and X have the meanings mentioned in Claim 1,
N-aryl nitrogen heterocycles of the general formula (Ic)

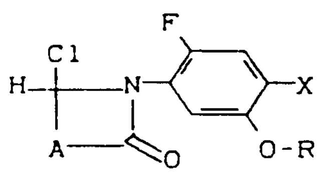

(Ic)

in which

A, R and X have the meanings mentioned in Claim 1,

are reacted with hydrogen, in the presence of a catalyst and also if appropriate in the presence of a diluent and if appropriate in the presence of an acid-binding agent, or in that

(f) in the event that Het represents the group

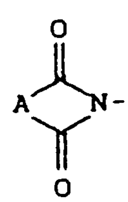

and

A, R and X have the meanings mentioned in Claim 1,

hydroxyarylimides of the general formula (IV)

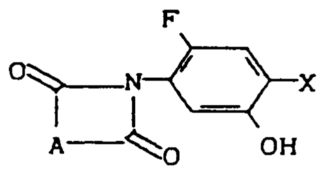

( IV )

in which
A and X have the meanings mentioned in Claim 1,
are reacted with alkylating agents of the general formula

$X^1$ - R (V)

in which
- R has the meaning mentioned in Claim 1 and
- $X^1$ represents a nucleophilic leaving group,

if appropriate in the presence of an acid acceptor and if appropriate in the presence of a diluent, or in that
(g) in the event that Het represents the group

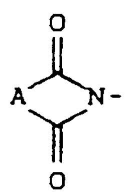

and
- A and R have the meanings mentioned in Claim 1 and also
- X represents halogen,

substituted arylimides of the general formula (Id)

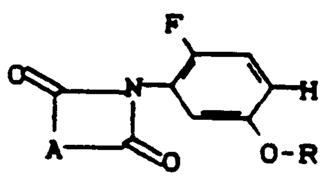

( Id )

in which
- A and R have the meanings mentioned in Claim 1,

are reacted with a halogenating agent, if appropriate in the presence of a catalyst and if appropriate in the presence of a diluent, or in that
(h) in the event that Het represents the group

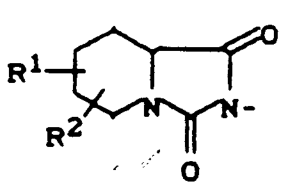

and
R, $R^1$, $R^2$ and X have the meanings mentioned in Claim 1,

arylamines of the general formula (III)

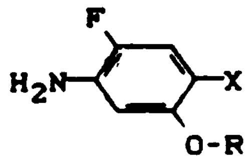

(III)

in which

R and X have the meanings mentioned in Claim 1,

are reacted with chloroformic acid esters of the general formula (VI)

$R^3O$ - CO - Cl (VI)

in which

$R^3$ represents lower alkyl, benzyl or phenyl,

if appropriate in the presence of an acid acceptor and if appropriate in the presence of a diluent, and the resulting arylurethanes of the general formula (VII)

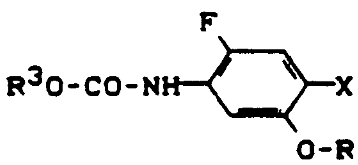

(VII)

in which

R, $R^3$ and X have the meaning mentioned in Claim 1

are reacted with piperidine-2-carboxylic acid esters of the general formula (VIII)

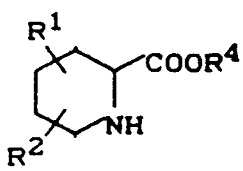

(VIII)

in which

$R^1$ and $R^2$ have the meanings mentioned in Claim 1 and

$R^4$ represents lower alkyl,

if appropriate in the presence of a diluent excluding the compounds in which

Het represents

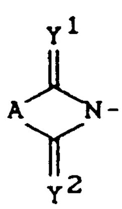

or

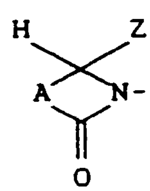

where

A represents the group

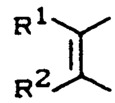

| | |
|---|---|
| Z | represents hydroxyl, |
| X | represents hydrogen or halogen, |
| $R^1$ and $R^2$ | in each case independently of one another represent hydrogen or alkyl, |
| $Y^1$ | represents oxygen or sulphur, |
| $Y^2$ | represents oxygen and |
| R | represents alkoxyalkyl, oxiranylalkyl or dioxolan-2-yl and 1,3-dioxan-2-yl radicals optionally substituted by 1 or 2 $C_1$-$C_4$-alkyl, |
| | and the compounds in which |
| Het | represents |

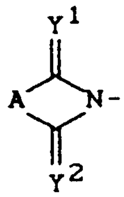

or

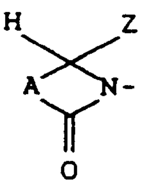

where

| | |
|---|---|
| A | represents the group |

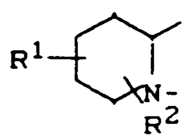

| | |
|---|---|
| $R^1$ and $R^2$ | in each case independently of one another represent hydrogen or alkyl, |
| $Y^1$ | represents oxygen or sulphur, |
| $Y^2$ | represents oxygen or sulphur, |
| X | represents oxygen or halogen, |
| Z | represents hydroxyl, and |
| R | represents alkoxyalkyl, furanylalkyl, pyranylalkyl and oxiranylalkyl, |
| | and the compounds in which |
| Het | represents |

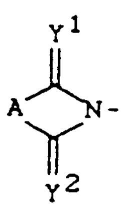

or

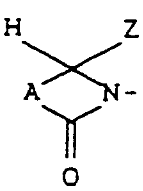

where

| | |
|---|---|
| A | represents the group |

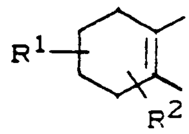

$R^1$ and $R^2$ in each case independently of one another represent hydrogen or alkyl,
$Y^1$ represents oxygen or sulphur,
$Y^2$ represents oxygen or sulphur,
X represents hydrogen or halogen,
Z represents hydroxyl and
R represents alkoxyalkoxy,

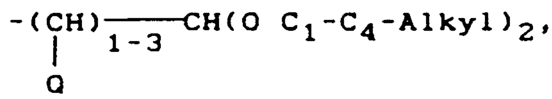

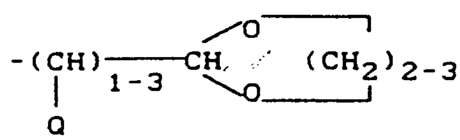

or cycloalkylalkyl or cycloalkenylalkyl interrupted by at least one oxygen atom and
Q represents hydrogen or $C_1$-$C_4$-alkyl.

**5.** Herbicidal and plant growth-regulating agents, characterised in that they contain at least one N-aryl nitrogen heterocycle of the formula (I) according to Claims 1 to 3.

**6.** Method for combating weeds, characterised in that N-aryl nitrogen heterocycles of the formula (I) according to Claims 1 to 3 are allowed to act on the weeds and/or their environment.

**7.** Use of N-aryl nitrogen heterocycles of the formula (I) according to Claims 1 to 3 for combating weeds and/or as plant growth regulators.

**8.** Process for the preparation of herbicidal and/or plant growth-regulating agents, characterised in that N-aryl nitrogen heterocycles of the formula (I) according to Claims 1 to 3 are mixed with extenders and/or surface-active substances.

**9.** Arylamines of the general formula (III)

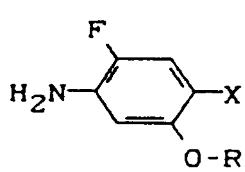

(III)

in which
R represents alkyl, alkenyl, cycloalkyl, cycloalkenyl, cycloalkylalkyl or cycloalkenylalkyl, each of which is optionally branched and each of which is interrupted by at least 1 oxygen atom, and
X represents hydrogen or halogen excluding the compounds in which
R represents alkoxyalkyl,

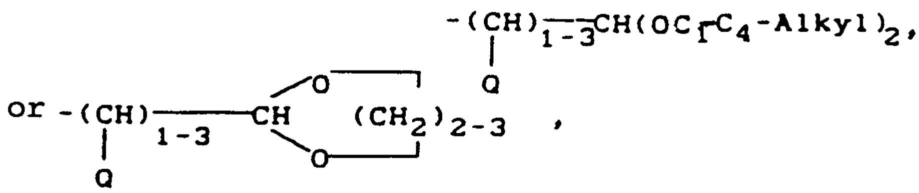

cycloalkylalkyl or cycloalkenylalkyl substituted by 1 or 2 alkyl and interrupted by at least one oxygen atom and

Q represents hydrogen or $C_1$-$C_4$-alkyl.

## Revendications

**1.** Hétérocycles N-aryl-azotés substitués de formule générale (I)

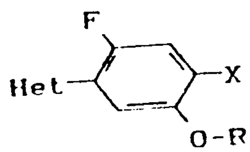

(I)

dans laquelle

Het désigne un des groupes hétérocycliques suivants

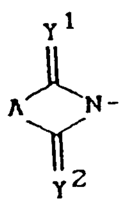

ou

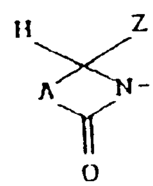

A désignant un des groupes suivants,

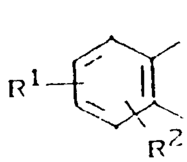 , 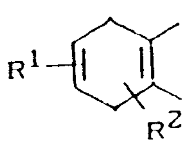 , 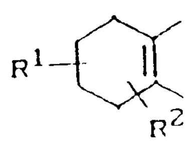 , 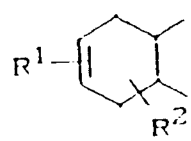 ,

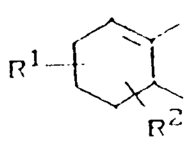 , 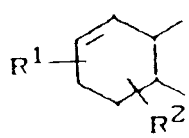 , 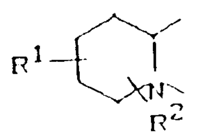 , 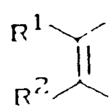 ,

dans lesquels

$R^1$ et $R^2$ désignent respectivement, indépendamment l'un de l'autre, l'hydrogène, un halogène, un radical halogénoalkyle ou alkyle,

$Y^1$ et $Y^2$ désignent respectivement l'oxygène ou le soufre et

Z désigne l'hydrogène, un radical hydroxyle ou le chlore,

R désigne un radical alkyle, alcényle, cycloalkyle, cycloalcényle, cycloalkylalkyle ou cycloalcénylalkyle éventuellement ramifié et respectivement interrompu par au moins un atome d'oxygène et

X désigne l'hydrogène ou un halogène,

en excluant les composés dans lesquels

Het désigne

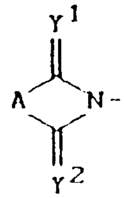

ou

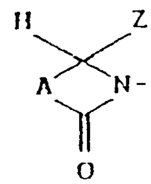

dans lesquels

A représente le groupe

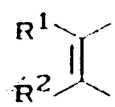

Z désigne un radical hydroxyle,

X désigne l'hydrogène ou un halogène,

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène,

R désigne un radical alcoxyalkyle, oxiranylalkyle ou des radicaux dioxolan-2-yle et 1,3-dioxan-2-yle éventuellement substitués par 1 ou 2 radicaux $C_1$-$C_4$-alkyle,

ainsi que les composés dans lesquels

Het désigne

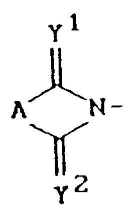

ou

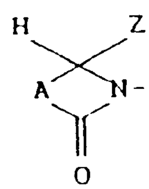

dans lesquels

A désigne le groupe

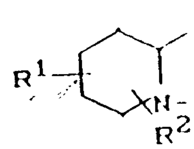

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène ou le soufre,

X désigne l'oxygène ou un halogène,

Z désigne un radical hydroxyle et

R désigne un radical alcoxyalkyle, furanylalkyle, pyranylalkyle et oxiranylalkyle,

ainsi que les composés dans lesquels

Het désigne

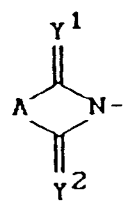 ou 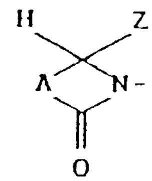

dans lesquels
A désigne le groupe

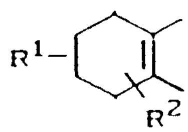

$R^1$ et $R^2$ désignent respectivement, indépendamment l'un de l'autre, l'hydrogène ou un radical alkyle,
$Y^1$ désigne l'oxygène ou le soufre,
$Y^2$ désigne l'oxygène ou le soufre,
X désigne l'hydrogène ou un halogène,
Z désigne un radical hydroxyle et
R désigne un radical alcoxyalcoxy

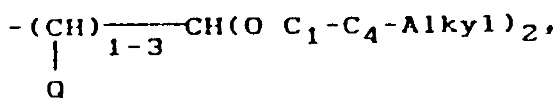

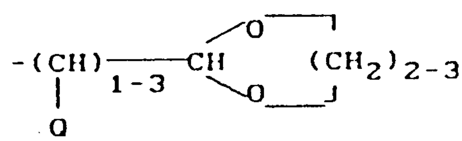

ou un radical cycloalkylalkyle ou cycloalcénylalkyle interrompu par au moins un atome d'oxygène et
Q désigne l'hydrogène ou un radical $C_1$-$C_4$-alkyle.

**2.** Composés de formule générale (I) selon la revendication 1, caractérisés en ce que
Het désigne un des groupes hétérocycliques suivants

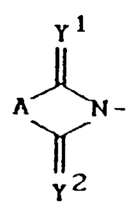 ou 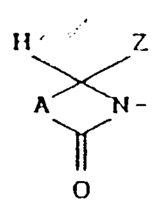

dans lesquels
A désigne un des groupes suivants,

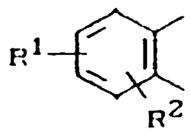 , 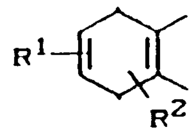 , 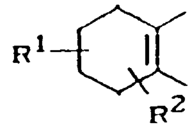 , 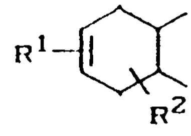 ,

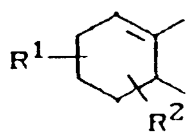 , 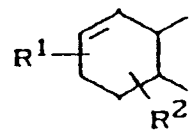 , 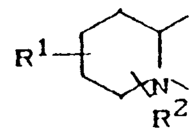 , 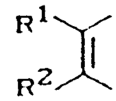 ,

$R^1$ et $R^2$ désignant, indépendamment l'un de l'autre, l'hydrogène, le fluor, le chlore, le brome ou un radical alkyle ou halogénoalkyle à chaîne droite ou ramifiée avec respectivement 1 à 3 atomes de carbone et, dans le cas de l'halogénoalkyle, avec 1 à 5 atomes d'halogène identiques ou différents, en particulier le fluor ou le chlor,

$Y^1$ et $Y^2$ désignent l'oxygène ou le soufre,

Z désigne l'hydrogène, un radical hydroxyle ou le chlore,

R désigne respectivement un radical alkyle, alcényle, cycloalkyle, cycloalcényle, cycloalkylalkyle ou cycloalcénylalkyle éventuellement ramifié et interrompu par 1 à 4 atomes d'oxygène avec respectivement jusqu'à 20 atomes de carbone et

X désigne l'hydrogène, le fluor, le chlore ou le brome,

en excluant les composés dans lesquels

Het désigne

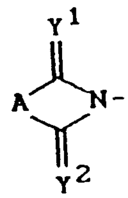 ou 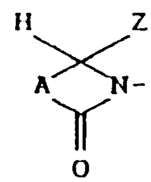

dans lesquels

A représente le groupe

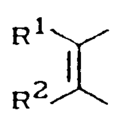

Z désigne le radical hydroxyle

X désigne l'hydrogène ou un halogène,

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène et

R désigne un radical alcoxyalkyle, oxiranylalkyle ou les radicaux dioxolan-2-yle et 1,3-dioxan-2-yle éventuellement substitués par 1 ou 2 radicaux $C_1$-$C_4$-alkyle

ainsi que les composés dans lesquels

Het désigne

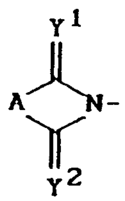

ou

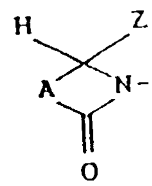

dans lesquels

A représente le groupe

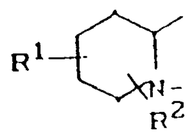

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène ou le soufre,

X désigne l'oxygène ou un halogène,

Z désigne un radical hydroxyle et

R désigne un radical alcoxyalkyle, furanylalkyle, pyranylalkyle et oxiranylalkyle,

ainsi que les composés dans lesquels

Het désigne

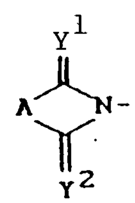

ou

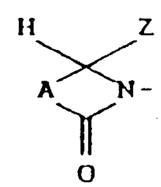

dans lesquels

A représente le groupe

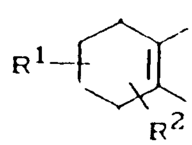

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène ou le soufre,

X désigne l'hydrogène ou un halogène,

Z désigne un radical hydroxyle et

R désigne un radical alcoxyalcoxy

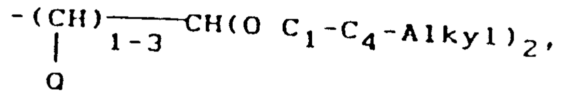

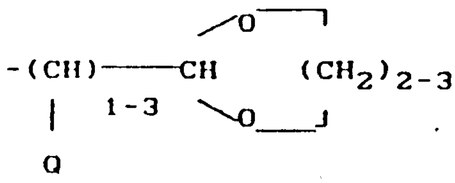

ou un radical cycloalkylalkyle ou cycloalcénylalkyle interrompu par au moins un atome d'oxygène et

Q désigne l'hydrogène ou un radical $C_1$-$C_4$-alkyle.

3. Composés de formule générale (I) selon la revendication I caractérisés en ce que
Het désigne un des radicaux hétérocycliques suivants

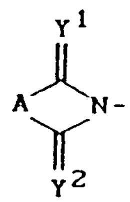 ou 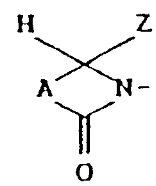

dans lesquels
A représente un des groupes suivants,

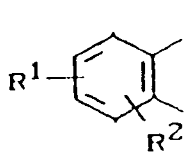 , 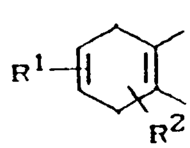 , 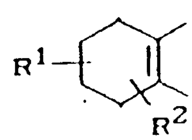 , 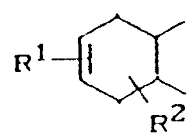 ,

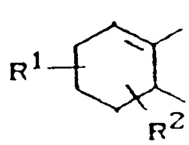 , 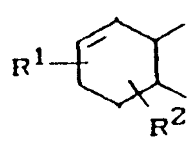 , 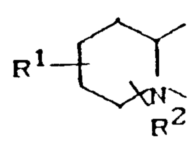 , 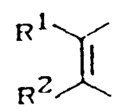 ,

$R^1$ et $R^2$ désignant respectivement, indépendamment l'un de l'autre, l'hydrogène, un radical méthyle ou trifluorométhyle,

$Y^1$ et $Y^2$ désignent l'oxygène ou le soufre,

Z désigne l'hydrogène, un radical hydroxyle ou le chlore

R désigne un radical oxaalkyle, oxaalcényle ou dioxaalkyle éventuellement ramifié avec respectivement 1 à 10 atomes de carbone ou un radical oxacycloalkylalkyle, oxacycloalcénylalkyle ou dioxacycloalkylalkyle avec jusqu'à 8 atomes de carbone dans la partie cycloalkyle, oxacycloalcényle ou dioxacycloalkyle et jusqu'à 3 atomes de carbone dans la partie alkyle et

X désigne l'hydrogène, le chlore ou le brome, en excluant les composés dans lesquels
Het désigne

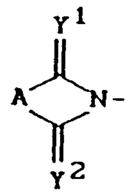 ou 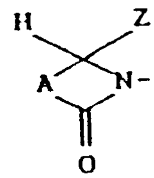

dans lesquels

A représente le groupe

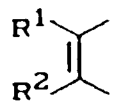

Z désigne un radical hydroxyle,

X désigne l'hydrogène ou un halogène,

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène et

R désigne un radical alcoxyalkyle, oxiranylalkyle ou les radicaux dioxolan-2-yle et 1,3-dioxan-2-yle éventuellement substitués par 1 ou 2 radicaux $C_1$-$C_4$-alkyle

ainsi que les composés dans lesquels

Het désigne

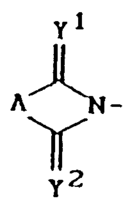 ou 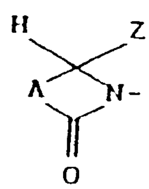

dans lesquels

A représente le groupe

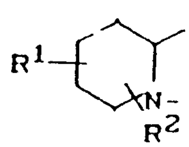

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène ou le soufre,

X désigne l'oxygène ou un halogène,

Z désigne un radical hydroxyle et

R désigne un radical alcoxyalkyle, furanylalkyle, pyranylalkyle et oxiranylalkyle,

ainsi que les composés dans lesquels

Het désigne

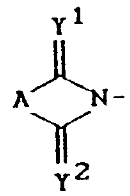

ou

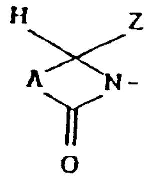

dans lesquels

A représente le groupe

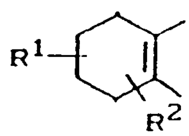

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène ou le soufre,

X désigne l'hydrogène ou un halogène,

Z désigne un radical hydroxyle et

R désigne un radical alcoxyalcoxy

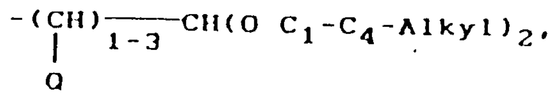

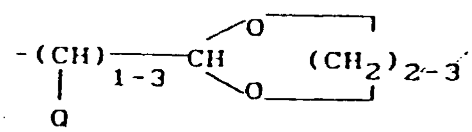

ou un radical cycloalkylalkyle ou cycloalcénylalkyle interrompu par au moins un atome d'oxygène et

Q désigne l'hydrogène ou un radical $C_1$-$C_4$-alkyle.

**4.** Procédé de préparation d'hétérocycles N-aryl-azotés substitués de formule générale (I) selon la revendication I caractérisé en ce que

(a) au cas où Het désigne le groupe

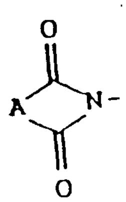

et A, R et X ont les significations susmentionnées,

des anhydrides cycliques de formule générale (II)

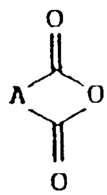

(II)

dans laquelle
A a la signification susmentionnée dans la revendication 1,
sont amenés à réagir avec des arylamines de formule générale (III)

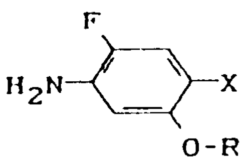

(III)

dans laquelle
R et X ont les significations susmentionnées dans la revendication 1,
éventuellement en présence d'un diluant et éventuellement en présence d'un adjuvant de réaction ou
(b) au cas où Het désigne le groupe

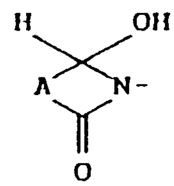

et A, R et X ont les significations susmentionnées dans la revendication 1,
des arylimides substitués de formule générale (Ia)

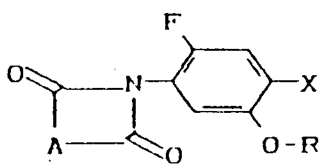

(Ia)

dans laquelle
A, R et X ont les significations susmentionnées dans la revendication 1,
sont amenés à réagir avec un réducteur, éventuellement en présence d'un diluant ou
(c) au cas où Het désigne le groupe

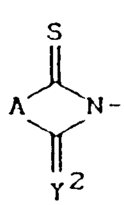

et A, R, X et $Y^2$ ont les significations susmentionnées dans la revendication 1, des arylimides substitués de formule générale (Ia)

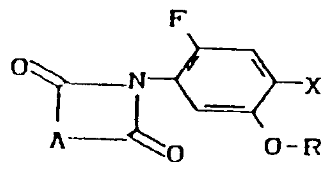

(Ia)

dans laquelle
A, R et X ont les significations susmentionnées dans la revendication 1,
sont amenés à réagir avec un agent de sulfuration, éventuellement en présence d'un diluant ou
(d) au cas où Het désigne le groupe

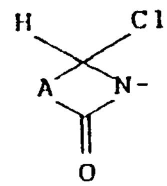

et A, R et X ont les significations susmentionnées dans la revendication 1, des hétérocycles N-aryl-azotés de formule générale (Ib)

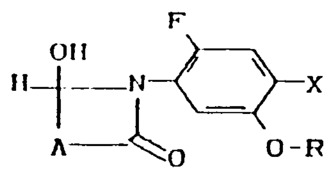

(Ib)

dans laquelle
A, R et X ont les significations susmentionnées dans la revendication 1
sont amenés à réagir avec du chlorure de thionyle, éventuellement en présence d'un diluant et éventuellement en présence d'un adjuvant de réaction ou
(e) au cas où Het désigne le groupe

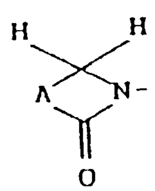

et A, R et X ont les significations susmentionnées dans la revendication 1, des hétérocycles N-aryl-azotés de formule générale (Ic)

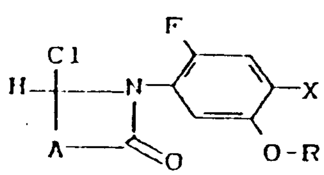

(Ic)

dans laquelle
A, R et X ont les significations susmentionnées dans la revendication 1
sont amenés à réagir avec de l'hydrogène en présence d'un catalyseur ainsi qu'éventuellement en présence d'un diluant et éventuellement en présence d'un agent neutralisateur d'acide ou
(f) au cas où Het désigne le groupe

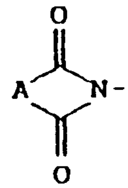

et A, R et X ont les significations susmentionnées dans la revendication 1,
des hydroxyarylimides de formule générale (IV)

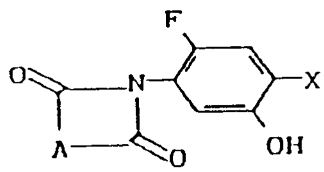

(IV)

dans laquelle
A et X ont les significations susmentionnées dans la revendication 1
sont amenés à réagir avec des agents d'alkylation de formule générale (V)

$X^1$-R (V)

dans laquelle
R a la signification susmentionnée dans la revendication 1 et
$X^1$ désigne un radical de perte nucléophile
éventuellement en présence d'un accepteur d'acide et éventuellement en présence d'un diluant ou
(g) au cas où Het désigne le groupe

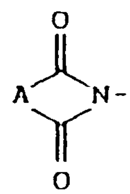

et A et R ont les significations susmentionnées dans la revendication 1
et X désigne un halogène
des acrylimides substitués de formule générale (Id)

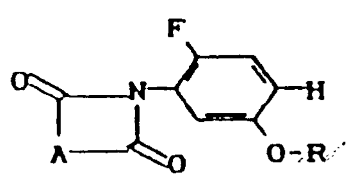

(Id')

dans laquelle

A et R ont les significations susmentionnées dans la revendication 1
sont amenés à réagir avec un agent d'halogénation, éventuellement en présence d'un catalyseur et éventuellement en présence d'un diluant ou
(h) au cas où Het désigne le groupe

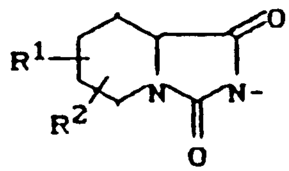

et R, $R^1$, $R^2$ et X ont les significations susmentionnées dans la revendication 1
des arylamines de formule générale (III)

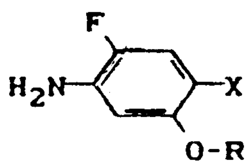

(III)

dans laquelle
R et X ont les significations susmentionnées dans la revendication 1
sont amenées à réagir avec des esters d'acide chloroformique de formule générale (VI)

$R^3O$ - CO - Cl (VI)

dans laquelle
$R^3$ désigne un radical alkyle inférieur, benzyle ou phényle
éventuellement en présence d'un accepteur d'acide et éventuellement en présence d'un diluant et les aryluréthanes ainsi formés de formule générale (VII)

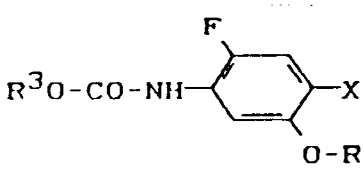

(VII)

dans laquelle
R, $R^3$ et X ont la signification susmentionnée dans la revendication 1
sont amenés à réagir avec des esters d'acide pipéridine-2-carboxylique de formule générale (VIII)

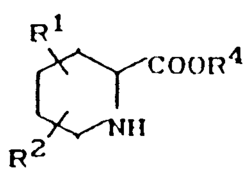

(VIII)

dans laquelle
$R^1$ et $R^2$ ont les significations susmentionnées dans la revendication 1
et $R^4$ désigne un radical alkyle inférieur,
éventuellement en présence d'un diluant en excluant les composés dans lesquels

Het désigne un groupe

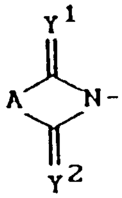

ou

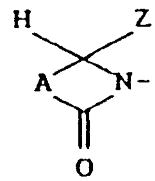

dans lesquels

A représente le groupe

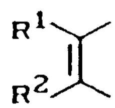

Z désigne le radical hydroxyle,

X désigne l'hydrogène ou un halogène,

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène et

R désigne un radical alcoxyalkyle, oxiranylalkyle ou les radicaux dioxolan-2-yle et 1,3-dioxan-2-yle éventuellement substitués par 1 ou 2 radicaux $C_1$-$C_4$-alkyle

ainsi que les composés dans lesquels

Het désigne

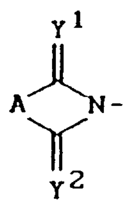

ou

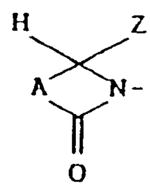

dans lesquels

A désigne le groupe

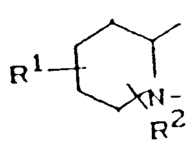

$R^1$ et $R^2$ désignent respectivement, indépendamment l'un de l'autre, l'hydrogène ou un radical alkyle,

$Y^1$ désigne l'oxygène ou le soufre,

$Y^2$ désigne l'oxygène,

X désigne l'oxygène ou un halogène,

Z désigne un radical hydroxyle et

R désigne un radical alcoxyalkyle, furanylalkyle, pyranylalkyle et oxiranylalkyle,

ainsi que les composés dans lesquels
Het désigne

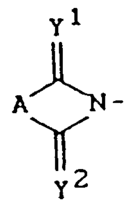

ou

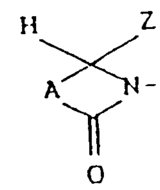

dans lesquels

A représente le groupe

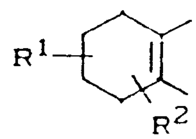

$R^1$ et $R^2$ désignent respectivement, indépendamment l'un de l'autre, l'hydrogène ou un radical alkyle,

$Y^1$ désigne l'oxygène ou le soufre,
$Y^2$ désigne l'oxygène ou lé soufre,
X désigne l'hydrogène ou un halogène,
Z désigne un radical hydroxyle et
R désigne un radical alcoxyalcoxy

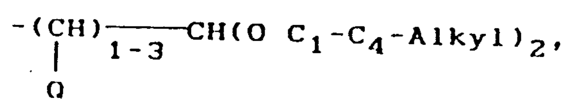

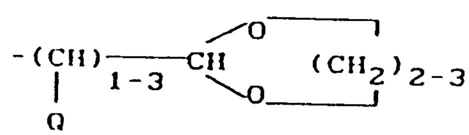

ou un radical cycloalkylalkyle ou cycloalcénylalkyle interrompu par au moins un atome d'oxygène et

Q désigne l'hydrogène ou un radical $C_1$-$C_4$-alkyle.

5. Herbicides et agents régulateurs de croissance végétale caractérisés par une teneur en au moins un hétérocycle N-aryl-azoté de formule (I) selon les revendications 1 à 4.

6. Procédé de lutte contre les mauvaises herbes caractérisé en ce que des hétérocycles N-aryl-azotés de formule (I) selon les revendications 1 à 4 sont amenés à agir sur des mauvaises herbes et/ou leur environnement.

7. Mise en oeuvre d'hétérocycles N-aryl-azotés de formule (I) selon les revendications 1 à 4 pour la lutte contre les mauvaises herbes et/ou comme régulateur de la croissance végétale.

8. Procédé de préparation d'agents herbicides et/ou régulateurs de croissance végétale caractérisé en ce que des hétérocycles N-aryl-azotés de formule (I) selon les revendications 1 à 4 sont mélangés avec

des diluants et/ou des substances tensio-actives.

**9.** Arylamines de formule générale (III)

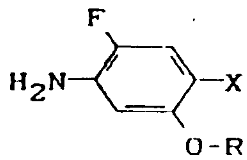

(III)

dans laquelle

R désigne un radical alkyle, cycloalkyle, alcényle, cycloalcényle, cycloalkylalkyle ou cycloalcénylalkyle éventuellement ramifié et respectivement interrompu par au moins un atome d'oxygène et

X désigne l'hydrogène ou un halogène,

à l'exception des composés dans lesquels

R désigne un radical alcoxyalkyle,

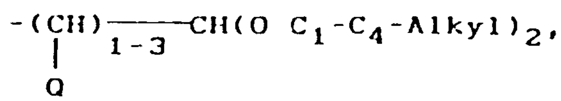

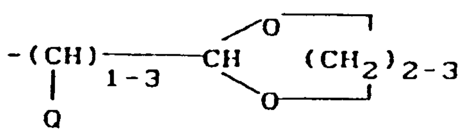

cycloalkylalkyle ou cycloalcénylalkyle substitué par 1 ou 2 radicaux alkyle et interrompu par au moins un atome d'oxygène et

Q désigne l'hydrogène ou un radical $C_1$-$C_4$-alkyle.